(19) Europäisches Patentamt
European Patent Office
Office européen des brevets

(11) EP 2 710 116 B1

(12) **EUROPEAN PATENT SPECIFICATION**

(45) Date of publication and mention of the grant of the patent:
**27.03.2019 Bulletin 2019/13**

(21) Application number: **12785922.1**

(22) Date of filing: **17.05.2012**

(51) Int Cl.:
***C12N 1/21*** (2006.01) ***C12N 7/00*** (2006.01)
***A61K 35/74*** (2015.01) ***A61K 39/112*** (2006.01)

(86) International application number:
**PCT/KR2012/003920**

(87) International publication number:
**WO 2012/157989 (22.11.2012 Gazette 2012/47)**

(54) **AVIRULENT MODIFIED SALMONELLA GALLINARUM STRAINS AND PHARMACEUTICAL COMPOSITION USING THE SAME**

AVIRULENTE MODIFIZIERTE SALMONELLA-GALLINARUM-STÄMME UND PHARMAZEUTISCHE ZUSAMMENSETZUNG DAMIT

SOUCHES MODIFIÉES AVIRULENTES DE SALMONELLA GALLINARUM ET COMPOSITION PHARMACEUTIQUE UTILISANT CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2011 US 201161487137 P**
**17.10.2011 US 201113274854**

(43) Date of publication of application:
**26.03.2014 Bulletin 2014/13**

(73) Proprietor: **CJ Cheiljedang Corporation**
**Seoul 100-400 (KR)**

(72) Inventors:
- **CHOI, Hyang**
**Anyang-si**
**Gyeonggi-do 430-738 (KR)**
- **SHIN, Soo An**
**Seoul 138-790 (KR)**
- **YANG, Si Yong**
**Incheon 405-796 (KR)**
- **CHO, Young Wook**
**Seoul 140-751 (KR)**

(74) Representative: **Pallini Gervasi, Diego et al**
**Notarbartolo & Gervasi GmbH**
**Bavariaring 21**
**80336 Munich (DE)**

(56) References cited:
**US-A1- 2010 135 962** **US-B2- 6 923 957**
**US-B2- 7 887 816**

- **MI AH KIM: "Development of oral vaccine candidate using attenuated Salmonella Gallinarum", 22 February 2010 (2010-02-22), DISSERTATION, CHONBUK NATIONAL UNIVERSITY, PAGE(S) 69PP, XP008171515, * abstract * * page 1, line 15 - page 5, line 13 * * page 12, line 18 - page 14, line 11 * * page 16, line 14 - page 18, line 19; tables 2-3 * * page 19, line 3 - page 23, line 4 ***
- **D. H. SHAH: "Identification of Salmonella gallinarum virulence genes in a chicken infection model using PCR-based signature-tagged mutagenesis", MICROBIOLOGY, vol. 151, no. 12, 1 December 2005 (2005-12-01), pages 3957-3968, XP055134588, ISSN: 1350-0872, DOI: 10.1099/mic.0.28126-0**
- **I RYCHLIK ET AL: "The presence of genes homologous to the K88 genes faeH and fael on the virulence plasmid of Salmonella gallinarum", FEMS MICROBIOLOGY LETTERS, vol. 159, no. 2, 15 February 1998 (1998-02-15), pages 255-260, XP055134586, ISSN: 0378-1097, DOI: 10.1016/S0378-1097(97)00579-X**
- **BARTH S., BAUERFEIND R.: "Virulenzplasmide bei Salmonella enterica - Vorkommen und Eigenschaften (Virulence plasmids of Salmonella enterica - Incidence and Properties)", BERL. MÜNCH. TIERÄRZTL. WSCHR, vol. 118, 1 January 2005 (2005-01-01), pages 8-23, XP9181172, ISSN: 0005-9366**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- C Roudier ET AL: "Correlation between the presence of sequences homologous to the vir region of Salmonella dublin plasmid pSDL2 and the virulence of twenty-two Salmonella serotypes in mice.", INFECTION AND IMMUNITY, 1 May 1990 (1990-05-01), pages 1180-1185, XP055152812, Retrieved from the Internet: URL:http://iai.asm.org/content/58/5/1180.full.pdf [retrieved on 2014-11-13]
- WIGLEY PAUL ET AL: "Oral infection with the Salmonella enterica serovar Gallinarum 9R attenuated live vaccine as a model to characterise immunity to fowl typhoid in the chicken", BMC VETERINARY RESEARCH, BIOMED CENTRAL, LONDON, GB, vol. 1, no. 1, 12 September 2005 (2005-09-12), page 2, XP021011435, ISSN: 1746-6148, DOI: 10.1186/1746-6148-1-2
- YOUNG J. LEE ET AL: "Safety and efficacy of Salmonella gallinarum 9R vaccine in young laying chickens", AVIAN PATHOLOGY, vol. 34, no. 4, 1 August 2005 (2005-08-01) , pages 362-366, XP055152998, ISSN: 0307-9457, DOI: 10.1080/03079450500180895
- GERLACH R. G., HENSEI M.: "Salmonella Pathogenicity Islands in host specificity, host pathogen-interactions and antibiotics resistance of Salmonella enterica", BERL. MÜNCH. TIERÄRZTL. WOCHENSCHR., vol. 120, no. 7/8, 1 May 2007 (2007-05-01) , pages 317-327, XP9181171, DOI: 10.2376/0005-9366-120-317
- MI AH KIM: 'Development of oral vaccine candidate using attenuated Salmonella Gallinarum' DISSERTATION February 2010, CHONBUK NATIONAL UNIVERSITY, XP008171515
- S. PORWOLLIK ET AL.: 'Differences in Gene Content between Salmonella enterica serovar Enteritidis isolates and comparison to closely related serovars Gallinarum and Dublin' JOURNAL OF BACTERIOLOGY. vol. 187, no. 18, September 2005, pages 6545 - 6555, XP055134542
- RAFAEL ROTGER ET AL.: 'The virulence plasmids of Salmonella' INTERNATIONAL MICROBIOLOGY vol. 2, no. 3, September 1999, pages 177 - 184, XP055134583
- I. RYCHLIK ET AL.: 'Distribution and function of plasmids in Salmonella enterica' VETERINARY MICROBIOLOGY. vol. 112, no. 1, 10 January 2006, pages 1 - 10, XP024935617
- I. RYCHLIK ET AL.: 'The presence of genes homologous to the K88 genes faeH and fael on the virulence plasmid of Salmonella gallinarum' FEMS MICROBIOLOGY LETTERS. vol. 159, no. 2, 15 February 1998, pages 255 - 260, XP055134586
- DEVENDRA H. SHAH ET AL.: 'Identification of Salmonella gallinarum virulence genes in a chicken infection model using PCR-based signature -tagged mutagenesis' MICROBIOLOGY. vol. 151, December 2005, pages 3957 - 3968, XP055134588

## Description

[Technical Field]

The present invention provides avirulent modified *Salmonella Gallinarum* strains and various uses thereof.

[Background Art]

Currently over 2,000 *Salmonella* strains are generally classified into host-specific serotypes, and non-host-specific serotypes pathogenic for both animals and humans. Representative among fowl-adapted pathogens are *Salmonella Gallinarum* (SG) and *Salmonella Pullorum* (SP) which are known to cause fowl typhoid and pullorum disease, respectively. These *Salmonella*-caused fowl diseases occur at low frequency in advanced countries, but have inflicted tremendous economic damage on poultry farming in developing countries.

Serologically, *Salmonella Gallinarum* strains have the same somatic antigen (O-antigen) structures and are classified as being non-motile because they have no flagella. After entering into a host animal via contaminated feed or a contaminated environment, *Salmonella* pass through the gastrointestinal tract, and invade intestinal epithelial cells by interaction with microfold (M) of Peyer's patch cells and penetrate into the intestinal membrane. *Salmonella* are transported by the M cells to macrophages in adjacent intestinal membranes, and then *Salmonella* infection develops into a systemic disease.

The type III secretion system (TTSS) is a protein appendage found in gram-negative bacteria, which consists of a needle-like protein complex structure through which virulence effector proteins pass from the bacterial cytoplasm directly into the host cytoplasm (Mota LJ et al., Ann Med. (2005);37(4):234-249). The type III secretion system is essential for the delivery of the pathogenicity of *Salmonella* (Schlumberger MC et al., Curr Opin Microbiol. (2006);9(1):46-54). Wild-type *Salmonella* take advantage of TTSS when adhering to and invading host cells, and then survives during the phagocytosis of macrophages and circulates throughout the body via the bloodstream, causing a systemic infection. Hence, *Salmonella* infection cannot proceed without the normal operation of TTSS. *Salmonella* pathogenicity island-1 (hereinafter referred to as "SPI-1") is a discrete region of the *Salmonella* chromosome encoding the type III secretion system and virulent effector proteins which are necessary for invasion into intestinal epithelial cells in the early stage of infection (Kimbrough TG et al., Microbes Infect, (2002);4(1):75-82). *Salmonella* pathogenicity island-2 (hereinafter referred to as "SPI-2") is also a discrete region of the *Salmonella* chromosome encoding the type III secretion system and effector proteins which involved in survival and proliferation during phagocytosis by macrophages in intestinal immune organs or immune organs such as the spleen and the liver after translocation across epithelial cells (Waterman SR et al., Cell Microbiol, (2003);5(8):501~511, Abrahams GL, Cell Microbiol, (2006);8(5):728-737). Genes within SPI-1 and SPI-2 and their functions are summarized in Table 1, below.

[Table 1]

| | Gene | Characteristics |
|---|---|---|
| **SPI-1** | **avrA** | **putative inner membrane protein** |
| | **sprB** | **transcriptional regulator** |

(continued)

| | Gene | Characteristics |
|---|---|---|
| | hilC | bacterial regulatory helix-turn-helix proteins, araC family |
| | orgA | putative flagellar biosynthesis/type III secretory pathway protein |
| | prgK | cell invasion protein; lipoprotein, may link inner and outer membranes |
| | prgJIH | cell invasion protein |
| | hilD | regulatory helix-turn-helix proteins, araC family |
| | hilA | invasion genes transcription activator |
| | iagB | cell invasion protein |
| | sptP | protein tyrosine phosphate |
| | sicP | chaperone, related to virulence |
| | iacP | putative acyl carrier protein |
| | sipADCB | cell invasion protein |
| | sicA | surface presentation of antigens; secretory proteins |
| | spaSRQPO | surface presentation of antigens; secretory proteins |
| | invJICB | surface presentation of antigens; secretory proteins |
| | invAEGFH | invasion protein |
| SPI-2 | ssaUTSRQPON VMLKJIHG | Secretion system apparatus |
| | sseGF | Secretion system effector |
| | sscB | Secretion system chaperone |
| | sseEDC | Secretion system effector |
| | sscA | Secretion system chaperone |
| | sseBA | Secretion system effector |
| | ssaE | Secretion system effector |
| | ssaDCB | Secretion system apparatus |
| | ssrA | Secretion system regulator:Sensor component |
| | ssrB | Secretion system regulator: transcriptional activator, homologous with degU/uvrY/bvgA |

In addition to these type III secretion systems, fimbriae gene (*faeHI*) (Edwards RA et al., PNAS (2000); 97(3):1258-1262) and the virulent factor (*spvRABCD* operon) present in virulent plasmids of *Salmonella* are implicated in the virulence of *Salmonella* (Gulig PA et al., Mol Microbiol (1993);7(6):825-830).

*Salmonella-caused* fowl diseases are difficult to control because they are transmitted in various ways including egg transmission, and feed or environmental infection, and show high recurrence rates even after post-infectious treatment with antibiotics. Therefore, it is important to prevent the onset of disease by using a vaccine as well as sanitizing breeding farms and feed. In the poultry industry, a lot of effort has been put into the use of live vaccines (attenuated *Salmonella Gallinarum* strains- SG9S, SG9R) and dead vaccines (gel vaccines, oil vaccines, etc.) to prevent the onset of fowl typhoid. However, the effects of the vaccine vary with the concentration of the vaccine used, the condition of the fowl vaccinated, and the environment of chicken coops. The efficacy of these vaccines is reported to be significantly lower than that of the vaccines for other diseases. Treatment with antibiotics, although reducing the resulting lesions, converts infected fowls into chronic carriers (See: Incidence and Prevention of Hen Salmonellosis, the National Veterinary Research & Quarantine Service, Korea).

Therefore, new *Salmonella-controlling* approaches that are better than conventional vaccines or antibiotics are being demanded. Many scientists have recently paid attention to bacteriophages, which infect and lyse bacteria specifically and are safe to humans, as a potent alternative to antibiotics. There are many reports concerning the use of bacteriophages in the prevention or therapy of *Salmonella* diseases (Atterbury RJ et al., Appl Environ Microbiol, (2007);73(14):4543-4549) and as disinfectants or detergents to prevent the putrefaction of foods (PCT 1998-08944, PCT 1995-31562, EP 1990-202169, PCT 1990-03122), and concerning phage display techniques for diagnosis (Ripp S et al., J Appl Microbiol, (2006);100(3):488-499). *Salmonella* vaccines prepared by deleting or modifying one or two genes within SPI-2 gene cluster have recently been disclosed (US 6923957, US7211264, US7887816).

The dissertation of Mi Ah Kim with the title "Development of oral vaccine candidate using attenuated Salmonella Gallinarum (22-Feb-2010, Chonbuk National University, Dissertation, Pages 69pp) discloses a Salmonello Gallinarum strain wherein the prgK-H gene has been deleted and a further Salmomello Gallinarum strain wherein the sprP gene has been deleted.

D.H.Shah (Microbiology, vol. 151, no. 12, of 1-Dec 2005) has identified Salmonella Gallinarum virulence genes in a chicken infection model using a PCR-based signature tagged mutagenesis system.

I. Rychlik et al. (FEMS Microbiology letters, vol. 159, no. 2, 15-Feb-1998) have identified genes homologues to the genes faeH and faeI on the virulence plasmid of Salmonella Gallinarum.

Furthermore, S. Barth and R. Bauerfeind (Berl. Münch. Tierärztl. Wschr, vol. 118, 1-Jan-2005) disclose a Salmonella typhimurium strain from which characteristics and functions of specific Spvproteins have been identified.

For industrial use, bacteriophages are produced by separating the phage progenies from the host cells lysed during the proliferation of bacteriophages which have been inoculated into the host cells cultured on a mass scale. As for bacteriophages specific for pathogenic bacteria, however, their lysates may contain the pathogenic host cells being not removed, and/or virulent materials such as pathogenic proteins of the host. This likelihood acts as a great risk factor to the safety of bacteriophages produced on the basis of pathogenic host cells.

Many bacteria have lysogenic phages on their chromosomes; however, most of the phages are cryptic and cannot produce progeny because of the accumulation of many mutations as ancestral remnants. Lysogenic phages, although inactive, may help the survival capacity of *Salmonella* upon host infection because they contain the genes necessary for lytic and lysogenic growth and some of the genes encode pathogenic factors. However, these genes are likely to undergo homologous recombination with the viral genome of other similar phages which newly infect animals, thus producing genetically modified phages. As for the typical *Salmonella typhimurium,* it has fels-1, fels-2, gifsy-1, and gifsy-2 prophages and two cryptic phages. In contrast, *Salmonella Gallinarum* could be used as a phage-producing host since *Salmonella Gallinarum* have neither prophages nor cryptic phages, and then the produced phage are not genetically modified by recombination (Edwards RA et al, Trends Microbiol, (2002); 10(2):94-99).

For the purpose of minimizing toxic remnants during progeny production and phage's opportunity for mutation, the present inventors designed the concept that the virulence gene clusters of *Salmonella Gallinarum* could be inactivated for producing bacteriophages. There have no precedent cases wherein avirulent bacteria, which had been converted from virulent bacteria by inactivating a virulence gene cluster, were used as a bacteriophage host cell.

In addition to the production of bacteriophages, the *Salmonella* deprived of virulence by inactivating virulence gene clusters are themselves used for developing attenuated live vaccines for controlling *Salmonella* or applied to the bioindustry, guaranteeing significant added value.

In the present invention, avirulent modified *Salmonella Gallinarum* strains obtained by deleting at least one of the main *Salmonella* virulence entire gene cluster selected from the following (i) to (iv): (i) Salmonella Pathogenicity Island-2 (SPI-2), (ii) Salmonella Pathogenicity Island-1 (SPI-1) and SPI-2, (iii) spvRABCD, and (iv) SPI-1, SPI-2, spvRABCD, and faeHI, are used as a bacteriophage-producing host cell and applied in various uses.

[Disclosure]

With the aim of solving the problems with the recombinational modification of progeny phages and the toxic bacterial remnants in the course of bacteriophage production on the basis of the above-described facts, the present inventors developed avirulent modified *Salmonella Gallinarum* strains in accordance with claim 1 to be used as a host cell for bacteriophage-producing. In addition, the present inventors primarily confirmed reduced virulence by measuring the efficiency of the invasion of *Salmonella Gallinarum* into avian epithelial cells, and reconfirmed by measuring the mortality of hens infected with avirulent modified *Salmonella Gallinarum* strains. On the other hand, the present inventors approve the use of bacteriophage-producing host, the use of the pharmaceutical compositions and feed additives for the prevention or treatment of avian salmonellosis through comparison of the productivity of bacteriophages between wild-type and the avirulent modified *Salmonella Gallinarum* strains.

It is another object of the present invention to provide the use of the avirulent modified *Salmonella Gallinarum* strain according to claim 1 in the production of *Salmonella*-specific bacteriophages or a method for producing phages using the avirulent modified *Salmonella Gallinarum* strain(s) according to claim 1. The avirulent modified *Salmonella Gallinarum* strains according to the present invention can be used for the mass-production of *Salmonella*-specific lytic bacteriophages free of remnant toxicity and applied to the development of a novel concept of antibiotic substitutes which have high industrial utility value and guarantee significant added value.

In accordance with the present invention, it is a further provided a pharmaceutical composition according to claims 9 and 10, a vaccine according to claim 11 and a feed additive according to claim 12.

The SPI-1 gene cluster encodes type III secretion system proteins which remain on cell surfaces, acting as an antigen while the SPI-2 gene cluster encodes proteins which are involved in survival in the phagosomes after passage across epithelial cells. Hence, the inactivation of the SPI-2 gene cluster alone, with the SPI-1 gene cluster remaining intact, leaves the antigen necessary for the production of an antibody inducing an immune response, but does not allow the *Salmonella* to survive during phagocytosis, thus not resulting in a systemic disease. Thus, the SPI-2 gene cluster-inactivated *Salmonella Gallinarum* strain might be used as a live vaccine.

In accordance with the present invention, avirulent modified *Salmonella Gallinarum* strains according to claim 1 are provided which are remarkably decreased in pathogenicity.

As used herein, the term "virulence gene clusters of *Salmonella*" refers to the four gene clusters involved in the virulence of *Salmonella Gallinarum*, including the *Salmonella* Pathogenicity Island-1 (hereinafter referred to as "SPI-1") operon coding for the structural proteins and toxic effector proteins of type III secretion system, the *Salmonella* Pathogenicity Island-2 (hereinafter referred to as "SPI-2") operon coding for the structural proteins and toxic effector proteins of type III secretion system, the *spvRABCD* operon coding for pathogenically active proteins on avian *Salmonella*-specific virulent plasmids, and the *faeHI* operon coding for fimbriae. So long as it functionally works in *Salmonella Gallinarum*, any gene cluster may be used.

The term "gene cluster" as used herein, refers to a population of adjacent genes on a chromosome or a plasmid that are commonly responsible for the same products. The genes in one cluster are under the regulation of common regulatory genes.

The inactivation of genes in bacteria can be achieved using various methods. For example, by genetic modification (mutation) of the signal structures of gene expression or also by the antisense-RNA technique known in the art without undue experimentation. Signal structures of gene expression are, for example, repressor genes, activator genes, operators, promoters, attenuators, ribosome binding sites, the start codon and terminators, but not limited(Jensen and Hammer, Biotechnology and Bioengineering 58:191195 (1998), Carrier and Keasling, Biotechnology Progress 15:5864 (1999), Franch and Gerdes, Current Opinion in Microbiology 3:159164 (2000), Knippers ("Molecular Genetics", 6th edition, 1995) or that of Winnacker ("Genes and Clones", 1990), the contents of each of which are in corporate herein by reference).

Also, an RNA interference mechanism (RNA interference: RNAi) which induces target gene mRNA degradation through the introduction of double-stranded RNA (dsRNA) consisting of the sense strand comprising the mRNA of the target gene and the homologous sequences thereof, and the antisense strand comprising the complementary sequence may be used. In addition, the method using transposon, which can be moved to another location within the genome of a single cell, can be used to inactivate the gene through transposon-mediated mutation to block the function of the target gene. Mutations which lead to a change or reduction in the catalytic properties of enzyme proteins of gene clusters are known from the prior art(Qiu and Goodman (Journal of Biological Chemistry 272:86118617 (1997)), Yano et al.,(Proceedings of the National Academy of Sciences, USA 95:5511 5515 (1998)), and Wente and Schachmann (Journal of Biological Chemistry 266:2083320839 (1991)). Summarizing descriptions can be found in known text books of genetics and molecular biology, such as e.g. that by Hagemann ("General Genetics", 1986).

Preferably, the inactivation of the gene cluster is accomplished using a method selected from the following: modification of singular or plural nucleotides in the gene, deletion of single or plural genes, insertion of an exogenous gene into the genes, deletion of all of the gene clusters, insertion of suppressing sequences into the promoter of the gene cluster, mutation of the promoter, insertion of an expression suppressing the regulation sequence of the gene cluster, introduction of the RNAi for single or plural nucleotides in the gene cluster, transposon-mediated mutations, and a combination thereof.

The methods described above may be commonly understood by those of ordinary skill in the art and conducted as described(Sambrook et al., Molecular Cloning : A Laboratory Manual, Third Edition (Cold SpringHarbor Press 2001)).

For example, single or multiple nucleotides of an active site within a gene may be modified to decrease the activity of the protein expressed. Alternatively, an antibiotic-resistant gene or other gene(s) may be inserted into the gene of interest to prevent the expression of intact proteins. The most preferable method is to delete the entire sequence of a gene cluster from the genome (Russell CB et al., J. Bacteriol. (1989); 171:2609-2613, Hamilton CM et al., J. Bacteriol. (1989); 171:4617-4622, Link AJ et al., J. Bacteriol. (1997); 179:6228-6237). In the present invention, entire sequence of the gene cluster of interest are deleted to effectively insure the inactivation of the genes. For this, the one-step deletion method using lamda Red recombinase, known as a method of deleting gene clusters, developed by Datsenk KA *et al.,* may be employed (Datsenko KA et al., PNAS, (2000);97(12):6640-6645).

With regard to the information of the virulence genes to be deleted, nucleotide sequences of SPI-1 and SPI-2 were obtained referring to the virulence gene sequences within the *Salmonella Gallinarum* chromosome (*Salmonella enterica subsp. enterica serovar Gallinarum str.*287/91, NC 011274), disclosed by the NCBI. For the *faeHI* operon sequence, reference was made to the sequence of the *Salmonella Gallinarum* virulence plasmid gene (*Salmonella Gallinarum* virulence plasmid minor fimbrial subunit genes, AF005899). For the *spvRABCD* operon, the sequence of the same name gene of *Salmonella Typhimurium LT2*, which is highly homologous with *Salmonella Gallinarum*, was consulted, because its sequence is not disclosed in the NCBI. The sequencing of the *spvRABCD* operon of *Salmonella Gallinarum* was also performed with reference to the sequence of the corresponding gene of *Salmonella Typhimurium.*

Examples of the *Salmonella* virulence genes clusters include the SPI-1 gene cluster (SEQ ID NO: 1), the SPI-2 gene cluster (SEQ ID NO: 2), the *spvRABCD* operon (SEQ ID NO: 3), and the *faeHI* operon (SEQ ID NO: 4) of *Salmonella Gallinarum* 287/91.

To prepare strains that had been rendered avirulent, at least one virulence gene clusters were deleted.

The present invention includes a *Salmonella Gallinarum* strain in which only the entire SPI-2 gene cluster is deleted (SG3-d2), a *Salmonella Gallinarum* strain in which both SPI-1 and SPI-2 gene clusters are integrally deleted (SG3-d1d2) and a *Salmonella Gallinarum* strain in which all of the four virulence gene clusters (SPI-1, SPI-2, *spvRABCD, and faeHI*) are integrally deleted (SG3-d4). SG3-d2 is deposited under accession No. KCCM11009P, SG3-d1d2 under accession No. KCCM11010P, and SG3-d4 under accession No. KCCM11011P.

Studies on the independent deletion of individual genes of the gene clusters have been reported (Hapfelmeier S et al., J Immunol, (2005); 174(3):1675-1685, Brumme S et al., Vet Microbiol, (2007); 124(3-4):274-285, Desin TS et al., Infect Immun, Jul (2009);2866-2875), but avirulent *Salmonella* strains developed by integrally inactivating two or more entire gene clusters had not been disclosed prior to the study of the present inventors. The *Salmonella Gallinarum* strain was named *Salmonella Gallinarum* SG2293-d2 when only the entire SPI-2 gene cluster is deleted, and SG2293-d1d2 when both SPI-1 and SPI-2 were integrally deleted. Further, it was named SG2293-d4 upon the deletion of all entire SPI-1, SPI-2, *spvRABCD,* and *faeHI.* The terms "SG2293-d2" and "SG3-d2", "SG2293-dld2" and "SG3-dld2", and "SG2293-d4" and "SG3-d4" can be used herein interchangeably.

To ascertain the avirulence thereof, the strains prepared by deletion of entire virulence gene clusters according to the present invention were assayed for the efficiency of invasion into avian epithelial cells and for disease outbreak and mortality (%) upon infection into poultry. Preferably, the *Salmonella Gallinarum* strains in which the virulence gene clusters had been deleted were allowed to invade avian epithelial cells so that invasion efficiency could be measured. Also, the strains were injected into brown egg layers to measure mortality.

In accordance with another aspect thereof, the present invention provides an avirulent modified *Salmonella* strain in accordance with claim 1 for use in producing *Salmonella*-specific lytic bacteriophages and a method for producing phages using the same.

ΦCJ1 (US 20100135962), a *Salmonella-specific* phage, was used to examine the bacteriophage productivity of the avirulent modified *Salmonella Gallinarum* strains. The phage shows a specific bactericidal activity against *Salmonella Gallinarum* and *Salmonella pullorum,* belongs to the morphotype group of the family *Siphoviridae* B1, characterized by isometric capsid and long non-contractile tail, and has a total genome size of 61 kb and major structural proteins with a size of 38 kDa and 49 kDa.

The method for producing a bacteriophage in accordance with the present invention comprises culturing the avirulent modified *Salmonella Gallinarum* strains according to claim 1 in a medium, inoculating a bacteriophage into the medium, and recovering the bacteriophage. In this regard, the phage may be produced briefly using a plate or on a mass scale using broth. In the case of production using a plate, a bacteriophage is inoculated at a suitable ratio into bacteria when the bacteria enter a log phase, mixed with top agar, and poured onto a plate. When phage plaques appear, the top agar fractions are collected and centrifuged, followed by filtering the supernatant to afford a phage stock. For mass production as a broth, a mixture of phages and bacteria is prepared in the same manner as in plate production, and incubated for 5 hours in fresh broth, instead of in top agar.

In accordance with a further aspect thereof, the present invention provides a pharmaceutical composition for the prevention or treatment of fowl typhoid, comprising the avirulent *Salmonella* strain in accordardance with claim 1 as an active ingredient. According to the invention it is further provided a vaccine according to claim 11 for the prevention or treatment of fowl typhoid, formulated with the avirulent *Salmonella* strain.

The composition or vaccine can comprise optionally a pharmaceutically acceptable carrier. The term "pharmaceutically acceptable carrier" as used herein, refers to a carrier or diluent which does not deteriorate the biological activity and property of the active ingredient and which does not irritate the subject. Preparations intended for oral administration can be in the form of tablets, troches, lozenges, aqueous or oily suspensions, powders, granules, emulsions, hard or soft capsules, syrups, elixirs, etc. In regards to the oral forms such as tablets and capsules, the active ingredient may be formulated in combination with a binder such as lactose, saccharose, sorbitol, mannitol, starch, amylopectin, conjugate such as cellulose or gelatin, an excipient such as dicalcium phosphate, a disintegrant such as corn starch or sweet potato starch, or a lubricant such as magnesium stearate, calcium stearate, sodium stearylfumarate or polyethylene glycol wax. As for capsules, they may further comprise a liquid carrier such as fatty oil.

The composition of the present invention can be formulated into preparations for non-oral administration, such as subcutaneous injections, intravenous injections, or intradermal injections. For this, the composition of the present invention may be mixed with a stabilizer or buffer in water to prepare a solution or a suspension which is then formulated into unit doses such as ampules or vials.

As used herein, the term "vaccine" refers to a biological preparation that improves immunity to a particular disease by inducing the formation of an antibody upon injection into the body, a preparation containing an antigen, e.g., killed or attenuated forms of a disease-causing microorganism. Vaccines may be prepared from killed pathogens. There are also live vaccines, but with the virulence thereof attenuated. The avirulent modified *Salmonella Gallinarum* strains of the present invention have the same antigenic proteins as those of the wild-type, but are greatly decreased in virulence compared to the wild-type, so that they can be used as live vaccines prophylactic of fowl typhoid.

In accordance with the present invention it is further provided a feed additive comrpising the avirulent modified *Salmonella Gallinarum* according to claim 1. When applied to poultry, the feed additive of the present invention serves as a live vaccine that prevents fowl typhoid.

The feedstuff of the present invention may be prepared by mixing feedstuff with the avirulent modified *Salmonella Gallinarum* strain as it is or in the form of a feed additive. In the feedstuff, the avirulent modified *Salmonella Gallinarum* strain may be in a liquid or dry state. The dry state can be accomplished by various drying methods including, but not limited to, pneumatic drying, spontaneous drying, spray drying and freeze drying. In addition to the avirulent modified *Salmonella Gallinarum* strain of the present invention, the feedstuff of the present invention may further comprise a typical additive useful for improving the preservation of the feedstuff.

The feedstuff comprising the avirulent modified *Salmonella Gallinarum* strain of the present invention may be vegetable matter such as a cereal, nut, a by-product of food processing, millet, fiber, pharmaceutical by-product, a vegetable oil, starch, oil seed meals and cereal remnants, or animal matter such as proteins, minerals, fats, mineral oils, unicellular proteins, animal planktons and leftover food, etc.

Examples of the feed additive comprising the avirulent modified *Salmonella Gallinarum* strain of the present invention include, but are not limited to, various agents for preventing quality deterioration and improving utility, such as binders, emulsifiers, preservatives, amino acids, vitamins, enzymes, probiotics, flavoring agents, non-protein nitrogen compounds, silicates, buffer, colorants, extracts, oligosaccharides, etc. Also, a mixing agent may be within the scope of the feed additive.

The composition of the present invention may be administered to animals in the form of a pharmaceutical preparation for animals, or by being mixed with feedstuff or water. Preferably, it is mixed in the form of a feed additive with feedstuff before administration.

So long as it allows the composition of the present invention to reach tissues or cells of interest, any administration route, such as non-oral, intraartery, intradermal, transdermal, intramuscular, intraperitoneal, intravenous, subcutaneous, oral or intranasal route, may be taken.

It will be apparent to those skilled in the art that the suitable total daily dose may be determined by an attending physician within the scope of medical judgment. The specific therapeutically effective dose level for any particular patient may vary depending on a variety of factors, including the kind and degree of desired reaction, the specific composition, including the use of any other agents according to the intended use, the patient's age, weight, general health, gender, and diet, the time of administration, the route of administration, and rate of the excretion of the composition; the duration of the treatment other drugs used in combination or coincidentally with the specific composition; and like factors well known in the medical arts. Typically, the composition may be administered at a daily dose of from $10^4$ to $10^8$ CFU once or in a divided dosage manner.

[Advantageous Effects]

The avirulent modified *Salmonella Gallinarum* strains according to the present invention, prepared by deletion of entire virulence gene cluster(s), are useful as host cells for effectively producing *Salmonella-specific* lytic bacteriophages on an industrial scale with the advantage of cost saving. The avirulent modified *Salmonella Gallinarum* strains simplify the purification process used to remove toxicity after bacteriophage production, thus greatly reducing the production cost and solving the safety problem of the products. In addition, the modified strains can be used as live vaccines that guarantee higher immunological effects and safety than do conventional vaccines.

[Description of Drawings]

The above and other objects, features and other advantages of the present invention will be more clearly understood from the following detailed description taken in conjunction with the accompanying drawings, in which:

FIG. 1 is a schematic diagram showing virulence genes of avian *Salmonella (Salmonella* pathogenicity island-1, *Salmonella* pathogenicity island-2, *spvRABCD,* and *faeHI*) and sites corresponding to primers for inactivating the virulence genes; and

FIG. 2 is a graph showing the efficiency of the *in vitro* invasion into avian epithelial cells of the virulence gene-inactivated *Salmonella Gallinarum* strains (SG3-d1, SG3-d2, SG3-dld2, and SG3-d4), together with controls wild-type *Salmonella Gallinarum* SG2293), *Salmonella Gallinarum* live vaccine (SG9R), and non-pathogenic *E. coli* (MG1655). Invasion efficiency is expressed as a percentage of the count of microorganisms within cells divided with the count of microorganisms within a culture medium. The microorganisms were used at a concentration of $8.0x10^7$ cfu per well.

[Best Mode]

Hereinafter, the present invention will be described in more detail with reference to Examples. However, these Examples are for illustrative purposes only, and the invention is not intended to be limited by these Examples.

[Mode for Invention]

## EXAMPLE 1: Screening of Target Genes to be Inactivated through Comparison of *Salmonella Gallinarum* Virulence Genes

The first step of preparing avirulent avian *Salmonella* strains was the screening of target virulence genes to be inactivated. *Salmonella* Pathogenicity Island-1 (SPI-1), and *Salmonella* Pathogenicity Island-2 (SPI-2), both of which are type three secretion system gene clusters essential for the delivery of the pathogenicity of *Salmonella,* and *spvRABCD* and *faeHI,* both of which are genes on virulence plasmids, were determined as target genes, and the NCBI data base was searched for the nucleotide sequences thereof the target genes (*Salmonella enterica subsp. enterica serovar Gallinarum str.* 287/91, NC 011274). As the nucleotide sequence of *spvRABCD* of *Salmonella Gallinarum* had not yet been disclosed, primers were synthesized with reference to the nucleotide sequence of the same name gene of *Salmonella typhimurium* (*Salmonella typhimurium* LT2 plasmid pSLT, NC 003277), which has high nucleotide sequence homology with *Salmonella Gallinarum.* As for the *faeHI* operon, the information of its nucleotide sequence was obtained from *Salmonella Gallinarum* virulence plasmid minor fimbrial subunit genes (AF005899).

## EXAMPLE 2: Preparation of Avirulent Modified Strains by Inactivation of Virulence Genes of *Salmonella Gallinarum* and by Integration of the Inactivated Sites

### 2-1. Inactivation of Virulence Genes of *Salmonella Gallinarum*

To delete TTSS-related virulence genes of the wild-type *Salmonella* Gallinarum (SGSC No.2293) as determined in Example 1, the one-step deletion method using lamda Red recombinase, developed by Datsenko KA *et al.,* (Datsenko KA et al, PNAS, (2000);97(12):6640-6645), was employed.

A chloramphenicol resistant gene of pKD3 was used as an antibiotic marker for identifying insertion into a target site of a chromosome. Using a pair of the primers SPI-1-P1 (SEQ ID NO: 5) and SPI-1-P2 (SEQ ID NO: 6) of Table 2, which correspond to 50 bp of 5 flanking region of the *avrA* and 50 bp of 3 flanking region of the *invH* gene, wherein SPI-1 comprising from *avrA* to *invH* is the target for deletion, and a part of the chloramphenicol resistant gene of pKD3, a polymerase chain reaction (hereinafter referred to as "PCR") was performed [Sambrook et al, Molecular Cloning, a Laboratory Manual (1989), Cold Spring Harbor Laboratories], with pKD3 as a template. The obtaind PCR product was a gene fragment about 1100 bp long.

In this regard, a PCR HL premix kit (BIONEER) was used and 30 cycles of denaturation at 94°C for 30 sec, annealing at 55°C for 30 sec and elongation at 72°C for 1 min was conducted. The PCR product was separated in 0.8% agarose gel by electrophoresis and eluted at a desired band size.

According to the method of Datsenko KA *et al.,* the 1100 bp-long gene fragment was introduced into pKD46-transformed, competent wild-type *Salmonella Gallinarum,* which was then spread over LB plates containing chloramphenicol (30 mg/L). As for the resulting transformant, its gene was examined by PCR using a pair of the primers SPI-1-P3 (SEQ ID NO: 7) and SPI-1-P4 (SEQ ID NO: 8), which correspond to regions about 1 kb distant from both ends of the deletion target gene. The PCR product thus obtained was 3100 bp long, indicating that the SPI-1 gene cluster was inactivated.

The resulting strain was cultured at 37°C, a condition of removing the pKD46 vector, to select a strain that could not grow on an LB plate containing ampicillin (100 mg/L).

Subsequently, the antibiotic marker inserted into the inactivated gene cluster was removed by transformation with pCP20. The removal of the antibiotic marker was identified by PCR using the primers SPI-1-P3 & SPI-1-P4. The resulting PCR product was 2000 bp long, also indicating the inactivation.

Afterwards, the strain which was now free of the antibiotic marker was cultured at 42°C (a condition for removing pCP20) to select a strain that could not grow on an LB plate containing ampicillin. The SPI-1 gene cluster-inactivated strain thus obtained was named SG3-d1 (*Salmonella Gallinarum* SG2293::ΔSPI-1). The SG3-d1 strain was deposited with the Korean Culture Center of Microorganisms, YuRim B/D Hongje-dong, Seodamun-gu, Seoul, Korea on July 2, 2009 under accession No. KCCM11009P.

SPI-2, *spvRABCD* and *faeHI* gene clusters were also inactivated in the same manner as in the SPI-1 gene cluster. The resulting gene cluster-inactivated strains were named SG3-d2 *(Salmonella Gallinarum* SG2293::ΔSPI-2, accession No.KCCM11009P), SG3-ds (*Salmonella Gallinarum* SG2293::Δspv), and SG3-df (*Salmonella Gallinarum* SG2293::Δfae), respectively. Primers used for deleting genes and for identifying gene deletion are summarized in Table 2, below.

[Table 2]

| Primers for deletion of SPI-1 gene from chromosome | |
|---|---|
| SPI-1-P1 (SEQ ID NO: 5) | TTATGGCGCTGGAAGGATTTCCTCTGGCAGGCAACCT TATAATTTCATTAGTGTAGGCTGGAGCTGCTTC |
| SPI-1-P2 (SEQ ID NO: 6) | ATGCAAAATATGGTCTTAATTATATCATGATGAGTTC AGCCAACGGTGATCATATGAATATCCTCCTTAG |
| Primers for Deletion of SPI-2 Gene from Chromosome | |
| SPI-2-P1 (SEQ ID NO: 9) | ACCCTCTTAACCTTCGCAGTGGCCTGAAGAAGCATAC CAAAAGCATTTATGTGTAGGCTGGAGCTGCTTC |
| SPI-2-P2 (SEQ ID NO: 10) | ACTGCGTGGCGTAAGGCTCATCAAAATATGACCAATG CTTAATACCATCGCATATGAATATCCTCCTTAG |
| Primers for Deletion of *spvRABCD* gene from virulence plasmid | |
| spv-P1 (SEQ ID NO: 13) | GTGCAAAAACAGGTCACCGCCATCCTGTTTTTGCACA TCAAA ACATTTTTGTGTAGGCTGGAGCTGCTTC |
| spv-P2 (SEQ ID NO: 14) | TTACCCCAACAGCTTGCCGTGTTTGCGCTTGAACATA GGGAT GCGGGCTTCATATGAATATCCTCCTTAG |
| Primers for Deletion of *faeHI* gene from virulence plasmid | |
| fae-P1 (SEQ ID NO: 17) | TTACCGATATTCAATGCTCACCGCCAGGGAGGTATGC CAGCG GGACGGTAGTGTAGGCTGGAGCTGCTT C |
| fae-P2 (SEQ ID NO: 18) | ATGAAAATAACGCATCATTATAAATCTATTATTTCCG CC CTGGCCGCGCTCATATGAATATCCTCCTTAG |
| Primers for identification of SPI-1 gene deletion from chromosome | |
| SPI-1-P3 (SEQ ID NO: 7) | ATGTTCTTAACAACGTTACTG |
| SPI-1-P4 (SEQ ID NO: 8) | AGGTAGTACGTTACTGACCAC |
| Primers for identification of SPI-2 gene deletion from chromosome | |
| SPI-2-P3 (SEQ ID NO: 11) | TGTTCGTACTGCCGATGTCGC |
| SPI-2-P4 (SEQ ID NO: 12) | AGTACGACGACTGACGCCAAT |
| Primers for *spvRABCD* gene deletion from virulence plasmid | |
| spv-P3 (SEQ ID NO: 15) | GACCATATCTGCCTGCCTCAG |
| spv-P4 (SEQ ID NO: 16) | CAGAGCCCGTTCTCTACCGAC |
| Primers for *faeHI* gene deletion from virulence plasmid | |
| fae-P3 (SEQ ID NO: 19) | CAGGCTCCCCTGCCACCGGCT |
| fae-P4 (SEQ ID NO: 20) | CAGGCCAACTATCTTTCCCTA |

### 2-2. Integration of Type III Secretion System-Related Virulence Genes Inactivation

To integrally inactivate the gene clusters in one strain, the SG3-d1 strain was sequentially subjected to the inactivation of SPI-2, *spvRABCD,* and *faeHI* gene clusters, using a method similar to that of Example 2-1.

To begin with, PCR was performed using the primers SPI-2-P1 (SEQ ID NO: 9) and SPI-2-P2 (SEQ ID NO: 10) for the purpose of inactivating the SPI-2 cluster gene, with pKD4 serving as a template, resulting a 1600 bp gene fragment. This PCR product was introduced into the SG3-d1 strain in which pKD46 vector remained (Example 1-2), followed by spreading the bacteria over an LB plate containing kanamycin (50 mg/L). As for the resulting transformant, its gene was examined by PCR using a pair of the primers SPI-2-P3 (SEQ ID NO: 11) and SPI-2-P4 (SEQ ID NO: 12), which correspond to both flanking regions of the deletion target gene. The PCR product thus obtained was 3600 bp long, indicating that the SPI-2 gene cluster was inactivated.

The resulting strain was cultured at 37°C, a condition of removing the pKD46 vector, to select a strain that could not grow on an LB plate containing ampicillin (100 mg/L).

Subsequently, the antibiotic marker inserted into the inactivated gene cluster was removed by transformation with pCP20. The removal of the antibiotic marker was identified by PCR using the primers SPI-1-P3 & SPI-1-P4 in case of SPI-1 and the primers SPI-2-P3 & SPI-2-P4 in case of SPI-2. The resulting PCR product was 2000 bp long, also indicating that the inactivation had taken place.

Afterwards, the strain free of the antibiotic marker was cultured at 42°C(a condition for removing pCP20) to select a strain that could not grow on an LB plate containing ampicillin. The SPI-1 and SPI-2 gene cluster-inactivated strain thus obtained was named SG3-d1d2 (*Salmonella Gallinarum* SG2293::ΔSPI-1ΔSPI-2, accession No. KCCM11010P). The SG3-d1d2 strain was deposited with the Korean Culture Center of Microorganisms, YuRim B/D Hongje-dong, Seodamun-gu, Seoul, Korea on July 2, 2009 under accession No.KCCM1 1010P.

In the SG-dld2 strain, *spvRABCD* and *faeHI* gene clusters were further inactivated. To this end, the *spvRABCD* gene cluster (the kanamycin-resistant gene of pKD4 was used as an antibiotic marker) was inactivated in the same manner as in the inactivation of SPI-1 in Example 1-2, while the inactivation of the *faeHI* gene cluster (the chloramphenicol-resistant gene of pKD3 was used as an antibiotic marker) was conducted in the same manner as in the inactivation of SPI-2 in the SPI-1-inactivated strain. As for the resulting transformants, their genes were examined by PCR using the primer set spv-P3 (SEQ ID NO: 15) and spv-P4 (SEQ ID NO: 16) for *spvRABCD* deletion, and the primer set fae-P3 (SEQ ID NO: 19) and fae-P4 (SEQ ID NO: 20) for *faeHI* deletion, which correspond to regions about 1 kb distant from both ends of the respective deletion target genes. The PCR products thus obtained were 3600 bp, 3100 bp long respectively, indicating that the *spvRABCD* and *faeHI* gene clusters were inactivated. The resulting strain was cultured at 37°C, a condition for removing the pKD46 vector, to select a strain that could not grow on an LB plate containing ampicillin (100 mg/L). The *Salmonella Gallinarum* strain in which all of the four gene clusters SPI-1, SPI-2, *spvRABCD* and *faeHI* were integrally inactivated was named SG3-d4 (*Salmonella Gallinarum* SG2293::ΔSPI-1 ΔSPI-2Δ*spv*R*ABCD*Δ*faeHI*) and deposited under accession No. KCCM11011P with the Korean Culture Center of Microorganisms, YuRim B/D Hongje-dong, Seodamun-gu, Seoul, Korea on July 2, 2009.

### 2-3. Sequencing of *Salmonella Gallinarum spvRABCD* Operon

Nowhere has the genetic information on *spvRABCD* of *Salmonella* Gallinarum (SGSC No.2293) been disclosed yet. Its nucleotide sequence was analyzed in the present invention. For this, primers were synthesized as summarized in Table 3, below.

[Table 3]

| | |
|---|---|
| spv-S1 (SEQ ID NO: 21) | GGTCAATTAAATCCACTCAGAA |
| spv-S2 (SEQ ID NO: 22) | ACGGGAGACACCAGATTATC |
| spv-S3 (SEQ ID NO: 23) | TTCAGTAAAGTGGCGTGAGC |
| spv-S4 (SEQ ID NO: 24) | CCAGGTGGAGTTATCTCTGC |
| spv-S5 (SEQ ID NO: 25) | ACTGTCGGGCAAAGGTATTC |
| spv-S6 (SEQ ID NO: 26) | TTTCTGGTTACTGCATGACAG |
| spv-S7 (SEQ ID NO: 27) | TCCAGAGGTACAGATCGGC |
| spv-S8 (SEQ ID NO: 28) | GAAGGAATACACTACTATAGG |
| spv-S9 (SEQ ID NO: 29) | GTGTCAGCAGTTGCATCATC |

(continued)

| | |
|---|---|
| spv-S10 (SEQ ID NO: 30) | AGTGACCGATATGGAGAAGG |
| spv-S11 (SEQ ID NO: 31) | AAGCCTGTCTCTGCATTTCG |
| spv-S12 (SEQ ID NO: 32) | AACCGTTATGACATTAAGAGG |
| spv-S13 (SEQ ID NO: 33) | TAAGGCTCTCTATTAACTTAC |
| spv-S14 (SEQ ID NO: 34) | AACCGCTTCTGGCTGTAGC |
| spv-S15 (SEQ ID NO: 35) | CCGTAACAATGACATTATCCTC |

The analysis result is given in SEQ ID NO: 3.

**EXAMPLE 3: Assay of Virulence Gene Clusters-Inactivated *Salmonella Gallinarum* SG3-d4 for Avirulence by Measurement of Invasion Efficiency into Avian Epithelial Cell**

*Salmonella Gallinarum* and *Salmonella pullorum,* which are unique *Salmonella* species due to the lack of a motile flagella, are specifically infected to avian cells and can invade other animal cells but at very low efficiency. In this example, an *in vitro* cell invasion assay was conducted (Henderson SC et al, Infect Immun, (1999); 67(7):3580-3586) on the avian epithelial cell line BAT (Budgerigar Abdominal Tumor), provided from MD. Lee, Georgia University. The modified *Salmonella Gallinarum* strains SG3-d1d2 and SG3-d4, developed by the above-described gene deletion method, were expected to invade the host cell with very low efficiency based on the reduced level of TTSS-related protein. A recent research review on the infection mechanisms of pathogenic microorganisms has it that even when only a specific gene of SPI-1 is deleted, the *Salmonella* strain shows a decrease in invasion efficiency into epithelial cells (Lostroh CP et al, Microbes Infect, (2001); 3(14-15):1281-1291).

In the present invention, TTSS-related gene deletion was proven to lead to a decrease in virulence by measuring the efficiency of the invasion of the avian modified *Salmonella* strains into the avian epithelial cell line BAT.

Invasion efficiency into avian epithelial cells was measured on 24-well plates in triplicate, and the mean values of three measurements were given. The BAT cell line was cultured at 37°C in DMEM supplemented with 10% fetal bovine serum, 1 mM glutamine, 100 IU/mL penicillin and 100 $\mu g$/mL streptomycin under the condition of 5% $CO_2$. The BAT cell line was seeded at a density of 2.5x$10^5$ cells/well into 24-well plates and incubated at 37°C for 1-2 days in a 5% $CO_2$ incubator to form monolayers of cells. After distribution of the cell and incubation for one day, the culture medium was changed out with antibiotic-free DMEM. For comparison of invasion efficiency, wild-type *Salmonella Gallinarum* SG3 (SGSC#: 2293), the virulence gene clusters-inactivated *Salmonella Gallinarum* strains SG3-d1, SG3-d2, SG3-dld2 and SG3-d4, and SG9R, which is a commercially available live vaccine, were employed, with the non-pathogenic *E. coli* MG1655 serving as a control.

After being primarily seed cultured, all of test bacteria were vigorously incubated for 4-5 hours in a main LB medium, and the cultures were diluted to $OD_{600}$= 1.0. To 200 $\mu l$ of the animal cells incubated in the antibiotic-free medium, 200 $\mu l$ of each of the diluted culture was added so that the bacteria were aliquoted at a concentration of 2.0 x $10^8$ CFU/mL per well. The plates were incubated at 37°C for one hour in a 5% $CO_2$ atmosphere to allow the bacteria to penetrate into the epithelial cells. Thereafter, the medium was aspirated off and the plates were washed with IX PBS to remove remaining microorganisms. Then, the epithelial cells were incubated at 37°C for 2 hours in the presence of 50 $\mu g$/mL gentamycin in a 5% $CO_2$ incubator to clear the microorganisms remaining outside the cells. The antibiotic was removed by washing with IX PBS. To examine the microorganisms which succeeded in penetrating into the epithelial cells, the animal cells were lyzed for 15-30 min in 500 $\mu l$ of 0.1% Triton X-100. The cell lysates were spread over LB plates and incubated overnight at 37°C so that the microorganisms that had grown could be counted. To calculate the invasion efficiency, 200 $\mu l$ of the microorganism culture with $OD_{600}$= 1.0 was also incubated.

Invasion Efficiency (%) = Count of Microorganisms invaded to Cell/Count of Microorganisms within Culture Medium ($OD_{600}$= 1.0) x 100

The BAT cell invasion efficiencies of the four transformed *Salmonella Gallinarum* strains prepared by the inactivation of virulence gene clusters were calculated. Of them, the modified strain in which only the SPI-1 gene cluster, responsible for cell invasion mechanism, was inactivated, was decreased in invasion efficiency by 84% compared to the wild-type. The SG3-dld2 modified strain with the deletion of both SPI-1 and SPI-2 and the SG3-d4 modified strain with the deletion of all the four gene clusters were found to decrease in cell invasion efficiency by approximately 89% and 91%, respectively, compared to the wild-type *Salmonella Gallinarum* (SG3). The modified strains of the present invention were also remarkably reduced in invasion ability, in comparison to that of the commercially available live vaccine Nobilis SG9R. These data demonstrated that the inactivation of TTSS-related gene clusters decreases the virulence of *Salmonella Gallinarum* (see Table 4 and FIG. 2).

[Table 4]

| | Strain | Property | Genotype | Index of Internalization (%) |
|---|---|---|---|---|
| Control Group | MG1655 | Avirulent *E. coli* | Wild type | 2% |
| | SG3 | Virulent *Salmonella Gallinarum* (Wild-type, SGSC No. 2293) | Wild type | 100% |
| | Nobilis SG9R | *Salmonella Gallinarum* Live vaccine (commercially available) | SG:: Δ*recA* | 67% |
| Test Group (avirulent *Salmonella Gallinarum*) | SG3-d1 | Virulence gene-deleted *Salmonella Gallinarum* | SG:: ΔSPI-1 | 16% |
| | SG3-d2 | Virulence gene-deleted *Salmonella Gallinarum* | SG:: ΔSPI-2 | 34% |
| | SG3-d1d2 | Virulence gene-deleted *Salmonella Gallinarum* | SG:: ΔSPI-1/ΔSPI-2 | 11% |
| | SG3-d4 | Virulence gene-deleted *Salmonella Gallinarum* | SG:: ΔSPI-1/ΔSPI-2/ Δspv/ Δfae | 9% |
| (SG3 100%= 0.36% invasion efficiency in practice) | | | | |

The avirulence of modified *Salmonella Gallinarum* strain SG3-d4 was confirmed by *in vitro* test which shows extremely low invasion efficiency into avian epithelial cells, and was reconfirmed in animal tests and the results are given in Example 4.

**EXAMPLE 4: Assay of *Salmonella Gallinarum* SG3-d4 for Avirulence by Measuring Mortality of Chickens**

The Research Institute of Veterinary Science, Seoul National University, was entrusted with this assay. One-week-old brown egg layers (Hy-Line chicken) were divided into many groups of 10 which were separated in respective chicken houses before infection with pathogens. No vaccine programs were used on the experimental animals after they hatched.

Five avian *Salmonella* strains including the wild-type *Salmonella Gallinarum* SG3 (SGSC#: 2293), the virulent gene cluster-inactivated *Salmonella Gallinarum* SG3-d2 and SG3-d4 (identified to decrease in virulence by *in vitro* invasion assay), the commercially available live vaccine Nobilis SG9R, and the non-pathogenic *E. coli* MG1655 were employed in the *in vivo* assay.

After being primarily seed cultured, the five strains were vigorously incubated for 4-5 hours to $OD_{600}$ = 1.0 in a main LB medium, and the concentration of each of the cell cultures was adjusted to $1.0 \times 10^8$ CFU/mL. The bacteria were subcutaneously injected at an adjusted dose into the chickens which were then monitored for two weeks for mortality. Subsequently, the chickens which were alive were autopsied to examine lesions and to isolate bacteria.

For the two weeks after artificial infection of the pathogens ($1.0 \times 10^8$ CFU/mL), the chickens infected with modified *Salmonella Gallinarum* (SG3) were observed and showed typical external syndromes such as low motility, blue diarrhea and low uptake of feedstuff, and looked to be dying. The mortality was not high, but an autopsy disclosed lesions in almost all of the chickens.

In contrast, the chicken group infected with the modified *Salmonella Gallinarum* strain (SG3-d4) were observed to actively move and not die although some of them had diarrhea during the two weeks. Also, they were found to have almost no lesions in the autopsy. Therefore, the modified *Salmonella Gallinarum* strain of the present invention was again proven to have greatly decreased virulence. The chicken groups infected with the modified SG3-d2 strain in which the gene responsible for primary invasion into host cells remains intact while the SPI-2 gene involved in systemic infection and survival over phagocytosis is inactivated, or with the modified SG3-ds strain in which the spv gene known to participate in pathogenicity is inactivated, were observed to have low or no mortality (%). Thus, even the inactivation of single gene clusters had a great influence on the reduction of pathogenicity (see Table 5).

[Table 5]

| | Strain | Property | Geno-type | Mortality (%) | Frequency of lesions in live birds (%) |
|---|---|---|---|---|---|
| Control Group | MG1655 | Avirulent *E. coli* | Wild-type | 0% | 20% (2/10) |
| | SG3 | Virulent *Salmonella Gallinarum* (Wild-type, SGSC No. 2293) | Wild-type | 20% | 88% (7/8) |
| | Nobilis SG9R | *Salmonella Gallinarum* Live vaccine (commercially available) | SG:: Δ*recA* | 0% | 40% (4/10) |
| Test Group (avirulent *Salmonella* Gallinarum) | SG3-d1 | Virulence gene-deleted *Salmonella Gallinarum* | SG:: ΔSPI-1 | 40% | 17% (1/6) |
| | SG3-d2 | Virulence gene-deleted *Salmonella Gallinarum* | SG:: ΔSPI-2 | 10% | 0% (0/9) |
| | SG3-ds | Virulence gene-deleted *Salmonella Gallinarum* | SG:: Δspv | 0% | 20% (2/10) |
| | SG3-d4 | Virulence gene-deleted *Salmonella Gallinarum* | SG:: ΔSPI-1/ ΔSPI-2/ Δspv/ Δfae | 0% | 10% (1/10) |

According to autopsy findings, the liver and spleen were swollen and weakened, with the significant frequency of greenish brown or bluish green liver lesions, in the chicken group infected with the wild-type *Salmonella Gallinarum* (SG3). Like the commercially available live vaccine Nobilis SG9R or the non-pathogenic *E. coli* MG1655, however, the virulent gene cluster-inactivated strains of the present invention (SG3-d1d2 and SG3-d4) were found to have almost no lesions, and were demonstrated to be harmless to chickens.

**EXAMPLE 5: Comparison of the Productivity of ΦCJ1 Bacteriophage Specific to modified *Salmonella Gallinarum* strains**

Ultimately, the development of avirulent *Salmonella* stains is to be appiled to the production of *Salmonella-specific* lytic bacteriophages. The modified *Salmonella* strains prepared in Example 2 were proven to have greatly attenuated virulence in Examples 3 and 4. Finally, ΦCJ1 (Korean Patent Application No. 10-2008-121500/US 20100135962), which specifically infects avian *Salmonella,* was used to examine a difference in bacteriophage productivity between the wild-type and the modified *Salmonella Gallinarum* strains.

The avian-specific bacteriophage ΦCJ1 was cultured on a mass scale, with the wild-type *Salmonella Gallinarum* strain (SG3) or the modified strain serving as a host cell. For this, each bacterial strain was cultured to an $OD_{600}$ of 0.5 ($2.5x10^{10}$ colony forming units (CFU)) in 50 mL of LB broth in a flask with agitation. ΦCJ1 was inoculated at $1.25x10^9$ PFU (Plaque Forming Unit) to form an MOI (Multiplicity of Infection) of 0.05, and allowed to stand for 20 min at 37°C, followed by additional incubation at 37°C for 4 hours. Chloroform was added in an amount of 2% of the final volume and shaken for 20 min. After passage of the supernatant through a 0.2 μm filter, the titer of ΦCJ1 was counted.

ΦCJ1 was produced at a titer of $6x10^{11}$ PFU/mL from the wild-type strain (SG3) and at a titer of $8x10^{10}$ PFU/mL from the modified *Salmonella Gallinarum* strain (SG3-d4). These data demonstrated that the modified strains prepared by inactivating virulence gene clusters have no problems with infection with bacteriophages and can be used as host cells for producing bacteriophages (see Table 6). In addition, ΦCJ2 (US 20100158870) and ΦCJ3 (US 20100166709), which were both developed by the same applicant, were produced using the modified strain as a host cell. The host cell was found to allow the production of ΦCJ2 at a titer of approximately $2x10^{10}$ PFU/mL and ΦCJ3 at a titer of approximately $5x10^9$ PFU/mL. Like ΦCJ1, ΦCJ2 and ΦCJ3 were produced from the modified strain of the present invention, without significant difference from the wild-type.

[Table 6]

| | Strain | Property | Genotype | Production Titer of ΦCJ1 (PFU/mL) |
|---|---|---|---|---|
| Control Group | SG3 | Virulent *Salmonella Gallinarum* (Wild-type, SGSC No. 2293) | Wild type | $6x10^{11}$ |
| Test Group (avirulent *Salmonella Gallinarum*) | SG3-d4 | Virulence Gene-Deleted *Salmonella Gallinarum* | SG3:: ASPI-1/ ASPI-2/ ΔspvΔfae | $8x10^{10}$ |

As described hitherto, the avirulent modified *Salmonella Gallinarum* strains, prepared by inactivating virulence genes, according to the present invention are useful as host cells for effectively producing *Salmonella*-specific lytic bacteriophages on an industrial scale with the advantage of costs saving. The avirulent modified *Salmonella Gallinarum* strains simplify the purification process used to remove toxicity after bacteriophage production, thus greatly reducing the production cost and solving the safety problem of the products. In addition, the avirulent modified strains can be used as live vaccines that guarantee higher immunological effects and safety than conventional vaccines.

Although the preferred embodiments of the present invention have been disclosed for illustrative purposes, those skilled in the art will appreciate that various modifications, additions and substitutions are possible, without departing from the scope of the accompanying claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
500 5-GA NAMDAEMUN-RO
CHUNG-KU, SEOUL
REPUBLIC OF KOREA

RECEIPT IN THE CASE OF AN ORIGINAL
issued pursuant to Rule 7.1 by the
INTERNATIONAL DEPOSITARY AUTHORITY
identified at the bottom of this page

I. IDENTIFICATION OF THE MICROORGANISM

| | |
|---|---|
| Identification reference given by the DEPOSITOR : *Salmonella gallinarum*-d2 | Accession number given by the INTERNATIONAL DEPOSITARY AUTHORITY: KCCM11009P |

II. SCIENTIFIC DESCRIPTION AND/OR PROPOSED TAXONOMIC DESIGNATION

The microorganism identified under I above was accompanied by:

☐ a scientific description

☐ a proposed taxonomic designation

(Mark with a cross where applicable)

III. RECEIPT AND ACCEPTANCE

This International Depositary Authouity accepts the microorganism identified under I above, which was received by it on July. 02. 2009. (date of the original deposit)[1]

IV. INTERNATIONAL DEPOSITARY AUTHORITY

| | |
|---|---|
| Name : Korean Culture Center of Microorganisms<br>Address : 361-221, Yurim B/D<br>Hongje-1-dong,<br>Seodaemun-gu<br>SEOUL 120-091<br>Republic of Korea | Signature(s) of person(s) having the power to represent the International Depositary Authority or of authorized official(s):<br>Date: July. 02. 2009. |

[1] Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authouity.

Form BP/4 Sole page

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
500 5-GA NAMDAEMUN-RO
CHUNG-KU, SEOUL
REPUBLIC OF KOREA

RECEIPT IN THE CASE OF AN ORIGINAL
issued pursuant to Rule 7.1 by the
INTERNATIONAL DEPOSITARY AUTHORITY
identified at the bottom of this page

| I. IDENTIFICATION OF THE MICROORGANISM | |
|---|---|
| Identification reference given by the DEPOSITOR : *Salmonella gallinarum*-d1d2 | Accession number given by the INTERNATIONAL DEPOSITARY AUTHORITY: KCCM11010P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authouity accepts the microorganism identified under I above, which was received by it on July. 02. 2009. (date of the original deposit)[1] | |
| IV. INTERNATIONAL DEPOSITARY AUTHORITY | |
| Name : Korean Culture Center of Microorganisms<br><br>Address : 361-221, Yurim B/D<br>Hongje-1-dong,<br>Seodaemun-gu<br>SEOUL 120-091<br>Republic of Korea | Signature(s) of person(s) having the power to represent the International Depositary Authority or of authorized official(s):<br><br>Date: July. 02. 2009. |

[1] Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authouity.

Form BP/4 Sole page

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

To. CJ CheilJedang Corporation
500 5-GA NAMDAEMUN-RO
CHUNG-KU, SEOUL
REPUBLIC OF KOREA

RECEIPT IN THE CASE OF AN ORIGINAL issued pursuant to Rule 7.1 by the INTERNATIONAL DEPOSITARY AUTHORITY identified at the bottom of this page

| I. IDENTIFICATION OF THE MICROORGANISM | |
|---|---|
| Identification reference given by the DEPOSITOR : *Salmonella gallinarum*-d4 | Accession number given by the INTERNATIONAL DEPOSITARY AUTHORITY: KCCM11011P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authouity accepts the microorganism identified under I above, which was received by it on July. 02. 2009. (date of the original deposit)[1] | |
| IV. INTERNATIONAL DEPOSITARY AUTHORITY | |
| Name : Korean Culture Center of Microorganisms<br>Address : 361-221, Yurim B/D<br>Hongje-1-dong,<br>Seodaemun-gu<br>SEOUL 120-091<br>Republic of Korea | Signature(s) of person(s) having the power to represent the International Depositary Authority or of authorized official(s):<br>Date: July. 02. 2009. |

[1] Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authouity.

Form BP/4 Sole page

<110> CJ CHEILJEDANG CORPORATION

<120> AVIRULENT MODIFIED SALMONELLA GALLINARUM STRAINS AND PHARMACEUTICAL COMPOSITION USING THE SAME

<130> OPA09048PCT

<150> US61/487,137
<151> 2011-05-17

<150> US13/274,854
<151> 2011-10-17

<160> 35

<170> KopatentIn 1.71

<210> 1
<211> 34934
<212> DNA
<213> Artificial Sequence

<220>
<223> Salmonella pathogenicity island-1

<400> 1

```
ttacgattta agtaaagact tatattcagc tatccttttt ttatgagcgg atatagagag      60
tttttatcg tttagcataa cggcattgtt atcgaatcgc tcataaagcg tttcattctt     120
tttgtttact atactgcttc ccgccgccgg attggcctcc acatattcat ttaatcgttg     180
tacgccttga gtatgtttgt aaaaactcac cggcagataa cggtctgctt tatcggacgg     240
gagaaaaggt tcttcaccac acagacgttc acaaatatta tcttcatgaa tttttactaa     300
gttcataaat tcaagctgaa gtttttggc gagcgccagg ctaaaaatac cgcattcaga     360
agagcttcgt tgaatgtcca gctcgaccat agcaaaataa caatcaggca gttgttcacg     420
ttcaagagct gctttggtcc tcaacgccag taaagcaggt ccaaaagcgc tacacgctgc     480
tggttcgaac aaaatcaccg atgtctttcc gtccataact ctaaaatcga cgactgaaat     540
atggatacct gaacttccca tatttacgag aaatcgggca gattcaacgc cttccattct     600
ggtctccttt atagaggaaa caagctcatg gactgacata acaaatttaa gatttaactc     660
tggatacttc ttattggcct gtgcaacaag aaaaggcatc atttcgagat cggtttcctc     720
gtaactgata tgaatccagc tgccatctat aatttcactt tccagacgct caacaataca     780
ggtcaatgct tcggtgttta gctccccgct gacgtcaggc tgaggcgata aactcgcccc     840
catattacga gtcgtaggac ttagcatact tttccctcca catgatagct cctgcaccga     900
aaatatcatc tttaactttt cagattcaat atattgcctg caaaaatatg cctcaatgat     960
tgagccaggc taccaaccac ctccggatga ttatatataa gaattactac tcaaaaaatc    1020
tttttataa taaaagctca acacatggtc ataaatgata aaaaatattt taattcattc    1080
```

ctaccgcaat cggtaacgcg caattatcgt caggtacagc agggttatgt gcaaaagcag 1140
tgcgctgtaa atgcgcgtct agtttcagtc cccggaacag cgatagcggt gaagagtcca 1200
tccccaaacg atacataacc ttcttacgat aaatactgac ggtttttgtt cccagaccaa 1260
atttttcgc cagttcaatt gccggatgtc cggaggataa taatatcagc agcgcatatt 1320
tcgcctgcgt gacaccggga ggtagattcc accaggcgta ttgattgata ttaaacaata 1380
cctcttccgg cgtctcgccg gcattaaaag catacgtagc agccacggtt ttcttttgtg 1440
gcctgtggca gaaacgcagc caggcttccg gaagacggag cttctctcgc tccgagcgga 1500
tagcgcagga tagttcgtct tttaaaacat aatccataac gccaaaatat tgcagcacac 1560
agcgatcgat ataatacaag cgatctgcca ctaccaaaac tttacggttc tgcaaccgcg 1620
tcagcaacgc atgaaaaaga taaacatgct catgcgggtt caaagctaaa atcagcccgg 1680
cgtccggcat atcggataga gaatgcaaaa gtgcggttaa tgagttacac gttttaacgc 1740
acttttccgg atatttttgc ttaaaaatag actgaagggc ataacaatta gtccagttaa 1800
taccgtatat aattacattt ctcatttatt tatccttttt tgaaaactga ccacagcttc 1860
ggtaatgatt tttcttcctg ggcgactact gcgcaagtag ataacgcctt cttactacaa 1920
aggtaataag accagatacg ttattacatg cgcaatgtcg ttaccgaaat gaattccttt 1980
tacaaatctg ataatgatta aatttactgt tttactttac tgtaatctct tagagtacaa 2040
cgattgcccg gcgcctggtg gccatgtatg tctgacaatg aacgctttcg attccctttc 2100
attaactaca tatcactggt gtagcgatac tgaaatatac actacgatta aaaaaatatt 2160
tggtatctgt aacgcaaaca gatagtaacg tttaaaataa tttcacaaat caatggttca 2220
tcgtacgcat aaagctaagc ggtgtaatct taaaatgccg tttaaaaata gcgataaaat 2280
aagaaggcgt atcatagcca cacatcgtcg cgacttgtga aatatttcca gcccccatac 2340
gtaataattt tatagcctga ttcatacgag catctaggta tattttgcta aaactcacct 2400
cttcagcggc cagttttcgc ttcagacttg atacgctcat aaacagcttt cccgccacct 2460
cagcctgtga ccatttgcgg gtgagatcgc tgataataat gttataaact ttctcttttg 2520
tcgtaatttt tattgctcgc tcaaggaaat caaacccacc gggcttacgt acaaatgccg 2580
atataagata catcaatgag aaatatgaat aatcatgatc atcaatactc acattactac 2640
aaacccgtgg acatgccaca ccatgcaaaa tagagtcaaa agtatcactc atccctggca 2700
acaagtccgc atgaaaaaaa tactttggtt tcgtttttaa tgatagctct cgatcattat 2760
agtttcttgt actgtaaaaa actttgtaga atttttgcat taagtcatag gaaacttcca 2820
gtgaagaaaa atcaatatgc ccttctattt cgctcatact aagcgtgatt gtttgatctt 2880
tttccaataa aaataaacac ggcgcagatt gttcgatgaa ctccccaaat tcgttttcaa 2940
ttcgcaaact gcctttatta agtttaaaca ataagcagtt tgcgacataa tagtctctta 3000

cgtcagctaa tccatttatt aatggaaatt tgttcggctg ttgaaggtga ttattgctaa 3060

tggcctcaac tgatttattc attgaaggca ataccatatt ttatcctgtg tgctataagg 3120

aactcaaaat cgttatattc ttataaacaa ataattaaaa ctcacagaga tgatttaaat 3180

ccgatttttt tattattata gccaataatt acattccaac gcgcgttcat ttcgtcacaa 3240

aaagataccc ttacaaactt tatgcacaat tttgtaatga aagcttacaa tattaatata 3300

atcatttcag aataaaacgg ctggcagaca tcttaataat ccatatacat caataagata 3360

gacacactgg catggtgcat tttctgcatt atttgctgat atatacacca taccttatca 3420

caaatcgcca gcaatggggg ttcaccagtc aattgcctct ttgttttccc cgcccgataa 3480

aataatctcc tgcatccagg aggtcatttg tgactgtgcg ttcattgtac caactaatac 3540

cccgtttaaa gcctcatata aatgggtgcc cggttcaact tttgctaaca tgttttgtag 3600

catagccgtt tgctgctcaa aagaaacaaa agccgaatca ccactgttag gatctttgaa 3660

ggcattcatc tcttgataaa tgctatcttt aagcgtttca gaagaggctg actcaggaag 3720

cgccagaagt cgttggtaga atgcatcata aagatcaacg tcgccgccat tgcttaaagg 3780

cgcgctatcc acattattca gcatagcggc cctggcactc aacgaaacca cacccgtcgc 3840

ttcagtatct gctgtcggga ccaaataaga agtcggaatc gtacccggta tcaccttata 3900

acctccgctt gcgtttttgt cttccattca tcaataagtg cgttaatggc gttatcagaa 3960

attgtccggc agtcttgtgg aagttcatca agatgatgct taatgacgcc tactgccgtt 4020

tcaacaaatt gttcaggtga aaattctgcg atctgatcgc cgcaactcat gataaagcgc 4080

tgttcctgat gatatttaag attaaaagtg cctggccagt tctccataag caacaccatc 4140

agtttttggt gatctttttt cgcattaact ggcagtgtta aaaaaagttg cccctcaggc 4200

ttatcgaaat cccttagcca ctcatccagg acggttaaaa gcgtttcggg atggtcgacc 4260

gcagctgaaa ataactcgcg ggcataaatc tgtatttttt ccatccactt ccaggccatt 4320

gtctgattat cagtaagata agcggcgacc tgctgtaacg cgtctatcat tccctgctcg 4380

taaccttcct gataggcgta catccgcaag gtctttgcct cttcttccgc ctctcgcaaa 4440

atacgcttag cccgttgatg cgcctgctgt tctaatcttt caatagagaa ataacgttcc 4500

agcgttttac gctttatcag tatcccctca acaggcgaaa gcggggacgg tattgggata 4560

tttttgagca tattgtaagg ccagtagcaa aattgacatt tctacagcat cctgcttcaa 4620

tgcctcctca ataaatggag gaaaaagcaa aggaaaacgc tgtgctaaag attcaggtaa 4680

aaattcattt agggcattta actgtgcata cccgacgcta agtaaaaacc ggtgattcgg 4740

cgccttattg cagacagata aacttgttcc ctgatgcatt gccaaaaatg cttgcgccca 4800

atccggcagg ccaagcaagg ctccctgcct tgccagatcg gctctcagtt tatggcaacc 4860

gagtaaatac gctacctgcg gcagtcggcg ccactgacgc agccacagct gcgtcagtga 4920
gttttgaata cactcctttt ctccgttctt aagccgccat gccgccagta ttaactcatt 4980
tgccgccgcc ctggcggcgg gtctgacaat catttccggc gctatctgca accgctgagg 5040
atggatatac gataacggat caaaaatgat tctttgccag ataatgggta atggctgcct 5100
attcatttga cgatttcgcc ttatcatcag ccgttatgcc tttcttattg cgggcataat 5160
ggtttttgta ataccagacg ccaaagcctg ctgacatcac ggataacaaa ataatcaaca 5220
caatccaact ggttgcaaaa gaattacgtt ttactggtgt gccgggagcc tgtaattggg 5280
catcagaacg ttctgacaac acaacagaaa tgttgtcata atccacatcg gcaaaactat 5340
tctttaagaa acgcttgata tcgctgatct gatgcgcaag cggcgaacct cgttcatata 5400
cggctaatgc cgacagatga acaggttttg gcggacggcc attttcacca gcatcaatat 5460
cataactaat atggaccctg gcggagagca cgccctccat cgtctgtaat gactgttcca 5520
gtcgctgttc aatagccgaa tataacctgg ccttttcagc tcgcggagac gataccagcg 5580
aatccgccgg gaacatctgc gctatttcca cccgtggccg gggaggaagc tgataagttt 5640
taatccagta caccgcagcg gtaaaatcag gctcagcaac ggtaatgcta tagcccaatt 5700
ttccgctatc aattttattc gcctctatat tgtgcatttg cagaacggca atgacctcat 5760
tagcctgttc ctggtccagt ccttttaaaa gatccttatc cttacagccg gcaagggtca 5820
ttaccagcag aaaggtatat agatatcgac gaatcatgag cgtaatagcg tttcaacagc 5880
cccgactcct ttacgagtaa gggtacttac catagaaaca tacaggttat aatctgaaat 5940
catctcttgc gaaatagcca gctctttagg atccgtcacc agattagggt cctcaatcct 6000
gttggtaatc gtctgtttat ccacagccgt ggcaatcgcc gaaccagaaa aagcctggag 6060
tagccggtca tccagcgaga caatgtccgt ctccatagac ctgatattga ccgcctgccc 6120
tataacggca ttctctggga caatagttgc aatcgacata atccacctta taactgatta 6180
acggaagttc tgaataatgg cagcatcaat atccttaaag acttttaccg tgttcgattg 6240
cgcgttacgg tacaagttat attccgagag cttactctga tacgccgcca gtagcgccgg 6300
atcggagggt tttgctgcta atttatccag cgcctctgtt acctgcgttt gtagattatc 6360
aacgcccgta tcaaattttg ctgagacgtc atccagatag cctgaccaag gtgttgccat 6420
aatgacttcc ttatttacgt taaattaaag tgggcttggg aaataccaat ggcctgggct 6480
cattttgata taaccttccg ccccgtactg aaatgagcgc cccttgagcc agtcatcttt 6540
taattcgatc gcaaactgca catagcgtcc tccccatgtg cggtaatagc tatcgacaaa 6600
ttgacgggct ctgagtattt ctacatcatc gagcgccccc tgaataacaa acgttacgcc 6660
cccttatga ttcctgcggg aataaggtaa cgcctgctgt tttagccccg cttccgcctg 6720
gcctgctgcg gtaacatcgt ccatcaacgt gatgttaacc gaatccgcgt aaggcattag 6780

cgctctcagc ttttgactta acatctcgag ctctttcttg ctcatcgtgt ttcgctggcg 6840
gcttagccag aaaacgggtt tacgcggctc atcgaaatga atccgataat aagccagctg 6900
cggataatag gtatccagcc agatagagat acgcttattt tcttcgtttt cgttaatcac 6960
tcgcgcattt ttatcataat cgcccctcgc taaaacctga cgagcccaca gcgtatctct 7020
ttcattttgc gcagcgacat agagcatttt gtcccggcct ggcaacacct gaaaacgctc 7080
cttctcctgc cccaataacg aatcgagctc tgcggcctgc cgctgcggcg agttaagtat 7140
ccataacgtc cccacagtcc caattcccaa tataaaaaac ccggccagtg ctgctacaat 7200
tccgttttta aaacgcggct cgttcttttt tgcagacgtt tctaacttct caggctgctc 7260
gggcacccac ggctcgcttt ccgggcgaat caggataagc aattcaccga cctgtattgg 7320
cgtatttaat tgcaccgaac gagattcaga atttccttct ttcagctcat ggagtataat 7380
ttcggtcgta tccgtatcca cctggatttc aaaatttact ccgccatggt ccagcgggat 7440
aaaaaagcta tcggcaggta tatcagggag ttggcctgaa gcagtgagcg catcactctg 7500
acctaccaca aagagtgttc ggcctgtcag caatggaaac tcacagccgt tcagtgagct 7560
gttaagtaat cgaactatgt atggcccagg gcttgttatc gtcttctctt ttgatgtttc 7620
catatatact gttagcgatg tctgtcgttc tcgatagcag cagattaccg cacaggacac 7680
agggattcct gatgaaaata gaatgaaaag tgagaaataa aatcaattta ttctgtataa 7740
tgcgtctcaa cacatattaa aagaaccatc atccccattg ggcttaaac tactgtagat 7800
aaattaccca aatttgggtt cttttggtgt aacaatcaga ccattgccaa cacacgctaa 7860
taaagagcat ttacaactca gattttttca gtaggatacc agtaaggaac attaaaataa 7920
catcaacaaa gggataatat ggaaaatgta acctttgtaa gtaatagtca tcagcgtcct 7980
gccgcagata acttacagaa attaaaatca cttttgacaa atacccggca gcaaattaaa 8040
agtcagactc agcaggttac catcaaaaat ctttatgtaa gcagtttcac tttagtttgc 8100
tttcggagcg gtaaactgac gattagcaat aatcacgata cgatttactg tgacgaacct 8160
gggatgttgg tgctcaaaaa agagcaggta gttaacgtga cgcttgaaga ggtcaatggc 8220
cacatggatt tcgatatact cgagataccg acgcaacgac ttggtgctct ctatgcactt 8280
atcccaaacg agcagcaaac caaaatggcg gtacccacag agaaagcgca gaaaatcttc 8340
tatacgcctg actttcctgc cagaagagag gtatttgaac atctgaaaac ggcgttctcc 8400
tgtacgaagg atacaagcaa aggttgcagt aactgtaaca acaaaagttg tattgaaaat 8460
gaagagttaa ttccttattt tctgctgttc ctgcttactg cttttctccg actcccggag 8520
agttatgaga tcatccttag ctcggctcag ataacgttaa aggagcgcgt ttacaacatt 8580
atatcttcgt cacccagtag acagtggaag cttacggatg ttgccgatca tatatttatg 8640

agtacgtcaa cgctcaaacg gaaacttgca gaagaaggta ccagctttag cgacatctac 8700

ttatcggcaa gaatgaatca ggcagcaaaa cttttacgca taggcaacca taatgttaat 8760

gctgtagcat taaaatgtgg ttatgatagc acgtcctact tcattcaatg tttcaaaaaa 8820

tattttaaaa ctacgccatc gacattcata aaaatggcga accattaaca ttttttgtat 8880

ctgtcactta agtaaagatt tttattaaaa ttgtaataat ttaaaattca gactgcgcat 8940

taacacgctc tatcaggatg ggaggctatt caatatcatt gttctgtccg gaagacagct 9000

tatactgata tctctggtaa tttaaagtaa ggctgattat ataacacgat ttttgtgaac 9060

ttgtcatcgc tatgatgact ggtaaaacga tattgcctta ttcacagcgt aagaattcgt 9120

ccagatgaca ctatctcctt ccggctttaa ccctgtggat taaggccggc attttattca 9180

tatttataca tcatccgttc cctctgagaa ctatttgcct gaacggttta taccgaaaca 9240

gtcacgcttg ttagctttct gccaggcata cctcctctct tcctcctgat atcgatataa 9300

tgcctggggc cagcctgagg atgatactgc tcataaaccc cctgcctttt tgacgctata 9360

actgaaggga gtaaagaaaa gacgatatca ttattttgca aaaaaatata aaaataagcg 9420

caccattaaa aacagtcttt catttatatt ttggaaccta agacaaatta cactcttaaa 9480

ctttcaacga atggtcattt agtggaaatc ttcgagaaaa atggttctga tggtgtaatt 9540

atcagaccat taaccatgaa gatataataa gcagcattta caccccaaaa aaatgcagta 9600

agatagctac aaaactaatc tctattgcaa tgaggccaag ttaaatatgt aaatatttag 9660

atgccaggcg ctgactctct ctgcaccagg atatacggca gcgtccattc gataatcacg 9720

gttagttata acaatattat taccaacatg tcagttattt aaagcacagg cataagctaa 9780

ataatcaaat gttaaaaaca tataaacccg agcccgtaga atatgacatt aagctcataa 9840

taaaagctca acctgaccgt tagtactaac agcagaatta ctgaaacagt agattctatc 9900

ctaacgactt gtattagtta ttataacttt tcaccctgta agagaataca ctattatcat 9960

gccacatttt aatcctgttc ctgtatcgaa taaaaaattc gtctttgatg atttcatact 10020

caacatggac ggctccctgc tacgctcaga aaagaaagtc aatattccgc caaaagaata 10080

tgccgttctg gtcatcctgc tcgaagccgc cggcgagatt gtgagtaaaa acaccttact 10140

ggaccaggta tggggcgacg cggaagttaa cgaagaatct cttacccgct gtatttatgc 10200

cttacgacgt attctgtcgg aagataaaga gcatcgttac attgaaacac tgtacggaca 10260

gggctatcgg tttaatcgtc cggtcgtagt ggtgtctccg ccagcgccgc aacctacgac 10320

tcatacattg gcgatacttc cttttcagat gcaggatcag gttcaatccg agagtctgca 10380

ttactctatc gtgaagggat tatcgcagta tgcgcccttt ggcctgagcg tgctgccggt 10440

gaccattacg aagaactgcc gcagtgttaa ggatattctt gagctcatgg atcaattacg 10500

ccccgattat tatatctccg ggcagatgat acccgatggt aatgataata ttgtacagat 10560

tgagatagtt cgggttaaag gttatcacct gctgcaccag gaaagcatta agttgataga 10620

acaccaaccc gcttctctct tgcaaaacaa aattgcgaat cttttgctca gatgtattcc 10680

cggacttcgc tgggacacaa agcagattag cgagctaaat tcgattgaca gtactatggt 10740

ttacttacgc ggtaagcatg agttaaatca atacaccccc tatagcttac agcaagcgct 10800

taaattgctg actcaatgcg ttaacatgtc gccaaacagc attgcgcctt actgtgcgct 10860

ggcagaatgc tacctcagca tggcgcaaat ggggattttt gataaacaaa acgctatgat 10920

caaagctaaa gaacatgcga ttaaggcgac agagctggac cacaataatc cacaagcttt 10980

aggattactg ggctaatta atacgattca ctcagaatac atcgtcggga gtttgctatt 11040

caaacaagct aacttacttt cgcccatttc tgcagatatt aaatattatt atggctggaa 11100

tcttttcatg gctggtcagt tggaggaggc cttacaaacg attaacgagt gtttaaaatt 11160

ggacccaacg cgcgcagccg cagggatcac taagctgtgg attacctatt atcataccgg 11220

tattgatgat gctatacgtt taggcgatga attacgctca caacacctgc aggataatcc 11280

aatattatta agtatgcagg ttatgtttct ttcgcttaaa ggtaaacatg aactggcacg 11340

aaaattaact aaagaaatat ccacgcagga aataacagga cttattgctg ttaatcttct 11400

ttacgctgaa tattgtcaga atagtgagcg tgccttaccg acgataagag aatttctgga 11460

aagtgaacag cgtatagata ataatccggg attattaccg ttagtgctgg ttgcccacgg 11520

cgaagctatt gccgagaaaa tgtggaataa atttaaaaac gaagacaata tttggttcaa 11580

aagatggaaa caggatcccc gcttgattaa attacggtaa aatctgagag aggagatatg 11640

cattattttt ttatcatcgt aatctggttg cttagcataa atacggcatg ggctgattgc 11700

tggcttcagg ctgaaaaaat gttcaatatt gaatccgaac tactttacgc tatcgcccag 11760

caggaatcgg cgatgaaacc tggcgccatt ggtcataacc gagatggttc aaccgatctt 11820

ggcctgatgc aaattaacag cttccatatg aaaaggctga aaaaaatggg gattagtgaa 11880

aaacagttgt tacaggaccc ctgcatttct gtcattgtgg gcgcttccat tttatcagat 11940

atgatgaaaa tctacggtta tagctgggag gccgttggcg cttataatgc cgggacgtcg 12000

ccgaaacgat cggatataag gaaacgttat gctaaaaaaa tttgggagaa ttacagaaaa 12060

ttaaaaggaa tgtcagcaga agagaaaaac aaaagacttt ctatcgcgtc aaacaaataa 12120

ttatacagaa atagcttact ttcagatagt tctaaaagta agctatgttt ttatcagcgt 12180

gccgtcgtca taagcaactg ggcttgcatt gcttttagtt gtacaaactg tgaggcgtct 12240

tccagcattc tattgttccg tgaattccgg aaatctgcac gtacctgctc cagattacta 12300

tgaggattat ccttaagtac aagggccgcc gccatcgttc cggttctccc cactccgccc 12360

agacaatgaa tcatcggtaa atgcttatct gatgaactac gccccggcgc gccattttgg 12420

ttactatttt tcaccctatc cgccaggtat tctaactgat ccgtagacgg taacggctgg 12480
tgatctggcc aatttttcac atgcaatacc gggattgtat accgcttttc cccgcaggac 12540
agttgcatat tgtattggtc tatcgcttct ccctgactgg ctgagctcac tttttggctg 12600
ttggtatgca cctcgccaaa ggtgtagctc cctctgaaat agggtggtaa ttgttttgcc 12660
tgcatctgat cttccgacgt taacaccacc aggcatgagc attctttttc aagaagcatt 12720
ttcatatgcg ctgccagcgc atccggcgta ttttttgggt acgaaccggc taatgccaca 12780
ggcttaccgt caaaagttaa cgtattcact ggcacaggca ttccatcgct cagtttcacc 12840
tgggtttgct gattaattgg aatgctgctg accgcaaacc gtgccaggcc cagtgtcggt 12900
ccgctcatcg tctgtggcat tggcgcgccg gcttctattt tctcaagttc agctgtaaca 12960
tttttcagtt ctttagcaat aacgtgaatt ttttttacag cctgggttaa ttcatgagta 13020
gaggctttat caacccacct ctcaacctct cctccacagg ttccccattg tgagaaccgg 13080
gctacaccga cctgaatatt tgttaacgtt gtaacataat cattaagttg tttagcctct 13140
ggaattttat ttaaattctg taaattcgtc attaatgaac gcagcgggcc gttacctgaa 13200
gccatctcct ggaagttttc tcgcaggcta tttccatcca tttgttccag ttgcggtaat 13260
gttcttttaa gtccctttag cgcgatatcg agtaaaggtt gcttactttc tgctccaaca 13320
tcgttatttt tttctgccac ttttgtatcg ccgcctttta tgactaaagc ggcattcctg 13380
acaccaacat tatccttgct cttaataagg tttataaacc cttcgtcagc agcttttaca 13440
cactccgtga tctgcactgc taaacgttgg gtaaggggtt tgttcatatt tatacgggac 13500
attaacagtg cgtcattaac cgctgtttcc ccatattttt ccgttagtgc atggagaaat 13560
gtctgtaaaa tcttttggtc ctgtactctg atattttccg tatgtttttg caccacttca 13620
gtgtttttaa ataacggcat ttttccaagc caggttaata cttttgacga aaatttttca 13680
ggcgcaacat atgccttatc agtattttcc ttagcaatat aaagtcgggc atcattcgac 13740
acaccaactt ttgaaaacga agacaacgtt aaattattca attttctctc ctcatacttt 13800
agcatattcc tgcagtatgt ttttgagcgc ttcctgctga ttcacaaatg actcaagctg 13860
cgatataata tggtaagtat ttgtcagatc ggtaattgca tgtataagca acaacgtctc 13920
tgcggcatca atatacgcta acgtaccttc attattcgca gccagttcac cattaatcac 13980
cataatctgc cgccagatag aatcgccaca aacaggcgat aacggtataa tcataccgtt 14040
caataaccag atatcatctt tagcttcaat agacgtaaaa atatcgctat cgagtaataa 14100
taagcactga ttgttgtcgt caaaagtgag cggtaaaccc aatttctcac caatattagc 14160
gataatatcc tggtgtgctt gcaatttact ttcctcttga attatatctt ttataagatt 14220
gcttcttcaa atttaatctg gttacacaat gtcttgatac tttttcgcgc ccatcgccgg 14280
gcgcaatatt tctctccttt aatccagtag aatagccatt cactacgcat cggaacacat 14340

atcagcagct ccttcggatc attttcaaca tgacgtaact tgcctttaat aacaaaacgc 14400
gaactgtcag caatatcatc atatattgca gccatacctg aaccgggtac tacatgtgtg 14460
atgattttca taacaattaa tcttattcaa ttgttgtcaa gcgagagaaa aatactacac 14520
cctggactca agactttttt taacaacacg gcatatatcc gcaaaggtcg tcatatcagg 14580
aagatcgttt tcattgcaac taatgtcaaa ctcctcacta agaccaaata caatatcaat 14640
taaatccaat gagtcagcgt aaagatcctc aaccagattg gtctgaccat tgatactatc 14700
aacatcaacg gcaatacagg aggtgatcac ttttttgact cttgcttcaa tatccatatt 14760
catcgcatct ttcccggtta attaacgctg catgtgcaag ccatcaacgg tagtaataac 14820
ccgatccacg ccaggtttat tcaggtatga ctcgtaagcc gggccagctc gccagctacc 14880
gtctccgata aggccgtcca gcacattact taacacatag tcagtttccc cttttagcct 14940
ggtcagcccc gcctctctgg caaactgcag tgcaatctca gccagttttt ctttttgtgg 15000
atgatgagta atgacctctt tgagagtctc cattttcgct ttcaattctg ggtgcttgtc 15060
aatatcgcta cattgcgctt tcaacgctgc actctttatc gtgtcattgg gtaatatttc 15120
cgcacgcaag ccgtcaaacg ctctgcgtac agggaacggt gtggaggtat ctggctccag 15180
ggctttacgt atcacaccca aaaacgtctc acgggcgtcg aaatgcattg aatgcatatt 15240
cgtaacgctc ggtactgttg agaggaaact attttgctta aacttcaaac cagaaaatgg 15300
gccagtctta tctgtctgac tattatcatt atcggtcgta ccggctttat tacctgtaat 15360
taccgtcgtg tctgattgta aggtatcggt tttaacattc tcgacgccag ccagttgact 15420
ggcaagcggc ttcacattca caatctctgc cgtctggctt tgctgtttat tatcatcaac 15480
gccgtgcacc gtggcctgta ccggcttgcc gataatgctc ttgctggtta cgccatcgac 15540
ttcatcaaaa gaggttgttt cacccatagt gcccttttct gacgtgacca cctttccatc 15600
tttactttcg gatgaagcgt tggtcacagc ctctgccgtc gcatgagccg tgacgtcaat 15660
tttacccgcg atgccatggt caattgcccc tgtgctggcg ctgtgtgccg tctccgtttg 15720
atgcatagtc gaatccacac gcgaatgact atgacttacg ttgctgctgt tagtcgaatg 15780
gtgtgactcg ccattgcgtt ggctgttatc aataaatgtt cggctattat caatcgtctt 15840
ccggctatta tcatggttgc tattatcgat atggcgctgg ctattatcga catggcttcg 15900
gctattgtta atatgcttac tgttatccac gctatggtta ctgctatcaa tattaatatt 15960
gatattaata cccgtaggtt ctgctttttt cccaccatca ggtactggtc cagccgcagg 16020
ctccggaatt ttagggtcag gcagtttatc tgcaggaatt tttgcaaaaa cattacgtag 16080
cagcaggggt atcaacgttt gcatttcaag gtgccgggct tcccgtccta cgctggtacc 16140
ctgctcttgc gttaattttt ggtggcacat atcaagcgcc tcaaccgcct tcgccgccgc 16200

tttgtcaaca aggtgcgtaa gattgctgcg ggttaacgga tctaacgtac agccaaagtt 16260
atgttcaatg cagctggcaa tatagggcat cacctcctgc ataacaagat tcgtcgataa 16320
tttacttaat tcaccaccag tgttattttt gataatatct aacagctgct tttccaggtt 16380
ttccagcttc gcttccgctt tctttgtttc tggcagccat ggcccaaaag ctgacttttc 16440
tttcaggcca tcttttatga tttgctcggt atactctgcc cccaccttca tcagtagcgt 16500
cttcgcctca ggagaatcac tggtggcgtt gagcgctgaa cgaaagagcc cggcaaactc 16560
cattatcgct ttcttaccgg cgacattatt tgaattggta aaaacttctt ttaacgcctc 16620
agcgtctttc ccgcatttaa acaatgcatc cagactcgcc tgtttgatca gcgcgggaaa 16680
atcttccagt tgcgggcctt taatttcccc tgacagcgtc gttgtggcac tttctctgac 16740
tgcggaaaga ttcgccgcaa gattcgtggc ctgcgttttg atctcggtct gcatacctgg 16800
tattatgacg gggggctgag tccttacact tgtaaccatt attaatatcc tcttctgtta 16860
tccttgcagg aagcttttgg cggtttccag gctgctactt atcgtactgc tcagcacttt 16920
taccaggttg tcgtacaatg aattggcatt gctatatttt tgcgtcagcg tctgtaatgt 16980
ggttttcata ttttcttcct gcgctttaaa acccgactgc caggcttgat atttggcgtt 17040
atccatttcg agttttgagt cttttcccgg cgcgcctaaa ccatcaatat cctgaaccat 17100
tttttgtaat ggcgtcagat caacggtgac gacataaccg gatccataag atttcaggca 17160
gctattcggt aaattcaatt cactgagcca ctgtctcgct tccgcttcag tggctacttt 17220
aacgccgctg cctgactgag ctggaaataa aacggtatta ctgtttattt gattatattt 17280
attgactaaa ctgtttaaat catttttgag tgaggtaaca tctagcttaa cggtattacc 17340
gtccttacct ggtaataacc agcctcccat tttggaaaga atatcactga aggcctgata 17400
aaaatcggta tagactgcga caacgttttc ataaacgccc agatagctgt cacctatcgc 17460
cgatatattt tgggaaacca tatcccaaat ctcagcatca gaaatggttg ttctcggctg 17520
cgccataggc gaagcgctaa ataaggccga cgtcggcgca gaaaacgcgc tccgcaggtt 17580
ctcattttgt tctgcggata atgacacgcc agacttcgcc agagcattca ggctgctggt 17640
caactgctgg cgcgccagcg tgcgctcgtc attattctct tcagagatcg gtggcgttga 17700
ctgcagcgtc tgctgtgcct gctggatttt agtagccgcc tgcgataatg aaatgatatc 17760
tgtaccgcga tgttctgtgg tagacggtac cacggcagtc tcgacgtgct cgctcgccga 17820
gggagtctgc ggccgttcgg caacgatccc cggatgagga gaagcggaat aattttgaat 17880
attaagcata atatccccag ttcgccatca ggagcgcgat taaatcacac ccatgatggc 17940
gtatagatga cctttcagat taagcgcgaa tattgcctgc gatagcagca agtgcggatg 18000
ctttcgactg gttaatgctc tccattgttt tcagcatttc ctgaatcagg ctggtcgatt 18060
tacgtgaact ttcacgggct tcgtccgatg cggtgctggc aacacggtta ttcacctggc 18120

taatttgctg ctcggaacgt tcctgagtag cggcgtactg cccggacgcc cctgcaatac 18180
caccgaccgt gaccgagttc ttcataatca gatcgcccgt catctgcatc ttgcgcgcat 18240
caattcgggt catatccatg gtattctgct caagacgaat atcggattcg acagactcaa 18300
gacgtttcga cagaatagcc tgatgttcag gggagatttg tttattactg tctttaatac 18360
ccagactttc cgtggcgctg gttccggcat tagatttaag cgtcgcatca ttaagatttt 18420
ttgtcgcatc ggtaccggtt ttcttcatat ttaacgattt cagagaatcg acgccttcag 18480
caccgagttt gacgctattc tgcccgttca gcacgttttt aatactgtgg ctttcagtgg 18540
tcagtttatc gatcttcgcg gcattatgtt taagcgcgcc tctttcattc tgcagcccct 18600
tatattccag tttggcgccc acgccagtga tccccaactg aagcgcgctc tgggaaatac 18660
taccggacaa cgcattcatc ccttcgcgca tcatggagct tgctgtcgtt ttagctgcat 18720
caaagctgac taatgacaac ttaccagaca gtttgctatc agcctggttc aacgtcagca 18780
ttaacgtatt cgcggcagcc aacagcgcaa cggcactgga agacattccg ctaatatcaa 18840
aaaactttcc gacttctgcc tgctgctcgc gtaactgggt ttgcacaacc tcattcgctt 18900
tagtcgtgac attatttgcc agagcattca aatcctgatt catgtcggta ttttgaatac 18960
tggcttttaa aaaggacgtg atcgttccgg gggtttgcgt taatacccct ggcgcaggcg 19020
cgctcagtgt aggactcaac cccaggtcac tgactttact gctgctaata ccaatactat 19080
tcagaatatc tttagcgcta acggattgcg aagctgtctg tgaactattc tcaacagaat 19140
gattatttaa ataagcggcg ggatttattc ccacattact aattaacata tttttctccc 19200
tttattttgg cagtttttat gcgcgactct ggcgcagaat aaaacgcgaa gcatccgcat 19260
tttgctgtac cgcagaagac atggcttttt gcagttccgc cgttaccttc tggttttcac 19320
caaatatttc tacggattgt ttaagccact gctgaatctg atccatggca aaacgggcga 19380
gcataaaatc agcaagcgcc tcgctggcat ttttaataaa tacgccctcg gcaacaccac 19440
cggctgactg ggctgcggta ttcgtgactt ccatgcccaa cgccacttta tttagggtat 19500
tacctaccag ctctttactt aaggcattcg tttgcaggcc catcttgcta cctacattac 19560
ccagaccgct agtaatacgt tgcatcccct gggtaaagag tttgctgccg ttttgcgcca 19620
actgtttcag cacgttaggc accaacttct taatcgtttc gcccatcatt ttgctcagcg 19680
cgttacccag tttcgccgcc gcgcctttcc cgacaactgc gaccaccaca atgaccgcca 19740
ccatggcaat agcggcgaca atcgcaccaa caatgctgcc ggccatctct gccgttttct 19800
tatcgatgcc taatccttcc agcgctttgg taatcgcctt gccaatcagc tccattaacg 19860
gcttcagcac atgctccata atcgggttta gcgcctgctg aataaacgac acccccgtcg 19920
ccgccttcac aatttcatcg gccaccatta ccgcaagtcc caccgcagcc agcgccagac 19980

tcgccccacc ggtaaaaaca gcggccacaa cgctgacaat ggttagcagc gcgccgagga 20040

ctttcccgat acatcccata atgcggttcg tttcctcggc tttgcgcgtc tcttcctgga 20100

attcagccga tttcttttcc atctccgcct gacgcccttc ctgcaaggcg ttgaaaagcg 20160

caagatcgtt ttgcaggctt tcttccgtat ttttgcccac aatctcaata aacatggcca 20220

tgagcatagt gaggcgggcg acatttgaca gattatcctg ctcaccctgg gaaacctgat 20280

tctgagaggc ggcattagcc gttccctgga atttggtcag aatgttatcc gctttctcgg 20340

ctttcgcttt ggcgtctgtg cctgctttaa ccgtcgcatc cgtggcctta tctaaggcct 20400

ctttcgcctc tgtcgcttct tttccggcct gttctaccgc ggcttcagct tgtgcatagc 20460

cggggtcagc cgggtccagc gattgcaatt tattttgcgc ctgcgtcagt tttttggtcg 20520

cagcgtcata aacactcttg gcggtatccg tctttttgat actggcttca tagagatccg 20580

tcgcctcctg agcctctccc agagccgtct ggaattcttt cgatacctga atccccatct 20640

ctttttgtga ctcaatcatc gcctgccata ccgccagacg agactccagt tgagacagcg 20700

aaacatcacc cagtagcgtc attaacttgc caagcagtaa tgtcaattgc ccttcgctgg 20760

agagtttttc ccgggcggcg tccgtaggcg gcttcagacc caccgtatta atagcgctct 20820

cgccggactt tgttccggct ttaaggtcgc ccgctttcgt tgccaccaca tctttaaaag 20880

ctttatccgc cgcttttaaa aagtccgtgt tcttacgaac gccttcaaaa gccgcctcag 20940

cgaggcgcgg attttgggta tatccgctac ggctaatgct acttgcgtca tttaccataa 21000

ttattccttt tcttgttcac tgtgctgctc tgtctccgcc gtttttagcg cctccagata 21060

gaccaacgct tttgcccgca gagactcatc ttcagtacgt tcattgacaa gttcaaaaca 21120

ctgtctggct tttgctgcct tacgcattaa taattgacac tgcccggtaa aaaaaacggg 21180

gcgataatca tttttaagta acgtaaacgc tactgcataa aggtcacatg ctttctgaaa 21240

ttgtttttttc agttggcata ccgccgccag tcccatggtg taatcgggat tgtaaaaatc 21300

ataaatgcat aagaaacgaa agaatgtctc agcttcatcc agtcgtccct ggttataaaa 21360

ctcataagca tgagcatata aaccgtccat catatcttga gggatcccat gaacgtcttt 21420

tagcgtggcg ccttcactaa cggcatccca aatcatttcc gcaacacgtt cttcgctgac 21480

attattttga taatccatta cttactcctg ttatctgtca ccgactttgt agaacttaac 21540

gactgcgttt atctgatgca gttattaaac cccgacggtg gttagtgaac attcaaaaaa 21600

cgcccaatga atacatcgct actgctttac gcggctcaat gccgtacctc gttttcttgt 21660

ggctgaataa cgtctttgcc cgcgttttct acctcttcca gccaaaccag aagacgtaaa 21720

acttcatcaa tttcttccag actcaccaga tcataacggc gatgggtttt gaaaagactg 21780

cgcgccagtt tgatatcgac gatcacaggt acgccaacct tctccgcata ggcgcggacg 21840

gccagtgcgc gctgattcgt ttcatacacc gagatcatcg gaatcggcat caattcgggt 21900

ttaaaataaa tcccgatcgt aatatgcgtg gggttggcaa caatcaggcg tgagttttca 21960

atatcagatt tcacctgttc agacagaatt tccatatgaa cttcacgtct tttagattta 22020

acctctgggt tcccttcctg ctccttcatt tcacgcttca cttcttcctt atccattttc 22080

atatctttca tggtcaggaa atattccgca atagcatcca ataataagac aatcaatgcg 22140

caagcaaggc aagttaatac caatgcgagg agaagttcac gccaaatgac ggcaatacct 22200

acaatattgc catttagctg agaaaagatt tcaaccttat atttcttcca gcaaatgatg 22260

gcggccacca caaaggatga gagatacagt agggttttga ccgtatcttt aaccgtgcgc 22320

atactaaaaa gtttttttgc cccttctacc gggtttaacg ccgataaatt aggctttaat 22380

gcttctgtcg ccagcacaaa accggcctgt aataacgccg gtaatgcgga acacactaag 22440

cagagcagca taaatggaat cagatatttt aaccctatcc caaaaacggc caaactgtag 22500

tcagccatgc tctgatcaaa attatccgca ataatgatct taattatccc cataaactca 22560

ttaaatgagc catacgacac cagataggca attcctccca gcgtcaggca ggcgataatg 22620

agatctttac ttttaaatga ctggcctttt ttagcggagt cttccagccg ttttttagtc 22680

ggtttttctg ttttattcga ggacatgcgt cgcccctcgc tcgtaaaacc aactgcttaa 22740

ccctgtggcc tggaaagaga gtcgcagtac attgtccggt agtaccggag agaaataaag 22800

cagcataatt aaaacggcaa taccgctttt taccgtcagt gaaatcgcaa aagcgttcat 22860

ttgcggagca aagcgcgaca ataaacccag gaatacttct gacagcaaca gcactaatac 22920

caccggactg gccagaacca aggcgttttg agccacctga ttaataaacg ttaatagcgg 22980

cggtaatgaa ggcgtgcact cgttcatcgg atcgcatagc tgatagcttt tatttaacac 23040

gtcaaccatc gtgaccagac cgccgttttg taaataaacg acagcggcaa acatattcag 23100

gaaattagcc atttccgagg tatcaatacc gtttgccgga tcgatactgc tacttagcgt 23160

tgcccctcgc tggttatcga taatacaacc cagcgcatgc ataacccaaa aaggccatga 23220

tagcagacag cccagcatga cgcctaccgc cgcttcttgc agaactaacg ggatcatcgc 23280

caccgataaa aacggcggcg cctcgttcaa tgcatgcggc catactccca atgccaccag 23340

gatgataatg gcgtttctcg gcgcgccgct taataccccg ctattcaaaa acggcaggaa 23400

gaaaaaaatc ggcgctacgc gagcaaaccc tagtgccgca gacgcaacca ggtgatgaat 23460

ttcaaagtac aacgcgtaaa gcatttttta ccccttagcc aacgccagga atatcacctg 23520

acgcccgtaa gagagtaaaa cttcgccata ccagccagac agtaaaaaca agcataaaca 23580

cacgccaagt aatttaatgc caaaaggcag cgtctgttcc tgtaattgcg ttaccgtctg 23640

gaataaccct accaggaggc cgataatcgt tgcgacaatc gtcggccacc ctgacaggat 23700

caaaacaaga tagagcgcct tattacctgc aaacactaaa tcatccattt aactatcccg 23760

tctcgtaatg atgtcatgtt gcaatgtcca tatactgtaa tatcaatccc ttagacagta 23820
aggtccagcc atcaagcgcg acaaaaagca ccaacttaat aggtgtagat atcgtcaccg 23880
gactcatcat catcatcccc agcgccagta gcacgctgga taccaccagg tcgacgacaa 23940
caaagggcaa atagagataa aaaccaattt taaacgcgct ttttatttcg ctcagcgcat 24000
aagcaggtaa taacgcaaat attgatggtt tttcaatttc atctttgtca cgctttaccg 24060
tctcggtctc ttctccatac tgacgcttca gttgcgcgtt ttcaaaaaac tgaactaact 24120
cgcgatctga atatttgatc agataatcgc gataaccatc cagaccttca tcaacgtgtt 24180
tacttaatga cgaaatatca ttaaaggtga catcttcgtc ctcaaaatag acgtaggcat 24240
catgcattat tggccacata acaaacatag aaagcagcaa tgcgacgccg ttaagcgtca 24300
tatttgaagg tatctgctgc aatcccaggg cgttacgcac catgacaaat acaatagaaa 24360
atttaacgaa acaggttcct gacgcaataa taaatggcaa cagggtggaa aatgccagta 24420
aggcaattaa tgagatatca ttccccatta ccagactcgc tcagccattc atggatctca 24480
acgcctaagg tgtcattcat ctgtaccagt tcgccattac ccagcaaaac accattcgcc 24540
ataatttcaa cgttaagttc agcattggtc ggcagtgata atagctgttg ctgccccatg 24600
gcttcgagtt cggcgagggt aacgttctta cgatacaaaa caaattccag tttgacgggc 24660
aattgattca agccaggcag agtttctgca gtttcagttg tattattttc ttcttcgata 24720
tgttgaatat ctaacgtttc cacaataatt ccccttcaa cacggttgaa atgacctaac 24780
tttttcgcgt agcaataaac ttccgcacgg gaagtacgaa tcaggagtac atctccgatc 24840
ccgattcggc ccagcaacga acgctgcgta tcactgctac cgattacaaa gcgcaacggc 24900
caacgcagca ttttcggcct gccgccccg actgcaggca gttcaggaag atattcaaac 24960
cacaggccgc cccgatcgct cataatgtgc aacaatttcc cttccggcag cgcgcttccc 25020
ggcacggggt tctctacgca taaacgccga caggacaaat gcggcacggg caactcaaac 25080
ggtcgctctg tcgcagcaag ccagggaacg accaggtgct cagcgccagc agaaaccgcc 25140
gcccccgcca gagcgggaga gacatgctca agccagtccc caggttgaat ccacgccgac 25200
caccgttttt ctgcatcgct caaccgaacc cacattcctt gtcgcgtcgg atattccagc 25260
gtagcttcct ggccatggcg ctggcattct gtcgcggttt gcgccaatag ccattcgcga 25320
cgatcaatct gtctcacacg caatgacatc aggcgtcatc ctcctcgcca gattgctgtc 25380
tgtgctgttg ctgctgcgga ttttgttgat cgtctcgcgt caggtgccag cgctggggat 25440
taccgttttg ccattgatca tgcaaacgat gttcaacctg cgtatttgac ggtattaacg 25500
aaaactcccc tgcttgccgc gcctgaatat tgacggaata gtcatttccc cagcgctgaa 25560
aacggtaagt cagcgagcta tcctctcctt tcacgccatc ggcagtcgga aaaatagtca 25620
tcatcggctt tgattgcgcc gctaaaggca ttttttcatc gccgccggtt aattggctaa 25680

gatcggcgat agtggttggt tgcagcggaa gctgagaaac atctttaacc tttttatgat 25740
ctttatcgtc aggcttaccg gtattggctg cggccattcg ggcaggtgcg acatcccgcg 25800
ccagcggcgc gccctcttta cgaacgcctt cgcccgcgat ggctttatta tccccaggca 25860
atgccttgat attatcgtca gagattcccg tggcgttatc ggctacttca ctaacggact 25920
ccacggcttt taaatcagca gataattttt taccgcttac gctttctaac ggcctatttt 25980
tagacgatag caacgctgcg gatttatcta ctttggcctc cgcagaaatc aaaccgacag 26040
atttttcagc agtgactttc aacagttttt cagcaatcct gagttcgcct tttccgttat 26100
gatgcagacc agaaacgttg ccattgtgat gttctgattt cgctggcgcg ccatgtcgcc 26160
atgccgccag taataccggt aaagccgttt ctttatgcat tacgaaagca tcgccatagt 26220
cgcgatcttt tttatcaccg gaatattctg tcttatgttt ttccaccgct ttttttaatg 26280
cttctgataa accgccaacc tcatcctgct gcggcagtaa aatgttcccg gatgaactga 26340
cagctgacac atcgcccatt aaattatctc ctctgactcg gcctcttcct gctgtatctc 26400
tcgctggata tagaatcttt tctgacggat tatccagcgt tgatagttcc cttctttgcg 26460
caaccaatat ttactttttt tctgaaactc ttccctttc ttttccagct cgctccgttt 26520
ttcctgaatt tgtataatct ggagttctaa atcttttatc tgccggcgaa caatagactg 26580
cttacgtaat aacgtataaa tttcctcacg actgagctgt ctgttttctg cacgcagcgt 26640
atctaataac aatttcagac ccgctatttg ttcaaggatc gcctcctcct cggcctgcag 26700
cccgcggtcc tcatcctgat agcgaagtaa tatcgactca cactgtgaat gaaataccgt 26760
acagcgccgc tgcaatactt taattctggt cagcgaatgc attcataccg ctcaacgtgt 26820
catcaaagga tgaatactgc gctaccggct ggcataacca ggctttcagg ctatcccgca 26880
tctgcatcgc ccgatcgtta tcgatatttt cgccaggacg atattctccc aagtcaatga 26940
aaagctggag ctcttccaaa cgcgtcatta atttacgcac ggcagatgcc tgttcagcat 27000
gtgtcggcgt cgtgacttgt ccaaaaacgc ggcttacgct tttcagtaca tcgattgccg 27060
ggtaatgtcc ctgcccggcc agctttctgc tcagatacag gtgaccgtca aggatagagc 27120
gaatttcatc cgccatcggg tccgcctctt cctcgctttc cagcagtacc gtataaaagg 27180
cagtaatgct tccctcgctg gtcgcccctg ggcgttccag caagcggggc aaattatcga 27240
atacggaggc gggataacct cgacgggccg gacgctctcc cgacgccagt gccacgtctc 27300
gcaaagcacg cgcataacgg gtcatggaat cgataaaaag cacgacccgt tttccctggt 27360
cgcgaaaata ttccgctacg gttgtcgcca gttgcgccgc attgcagcga tcgaccgagg 27420
ggaaatcgga agtggcaaaa accagcacgc atttttcttt cttatgcgaa gcgcgcaaca 27480
tatccacgaa ttcagtgacc tcacggcctc gttcaccgat aagaccgata acaaagacat 27540

ccgcctccgt ttgctcgatc agcatatgca tcagcatggt cttaccgcat cctgcggagg 27600
caaaaatgcc cattcgctgg cctacgccac aggtcaataa cccgtcaatc gcgcgcacac 27660
cggtaatcag cggttcacgg acgccaacgc gtgaagcgta agacggcggt gcgacatcaa 27720
taacgcgttc ttcgctaatc ggcgccactt caggggtaaa acgctcaacg attttccctg 27780
tcggatccaa caccgcgcct aataccgagt atcccaccca cgccgataac gcacgtccag 27840
tgggataaag cacgacatcg cggctcagcc cctgggcatt gccgataagg ctcagcacgg 27900
tgcgttcccg ctgtaagcca accacctgcg cacgtgcaac aacctgtttt tggtgccagc 27960
cacggcgtat ttcacacagt tcgccaatgg ccacatcgcg caattccgcc tcaataattg 28020
ggccggttat tttttgtggg taggccagat attgcagtaa acgaggtgtt ttcatctcat 28080
tagcgaccga ctaaaaactt ccagatagtt gtaaaaccca ttcaaggcag tagagaactt 28140
ttcaccgtca gataaaaaat ccggatgcac taaggcttta agcattagct ccccattctg 28200
ctcccccagt agtaattgcc cgccgcgggc aaaatggcat ccttccatga tggtcattaa 28260
gatttcataa gcccgctgtt gtaataccac catgctgtca gcacccaatt gcgcccagat 28320
ccatacatca tcgtccttga cgctgataca gatacttggc aatgcaaata aatccagaac 28380
aattgttgaa tggctatcta ttcctccgat gagtgaagga tcgcaaccac ttacttccag 28440
tgcggaacga actaattcag cgatatccaa atgttgcata gatcttttcc ttaattaagc 28500
ccttatattg tttttataac attcactgac ttgctatctg ctatctcacc gaaagataaa 28560
acctccagat ccggaaaacg accttcaatc attttcttaa taaatcgacg gacatcgaca 28620
gacgtaagga ggacaagatc tttatgtgca atcaataaat catccaactt aagtgtaatg 28680
agatccatca aattagcgga ggcttccggg tcaaggctga ggaaggtact gccagaggtc 28740
tgacggatcc ctttgcgaat aacatcctca acttcagcag ataccattac tgctcgtaat 28800
tcgccgccat tggcgaattt atgacaaata taacgcgcca ttgctccacg aatatgctct 28860
acaaggttaa tgacatcttt ttctcttggc gcccacaatg cgagcgcttc cataattaat 28920
ttcatattac gcacggaaac acgttcgctt aataaacgct gcaaaacttc agatatacgt 28980
tgtaccgtgg catgtctgag cacttcttta agtaaatcag gaaatttcgc ttccagttgg 29040
tccagcatat gttttgtttc ctgaataccg aaatattcat tgacgttgcg cgccagcgtc 29100
accgccagac agtggtaaag ctcatcaagc gcgttccgca acacatagcc aagctcccgg 29160
agtttctccc cctcttcatg cgttacccag aaatactgac tgctaccttg ctgatggatt 29220
gttggattaa taccaaagga cacgacttca tcggaataat ttaccactcg catcaaatca 29280
aaatagaccg taaattgttc aacacggatc tcattaatca acaatacgat gctgttatcg 29340
tccaggccct cgccatcgcg taacaatact tccggcaggc gcacgccata atcaataaag 29400
aactgactac gtagacgctc cgcaagttga gctttttcca gatcttcacg ccggctcttc 29460

ggcacaagta atatcaacgg tacggtctct gtagagactt tatcgagatc gccaatcagt 29520
cccaacgacg cccctcttt ttcctcaata ctaagcggct gctcgccttt gctggtttta 29580
ggtttggcgg cgctacgttt tgcttcacgg aatttaaaat agaagagtac gcttaaaacc 29640
accgataaaa taacaaatac cggcagcggg aatcccggca gagttcccat tgaaatggtc 29700
aaaatagccg taacaaccaa tacaaatggg ttgttcaaca gctgcgtcat gatattccgc 29760
cccatattat cgctatcgcc atttacgcga gtcacgataa aaccggcact aatcgcaatc 29820
aacaatgcgg ggatctgggc gacaagacca tcaccaatgg tcagcatggt ataagtagac 29880
agagcggagg acaaatccat accatggcgg gtcatcccca ccgaaatacc gccaataaag 29940
ttcacaaaga taataatgat gccggcaata gcgtcacctt tgataaactt catcgcaccg 30000
tcaaaggaac cgtaaagctg gctttccctt tccagtacgc ttcgccgttc gcgcgcagca 30060
tccgcatcaa taataccggc cttcaaatcg gcatcaatac tcatctgttt accgggcata 30120
ccatccagag aaaatcgggc cgcgacttcc gcgacgcgtt ctgaaccttt ggtaataacg 30180
ataaactgga ccacggtgac aatagagaag acaacaaaac ccaccgccag gctatcgcca 30240
ataacgaatt gcccgaacgt ggcgataatt tcaccggcat cggcttcaat caagataaga 30300
cggctggtac tgatcgataa tgccagacga aagagcgtgg taattaacag taccgcagga 30360
aacgttgaaa aactgaggat tctgtcaatg tagaacgacc ccataaacac caatatcgcc 30420
agtacgatat tcagtgcgat caggaaatca accagatagg taggtaatgg aatgacgaac 30480
atagaaatga tcatcaccat tagtaccaga atcagtaatt caggtcgtaa acgagcactg 30540
ttaagtagag aaagcagcac tataggtatc ctgttaatat taaattaaga cagcttttca 30600
atagtacgac gctgttctgc catttcatgc ttgtaggcaa tatcggtcat actacgtaac 30660
gccattaaca attcttcctg ccaatattct tcataaaaga gtgaagaggg tatggcttta 30720
catacttgat aaaatatctg caaaaaggat gcatgttctt tatgactaag caataacgca 30780
ttcaaaccta taatatcggc taacagcgaa tccacttcat gtggctgttg caatagcgaa 30840
agcatcagta gtagccacga cgactcctcc gcattaaacg ctttggtaaa cgaatacgac 30900
aacaatgtac tcacaaacag taggtcagcg gagcgcaaca ttttaagttg cgtcaggcgt 30960
cgtaaaagct ggccaaactc caggcgcgaa caactggcgt cattcgcgtc aatatcggtt 31020
aatagcgaac cctcaataaa atccagtacc accagtcgac gttgatagcc ataactggct 31080
atccagtcag agtaaatctc cacttcatgt gattcactct ggataaattg ccgatagctg 31140
gcgcgcaata agcctggttt taacgataat gttttcccaa aaagccgggc cttcaacgca 31200
caattaatcc ctgccttgag ggtcttcgga tcggtttgct cttcaacgtg cttaagtaac 31260
gactccagct ttttccgcac gatctcttcc aggtctttac gacgaagcaa ttcgcgtaac 31320

acaaggacta aatcactggg gtcaggaaat aagctacgcg cctgacgtaa aaaatcttct 31380

aacgcgccgc catgtacgct aattagcttt aagatttgct tcgccttcgg taaagcctca 31440

tcttccagca cgcgttcaaa actgttagat aaattactgg atttttttc ataatcgcga 31500

cggttacgaa attgcgccag cgccgctgac atttcgtccg tcgactggac aaatttttgt 31560

acttccgccc ctggagacga atcctctgcg gcctgttgta tttccgcctg ttgcgcatca 31620

gtatgctggg tcgcatcctg atgagatgtc tgccgggaca atattctgga aaatgaaata 31680

ccggaggttg agccaggaat catttaattg cctcctgacc tctatccaga taaacacgaa 31740

cccatttctg taacttatcg tccccactcc aggcaccgct ttgcttcaga atattgttta 31800

ccgattcgct ggcatccggc gttaacgggt cgacaatttc ttttggttca atcatgaaca 31860

cacgaacaac attactttta ttcttactgg aatagcggaa caggctacca ataagcggta 31920

atttgcctaa aaacggaata ctttggacag tatcggtatt tgcatcccgt gtataaccac 31980

cgaccagcaa actttttccg tgcggcactc tcgcaatagt gctaattaac gttcgcccga 32040

cttcgggtaa cgcatctacg gaggtggtag tatcggattg cggcgtctta tcgttgccat 32100

cttcaatgtc cagcgacatt tctatctgac catctgcgga aaaacggggc agcactcgga 32160

tcattgttcc gtatgttaca tgctcaagcg ccacattacg ttccccaatc agcttggtgt 32220

aaaacgttct gttgttatca aaaatagcgg gaacattttc ctgggtcagt aataccgggc 32280

gtgaaaccac cgtcgcctgt ttcttctctt ctaacgcatt gaccgcggcg atgaatcgac 32340

tgccatcgag ggtacttatt gaagactggt ttaatgacac gccaagtttg tccccaatag 32400

taatgctgcc gctccatgaa gtgcccaaac gctccagatc gcttttatta agatcgacaa 32460

tccacaggga taattctacg tgacgtttgg cgacatccag cgctttaacc agcatttcga 32520

taaaattcac ctgctcagcc gttcccttta ctaacaaact gttggtatcc ggataggcca 32580

cgattttaat attgcccgcc gcggcatttt gctttaaagc ttcctgcaga ctcatgccac 32640

cggcataatt tgctgcttta ccttttctc cattcgctga aaacgctggc atcgccgggg 32700

gttcgctact gacaatatta cctaaaggtt gctcttctcc ctgcaacaac ctttcaatgg 32760

ccgtggcaat accggggata accattttct gatcgcgcag attataggta cgatcgccca 32820

cgaaggtatt gttcagacgc atcaccccta ttttctgacg tcccagctca ataccatcgt 32880

tttgcttgtc catcatggtg gcggcgttga ccaccatatc aacatagacg ggtggccctg 32940

aaacatagaa tgttccttta cggttatcgc cacgtagcgg gtaatttttg ttatataaac 33000

ctgagcgttt tagaaaattg ttgaactcat tgagtgagac gttgcgtaaa gaaaccacgg 33060

cattgcgcat ttcactggcg tcataaatat agatagcctg cccatcgaaa taccaaatca 33120

gccccagttg tagggaaagc ttctccagta atgcgttagg atcgtgaaac tcaaagttgc 33180

ccgtaatttt ttttcgtgcc gccattttgc taacaatgac aggctccttt agctgtagcg 33240

ccatggcatc gaaaaatgtc cgcaggctat cgtctttcgc aacaaaccca cttcccgtta 33300
caggtatttt ttcactagaa taaccaggtg taaccagaac aagcgcggca catgccagca 33360
ctctggccaa aagaatatgt gtcttcattt gtctgccaat tgaataatat ttgataattt 33420
ccgcggcgaa acgccgatca gctctttgat ctcactagaa aaatgtgaag gcgatgagta 33480
accatgatta acggctaatt gggtgatgtt ctcgtggcct tctacactat tcagcagcga 33540
ttgcgccata cgccagtttc gtaattcact cttcgctttt ccgcccaacg ctctgctgca 33600
caaacgacga aaatgggtat aagaaacgcc atagtcttct cccagcattc tcatcgtgtt 33660
gccgctggtt gactgagcga gtaaatagcc aaccaaccag taactctcgc tttttcgtaa 33720
cagagccagt accttattga aggccggaga aggtgtaata atttgctgca aaaaccagta 33780
ctcgcagcgt ttacgatctt gccaaatagc gcgaaactca ggactcagca aaacccattt 33840
atcggattca gcatatgtcg tgtccactaa tcctgcgcca tcgataaatg ccagtaattt 33900
gctgagtact tcaattttta acggtcgaaa aaccaggtct cctgatactg gtgcgacaac 33960
ggcctgctcg caaaaaagca gcgcgccttc ctgaatcagg caattttcat tgtgtcggct 34020
ttcagaaaat gacatatgca gcttttgcgc ggaacacgtc tgtataaacc atgcttccgg 34080
gctgcggatt ttccgcttct ctccttcttt aagtacttcc tgcgtattta gcatagttgt 34140
cagcaccagt taaaaatcat tttaatatgt aaacaatacc gggagcgggt ggcaaaatcc 34200
tgatgcaatc attatgaaac tgatgccgcc cgctaattaa attggccaac ttgcacagtg 34260
cttgctgatt taaaatagaa aattagctca tagtgtataa attctggctt attgttctgc 34320
agcagcaaaa attcagatat tgtcatctgg atggagaatt aattatttat atcaggagtt 34380
ttttttgcta gcattcctga aacgcattcg cctcttatca ctattgtcag ataacattct 34440
gacggttgtg taaaaacatt gcgcctcatt cttctgtagt tggagttaat atgaaaaaat 34500
tttatagctg tcttcctgtc tttttactga tcggctgtgc ccaggtgccc ctcccttcct 34560
ccgtgagcaa accggtacag caacctggcg ctcagaaaga gcaactggcc aacgcaaata 34620
gtattgatga gtgtcagtct cttccgtatg tgccgtcaga ccttgcgaag aataaatcat 34680
tatcaaacca gaacgctgat aattccgcat caaaaaatag cgcaatcagc tcaagcattt 34740
tttgcgaaaa atataaacaa accaaagagc aggcgctcac cttcttccag gaacatccac 34800
aatacatgcg ttcgaaagag gatgaagagc aactcatgac cgaatttaaa aaagttcttc 34860
ttgaacccgg aagtaagaat ttaagcatat atcagacgtt acttgctgcc catgaaagac 34920
ttcaagcctt ataa 34934

<210> 2
<211> 25262
<212> DNA

<213> Artificial Sequence

<220>
<223> Salmonella pathogenicity island-2

<400> 2

ttatggtgtt tcggtagaat gcgcataatc tatcttcatc accatacgta acaaggctgc 60

aacgggttca aataacgttt caggaatttt atctccgcgt tccacttcaa aaaataatga 120

gcgggccagc tcaacatttt caacaacggg gatgcagttg cgttcagcga tgttaacaat 180

atagttagct tgagcatcac tgcctttttc caggacgcgt ggtattggca tatcggtggg 240

atgatagcca agacaaaccg caatatgcgt tggattacgc actaccgcaa cagattgttt 300

aacagattga gctaaactcc cactttgtat ttcactctgc atttcccgac gccgtgtctt 360

catttgagga tcgccctcca gatctttatg ctcctgtttt acgtcatctt tactcatttt 420

tagatctttt ctaatcttat aatattgaaa agaatagtcc agtatgccaa cgacgatata 480

aaaagccatc accccaaccc ataaccattt tattaaagaa gaaaccacaa gcaggccaca 540

ggctaaccca cagtacggta gcgcccgaaa agtactggca taataataaa agaaaaaagc 600

aaagataaga gatagcatga taactttcag gctggattta cataattcta ctacgctatg 660

taaagagaat atctgcttaa aattacttac cggatttata tgctcgcttt taaaacctat 720

ggccttgctg gcaataacca cccccacctg aagaaacacg ctacccacag tagcaactat 780

taccccagcg cccagaaaca gcagtgcaga agtcagtgac tctattaaag catgactcaa 840

ttgcgttaat gcataagaaa atggtttatt tactaattgt aatgtgaaag ttattgactc 900

aatcagtatc aaaatcatct tttcagtaaa gaaatgaaaa tacaaataaa gcgcaatcag 960

ctgaaataat gatgttattt caatactttt gacaacctgc ccttccttac ggccatcacg 1020

taatttcttt tctgtaggct gttctgtttt ctcgctcata cagatggaaa ccagtctttt 1080

agataaatat aaaatttatc gctttcaacc aaatagtgat gaagagcata agggaatgag 1140

atcaggagcg tcagtagaac cgatatactt ttgagcggca ttgagaggaa aaacacattc 1200

aattgttgtg ccgaccgatt taaaagacct aaagccagat cggctaatac catacatatt 1260

atggcaggaa gagagaagct gatacataat tgataaagcg ttctccactc tgcctggata 1320

tattttaaaa attgctggtc aaataataaa gtacgccctg gtggtaaata ttgatatgac 1380

tcatacagaa tgtttaatat aaactccatg ccgccgctta taaagaaaat aacacacaag 1440

aactggctga aaagcaagcc aaaaagtgag gtttcagctt ctattgtaga attgaatatc 1500

gtacccattg tcgcgccacg taaagtatca agcagaaacc ccgccatatc aacggcccaa 1560

aagggaaccg ccgcacaaaa cccaattaaa aaccaataa tcacctctcc ggtgactaac 1620

cctaaccagc tgtaatcttt accaatatgc atcataatct tctgctggta aatgattggt 1680

aatatgggaa aggtaagtga cataagcacg ccattacgta aaatcgcgga ccctaaactg 1740

ccacttttta ataggggaag taataaagag aggctcaatg gtcgaataaa agccacagcc 1800

aatgcaataa gccactcatt tacctgttgt gccattcaac catgctctcc aactcgtaac 1860

attatctgcc gggtataatt caacaggata ccgctaagcc atgggtagct gaccattaag 1920

gttattgcaa ttgccaataa tttaatcatg aactgtagcg tttggtcctg tatttgagtc 1980

aaggcctgaa caaggcttac gatgacacca actaccgatg ccaccaacac caccggcata 2040

gacgtaaaaa ggacgatcca taaaagttgc gttacaaatt gcgtcaattc agaatcattc 2100

atgaaaagct ctgtaccaat tgcgccagtg tcagatccca accgcctgcc agtaaaaata 2160

ttagcagctt aaacggtaat gaaatggtca ttggcgatac catcatcatc cccatagcca 2220

gcagtatatt tgaaataagc aggtcaatag ccagaaaggg aagataaata agtaatccaa 2280

tccgaaatgc ctgcgtcaac tgactcaccg taaatgccgg aattaatatg agcaaagaat 2340

caggttttat ctttcttttt atgtcttcag gccaggttcg ttttatcaaa ctccgaaaat 2400

aattggcttc cttctcttca gagttttttt gcaaaaactg tcgataaggc gctaatgctt 2460

tactgtccca ctcagacgtc cagaaaggag cgccagcgac ctgaaccgga tgccagcgct 2520

cttttacagc taatagcgtc ggccccataa tgaataagga aagtacaagc gcgaggccat 2580

acagtgcgat atttggggga acttgttgaa tacccagagc atttcgtaaa atcgaaaata 2640

ccaccgccag tttaaggaaa gaggttccca tgacgataat gagaggcagt attgaaagca 2700

gaaacaatat accaatcagt tgcaaaggcg aatcgggtaa agacatactg tatctctcat 2760

gacacgacct agaacgctat tatattgttc gcatattatt tttcttatca ggtttacgct 2820

gtatttttgc aaagatacca acgtgtaata cgcaccataa attcattgcc acaggcaatc 2880

aactcacctt gcccaataat acggtcattt actcttatcg tcacctctgg cgcaaaacat 2940

ccacctacag gcaaaacgtc cccgtttta agttgtcgta attgtccaat ttccagactc 3000

gcacgtccga tctcaaagag cacctgttgt ggtatctgct caagttctac tgaagatgtt 3060

ccgtcactct ttgacattgg actccctgac gcaagtagcg tttcgatatc ctggactaat 3120

tcatcaaatt tcatcgtgtt atcctctgtc agcaacaccc tcgcgtagat ccccccaggt 3180

agttgaatag caaaaaaacc gagtctgatg tcgccaaagc aatgaatccg aacgcccatg 3240

ccgatttcga tagactcaag ttcaattaac gtaagctggc accagcctaa atatacaggg 3300

actactacag gaggggcagg ataaatctgt tgtcgcgcag cagaaagctc tccgactata 3360

ttgcgcaaaa aacccgttgg ccatgtaaaa ataatgctat ggaactcatg ttcttcaact 3420

gtccatttaa tatgcaacgc tagctgatgt ggtagattac tgcaggatgt tggcggttcg 3480

ttctgacaga gggttgcatc actggcttgc aataacggcg ccagcccca ttcagctatt 3540

ccatatagca attcaggatc gatagccgat cgattagcgg tgccaattaa cccttcacac 3600

cagcgctgcc agcattcttc tgcaatccac accctaccca gctcattatg ataatttatg 3660
gtaaataatg tcccttgctg tactggatat tgttgcatac tcaatgtcag ctcaccaatg 3720
gtagcgcctt gcgttggaag catctccatc cacggacgct cttcattcgc tattcttaac 3780
atagaatatc tccagggaaa ttatataccc catatgcagc aactgagact ccagccactg 3840
ctttagcgct ttcatcgaac ggtaaatttc atgatgaggg acattgattc ttaactgaat 3900
tagcccccct gattcacaga cttcacactc tacaccgtca agataaccgc cactaacacg 3960
ataacgttgc gtaaaaacca cgttcttatt caatgctgca ggaggattat tctcaccaat 4020
gggtaatgcc tggtgcatga gttgttcaaa gtccatacgt tccgcctccg cctcgttatc 4080
atcctgataa gactggcatg gcaacccaag acttccctca actttggtaa tacgcatcgc 4140
ttaataccat agtaattttt tctttctttt tcataagcgc attaaaattc ttctgtaatt 4200
cgcttcgccg ggagacaagc tgctgatact gattctctaa ctgctgccgt tgcgtcaaaa 4260
agctctgcgc ctgagtgaat aacccggcca tttgttgttt cttatccaac aataaatgac 4320
aagataacgt accttgccag cccattaatt ctttcagtct ggtagacact gctaaagcgc 4380
gcgtctggca aatctgctgt tccgtaataa tcgcctgttg ctgctgatca agtacggtaa 4440
gcttgccgcg taattgcttt tcacgccgcg cgattatctc cagcaaagtt tccatgatca 4500
ctcggtgagt atttggtgta atttttctat aagtagctcg ggtccgcata cttcatcctt 4560
actttgtcgc aaaaatgtgc aaatatccgg ataggtatca atggctttgt cagtatcagt 4620
atcaactcct cgctggtatt ccccaatgcg tattaacagt tcaacctcct ggtaaagcgc 4680
caggcgccgc cgcaatatcg ccgccagttg acgatgctca tggctggtaa cgactggaaa 4740
aacgcggctg agcgttgcca gcacgtcaat ggcaggataa tgcccccctct ctgcaagccg 4800
ccgggatagc acaatatgtc catcaagtag tgaacggact tcatccgcca acggctcatt 4860
catatcatcg ccttccacca gtaccgtata aaatgcggta atactgcctt tttcccccat 4920
tcctgtacgt tctaaaagtc gtggcaatgc actaaatacg cctggcggat attctccaga 4980
aactgcggtc tctccggcgg ccagagcgat ttctcgtgcg gccctggcat aacgcgtcag 5040
tgagtcggca agcaagacga ctcgctttcc attatcgcga aaaaattctg ctatcgtggt 5100
agccacaaac agcgccctca cgcgctctaa ggcgggtctg tcagaggttg cgacaacaat 5160
gacacagcgt tttcgggtct cttcagacag tgtaaaatcg atgaattcgc ggacttctcg 5220
tccacgttca ccaattaaca ccagaacatt gctgtctgcg tctggcgcat tacacagcat 5280
cgccagaagc gtgcttttcc ccacgccagg agcagaaaaa atacccactc gttgcccttc 5340
gccacaggtt gcaacgctat caatagcgcg aatccccgtc attaatggtt gagtgatagg 5400
ctgtcgaacc attgcgggag gaggcattgc atcatagtct ttccagcaga cgtcgggcag 5460

ttcgcggcca tcaaggggac gaccaaaacc atcaatgact cgccctaata acgcttcgcc 5520
cacgggaacc tgatggcttc gccttaaggc catcacttgc tgcccgcagt gaagtccgat 5580
tgtactcgta aaaggagata gcaaagcttt gctgccatta atccccacga cttcagcaag 5640
ttcttctcca ggctttatac agcacaactc acccataaat accccaggca accacgcatt 5700
taacaacgtt gcgctgacat cctgaattcg gccccatcga caataaccat cggggggcgg 5760
atatttcagc ctcagacgtt gcatcaattc attcttcatt gtccgccaac tcctcttcgc 5820
taaggtcaat actttctacc acttgtataa ggctctcctc tcctaattcc tgccatgaca 5880
aaatcggtac gtcgaacaag gtggcttctg taatttttcg caagaaacgt cgggtgtcga 5940
cagaagtgac aatgaataat ttggctgact gcttcagcgc ctgctcgata agttgcagga 6000
tctgcgtctt atgacgagac gacagcgcag tataggtccc cattaccgtc tggcgaatgg 6060
attcacgcac gaggttctca ataccttcgc cgatccgcaa aatcggcagc ggttttcctt 6120
ccggattaag acgacgcaga atatgacggc gaagcgcgat acggacatat tctgtcaaca 6180
tcaggacatc tttttcacgt ggcgcccagt caattaaggt gccgaaaata agacgtaaat 6240
ctctaataga aacccgttct gatacaagcc gttgcaaagt ttcagcgatt ttattaatgg 6300
gtaactggcg ttgaagctct ttcaccagct cagagtagtt tttttccatc gcattcatta 6360
gataacgcgt ttcctgaaca ccaataaact ctcccatatg ccgaagcagg acacatttta 6420
ataaggcaga gatacgttgg ctgcccgcga aaacgtccag tccaaaacct tgcgccttat 6480
gggccatgtc ttttgtaagc caacagatct gccccatccc gttcggtaac gtctggctgt 6540
cacccaccac actagcgtcc gcgcctatca ataaataatc cgcctgagcg ggaatagata 6600
aactaaatac gggttcctga tatagcagta ccgtcaattt ttcggtgggt tcaggcaaaa 6660
cctcaatatt cacctcaggg agagggacgc cggtatcctc aaataaaaac catctcatgg 6720
cgtcaatatc acgaatcagg tcggcagaat gtaacgtcgg gctaagacgt aagattagag 6780
gacatgcgcc gggaaccata ctatcttttt ccggtgcttc gacgccattt gcggaaacca 6840
cagacttttt gcggcgaatg aggataattg gcaatgctaa caacgctgaa aagaaagcga 6900
gagtgataaa aggaaagcca ggaattaaag cgaggagcat taaaaccaca gcggttaata 6960
tgagcgactg aggttgtctg gcaatttgag aactcaactc tgtcgccagg ttctggcgtt 7020
tctcacccgg gacacgggtg acaataattc ccgcgctaag ggaaatcagc agcgatggaa 7080
tttgcccaca taaaccatct ccgattgaca gtacgctata agtgtgaaca gcctcactca 7140
tcgacatatc atattgtacg atagcgataa tgataccgcc gataatgttc accagaacaa 7200
caataatacc ggcaatcgta tcgcctttaa caaatttcat cgcaccgtcc atcgcaccga 7260
gaaagcggct ttcctgctgg acatgctgtc ttaatgtacg ggcatggtct gcatcgataa 7320
ctccggcacg caaatcgcca tcgatactca tttgtttgcc tggcatccca tcaagcgaga 7380

aacgtgcgct aacttccgcc accctctcga tacctttgt aatgacaata aattgcacga 7440
tagtaatgat ggtaaatacg accaacccaa cggtgagatt tcctcctacg acaaacttac 7500
cgaaagcatc cacaatatta ccggcattat gttgtaacag taccagccgt gatgtgctga 7560
ttgtgagtga caaacgatat aatgtagtaa taagtaataa agacggaaat accgataaat 7620
cgagagggtc actaagataa atagcaatta agagcaggat cactgaaaac ataaggttga 7680
tagtaatcag gatatcaacc atccaggtcg gcaaaggtaa cagcatcatc acaatagcga 7740
ttaataacac cgtcgccaga accatatcct gccgacccgc gcatacactg agccactgtt 7800
gcgccctgac tccctcacct aaccatgaac gcattgcgac tccagaaatt ttatttgtcg 7860
atgatgtaat cgtaaccaga gctcggcgga gctggaaaga ggtggagaac aactcattgc 7920
aagcccatcg cgcaacaaaa ataatcgttg aggaataccc tggaacgctg cgggtttcca 7980
gttagccaac gctttaaaaa gcagttcttc gtctaagaag gtttgcttga gtatctgaca 8040
taagtgaata cgacagttat gatagtttag ataggtttca tattgtcctt gccgccagaa 8100
catattggtc gctaaaggcc gttcagctaa tcctgctaat tgaataaaaa gctgaatatt 8160
acgttcagta atgagatccc aatccatcct gacgcctcat gatgagccag aaagccaatt 8220
tacctaaata ttgaaagcca ggtatcagaa taaaacctga tttatcttta cttcacgaag 8280
cgtttcgaga atttgttcac gttgatcttc gtcgttaaaa cagttatcgg gtatcagcat 8340
aaactgcgca tcaagttgtt ggagtaaccg attgaacatc ttcgatgaag aaactatagc 8400
ggtaagtcta tcaagcaacc aatcactgaa aagccagcgc tcacaaataa tatcgagtag 8460
tagcggcagt aatgtattag gcggcaactg gcaaatccac tcctcacgct ggcactcttt 8520
ttcaaggcca aggaataaca gcaaacgacg caaacgtact aatgctgcgg ccaaacgact 8580
ttgctccgag ggttcgatgc atatgctaag ttcaaaggct attgctctta gcaaaatacg 8640
gacccgttca cagcgatccg gccagtctgc cacgcgtctg aaccactgcg ataagggcat 8700
ttcatcgttg tctatcgcct gttgcataaa acgcttcagc gaggacagcg tagcggtatc 8760
cacttcgcca agttccagta aactaaaaac ggcaagttcc catccctcct ccgctgtaag 8820
cgtatccagt tgcgattgca aatcgcgttt tttcttttt gacaacccgc cggcagtaag 8880
cgccattgca agagcgataa tttgatacgc attctgtaaa tcaggatcac tattctcttc 8940
ggtaagcgga cgcaacgctg ccccattatc ctcctgtatt tgttttatca aacgcagcaa 9000
agcctgctgc ctgcgctcca gtttctcagc atcagtgaat ttattacttt cgcgcagttt 9060
accactcagc gccattccta tttcttccat cgtctcatag agcgctgccc ccgtcgtttc 9120
ctgtaactcc tggagagcta acattgaagg cgaaataacc tcttgttcct ctataacctg 9180
gccaggggta aatgctgtag gggcgtcat ttttatctca ttaattttaa tattcatcgc 9240

tacctctttt atcttcacca ttacgtaacc atttcagtaa cgcgttgaaa tgacgagaaa 9300
gtgaaaactc aacggcatat cgtgttgagg aaagttcagc ctgatcggga gagaaaccag 9360
gctcgataat caacgtaaac cgcttgccaa aagtttcacg catcaatgcc tctttttcag 9420
gatgaatacg caaataaagc gctccctctt ccgccatagc cgtggcctgg cgtgccagac 9480
gatggcacat aacactgtct accgactgtt ggtcgaacca ggccaacaga acctgttcta 9540
tactattttt aatatgatgc gctgcgtgat cgaccaatga acgaaattga ttttcatctt 9600
cttgtaaatg ttttacatgc tgttccagcc attccacttc cattttttcc agcgtatttt 9660
tacgcaagca cgctagttct tgttgctgct caactttctg ttcacgctga taacgatagg 9720
cgtctcggat gatttttca gccttacggt aagcggagct cacaatagca tgtgaaactc 9780
tcttagcttg ttgctcttgc gcaaataaag ttaattgtaa tgttatccac tgtgactcaa 9840
taatatttcg agcgggtagc ttatggttaa tttccgtcag aggaagtgaa gtaaaactca 9900
tagcaaatgc tccatgaaga taatctcggt aagagaagtc ttcggccaaa gtatacgctg 9960
cggagggggt aataatacta atagcgcatg taaaaccgca tcgtcatgcg cttcccgatt 10020
aagaatggcg gtaccgatct gcaatgcagt ttgttgcatc acttgcggag gaagtatttt 10080
gccatctctt tgccccaacc aaccatatag ctgccagatc tcatcctcgc taaaccactg 10140
tagaagcaat tgccgatact ctggtagcat aaaatagtca ctacacctga gtttgaataa 10200
tcccagccca aaggcaaatg ccgatatacg cggcgcaaga cgaacctgcc gcttttgcct 10260
gtcatttaaa caggctggaa taacagagct tcctcttagt ctatttaacg ctctgtcaag 10320
aagacgatcc aactcgggcc gatcgccata acgccagcag tttgaaagat gaaagcccag 10380
cttatccagc cattccggta cagcgtaacg agcaggttgc cagaaataac gataaagttg 10440
caacacctcg ggatcaggtc ggctcaaaaa cggcgtctca ggcaaaaata gccgatcagg 10500
atgcccactc ctaataacag tcctgtcaac gataacatca actgataagg gtatttcatc 10560
aaccacttca ccaccttccc tttattggcg ttgataacgt ccataatcca gaatgtttgt 10620
ctcgcgggta cgtcagctac cattctgaat tcagcaggct gcatcaagag actaatctta 10680
ctgtattgca acccagggat tgacatctct attaaatcct taatttttac ccgaaaggcc 10740
tccatattga cctgtggtga atattttata aatacggcaa ctgagctcgg agaagcgtta 10800
cttccctcat cataagtcgg tagcgcaatg gtcacttttg cattaatcac gccctccatc 10860
tgactcagca ttccttcaat tctttgttct tttaaaaaat taatcttctg ctgttcttcc 10920
tggggtgata ccactaactg attagccgga aacatcttat ccgccgttgt aaactgacga 10980
tgcggataac cgttaagtct aagtagctca accgcattaa taaactgcga ctgctcgaca 11040
cgtaaggtaa caccgtcctc ttcctgtttt ttttccgcat caatatgatg ctgcataagt 11100
aatgccagca tttgattcgc ctcatcctct ggcaatgagc gataaagatc cacatcacat 11160

gccgtaagaa agaacgtaag gacagtaaga aatactatac gatgagcctt catgccatgt 11220
tatccagctt attaagcgct tgcgatgctg cgcctgatat tctggcaaga taatcgacgc 11280
ctaccgttaa ctgcatataa tccatttgtc tggtcaacat aacctgcggt aataaagcac 11340
tggcgttact ggtggatgct tcatctttca gcaattgttc aaaaaaatta atttgctcct 11400
ggctcggttc tgcagaggac tttacataag attgagtgct tacaggcact acgctcatat 11460
cagaaatatt caattttcaa acccctcatt tggtgcagga aataacagac gcagcgccat 11520
agcctctggc aaatctatat ccgataaaat tttcgcggct tttagcggct catttaaacc 11580
cgccaacaat aatgccagac ataccaactg taattttta tccggaacaa taaccgttag 11640
cgctggtaac atcgcatgta cctgggaaat caggctatgg ttaacgcccg caaacatgat 11700
ttccagcagc aaccgtcgaa catcgtcgct aataacttca gattttagca atgattccac 11760
taagcatatc cttgatcatt ttgatcagtg aactttcgta attaataaat gtagaatact 11820
gctgtaaggc aaattgcgct ttaatcatcg attctgggtt gagcaaatca ttaccattca 11880
ttttgtcatt aatggcctgg cctgcctggt gcgccatgtg ggagagcata tccactaatt 11940
gtgcaatatc cataatgctt ttccttaaaa taaatacatc gtaaggatac tggcaacata 12000
gcaaaattta gaaagcaatg aacatccggt atatacctga aaacgattac tccggcgcac 12060
gttgttctgg cgttacctga gccagcaaac gatataatgg gctgctgacc tgcataccgg 12120
tcattgccat cccatccata ccgaagcgag taaaactcat tagtccatag gtaatatcat 12180
taagacgctc taataaatga ggctgtagtc ccaaactacc actccagtat gaatgcgtca 12240
ttaccgtcgc ggttaaggct aatctaccgc ccagggagac ggctttagca atcgccatac 12300
ttttgcgttg attggcgaaa caattagcaa tataaaaaac ggcattgcct atactgtcgt 12360
gagccatagg caaatgatgt ttatgatggt agataagaca ggcgacatcc gcgatggcaa 12420
tagcaaggcc aatccctgcc agggctacga gcggcgcgcc tccaccactt agcactgtta 12480
atgctattcc agcggaacag cataaaatct gtccgcccaa aataaccgtt tgccaactaa 12540
aaatttcttt tggaaaacac tctatcactc gtttcgcaag tccggccaat aaccgctctt 12600
ttccttgttg aggacctatt ctaccactct ccatattggt ttccggatgt ggcaatgagg 12660
gacatggagg tgattcctca ggcgcgttaa caggacgttg ccctcctacc tgagcatttg 12720
ggctaacagg tttcatggtt ctccccgaga tgtatgacca gaactgtcca ttaatgcagg 12780
tgcagtagca gattgacaga gcgctgccat ttgttccgcc aataacgcac tgggatcggc 12840
ataaagttca tcaacagaat tttcctgatc gtcgccagag ggcgggcaa ggcaataatc 12900
cagtaccgca cctatcgccg tcaggctaac ggaggtaatt acactcccca tgtccaaaga 12960
ggccgcaata ttttcagccg cgggcagtgg aaactgtagg ggtaaaacca acatagaaat 13020

agcgattcct gaacgtatta ataaagaaag acaattagca agggtgttag cgcagttaag 13080

acttgcccca cattttaagg ccagcgcact gaccacaaga gcaacgctat cactggcggt 13140

ttgtaatggc tccttttgct gacatatcga ttgataatta tgatacgcac agcaagcatc 13200

cccaatagca atcacgagcg ccgcccccgc aagaatagca atgggtaatc ctgccccgcc 13260

agaaattacc gctgcagcaa ccgataaccc aaacacgact gtcgcaccca gcgcacgaat 13320

agtgtattgc ataaaatgta tagcataatc cctctgctgc cttatttgtt caggcgtaag 13380

cagcacaggg gctgcggggg taccaggcgc tggaatttca gggggaggaa acgatacctc 13440

cttcgcttgt atatcggaag gaggactatt accatcgact atattacttg ccgctgacgg 13500

aatatgaatt ttcatatttc gttctgttat ttaagcaata agagtatcaa ccattatttg 13560

cgcattctgg cgaatctcac tccatgaggc atccgcataa ctcatcttga ttgcggtttg 13620

aaaagcctct ctcgccaacc cgggttcccc catcattttg agacagacgc ccgtttggta 13680

aaccggttct ggatggctgg catccagcat caaggcatgt ccatagaaat taatggccgt 13740

tgtgtattct ttaagcatca tccaggtgcc agccaatgca atatgggcac gccaactcca 13800

tggctgggcc atcaccagcc aactaaaatc gattacggcg cgcgaataat ccccctcctg 13860

ccatgaggcg taaccactgg cataaacggt ttccggatca acggataata gctgtttcag 13920

aatgtcttcg ggtattttat ttttctgatc ttctttcatc atcataccta ttgattgtta 13980

ttttcacgtg ataatgattt acgttaggaa ggtcatttaa aaacgtcgct ggataagatg 14040

ctcggcggat aaaactgtcc agttatcgcc atcaagctgt gtaaaggtcg ctcccattac 14100

tgtcaggatg cgcaataatt tcctgcgtag catggctttt ttttcatcca gaacgtcggt 14160

gattatcaac atctttaaac atgttaactg cgggtgatgc acaaatatcc cgcgtaaaag 14220

tcccagtaag tgaaacaatt gctgtggtcg aggttgcccg ggcgtcaggc gcctgaactc 14280

acaaataatc atttcttttg cctcaatacg atagatcacc aggtaaggcg ataatataaa 14340

ctgctgccca agtaatatgg cggtctcccc taaatatgca ggctcagtaa acacctgatg 14400

ccgacgcaac cattgctcta tttcttgcac catgtttacc tcgttaatgc ccggagtatt 14460

tcagcaagaa ccgtgaccag tgacgacccc acgccgatga tttgctgcat aatttcagtt 14520

gctttctccg taatttccgt cagggattta ttataagatt gggccccatt ttgttgcagg 14580

tcggcaatcg ctttatcttg atcactttga cgttgcgcta caccagcccc caggcccatg 14640

acgcccccag ctgtgtggcc tacggcttga cccgctataa gaccggtttc cccgcctacg 14700

gcccctaatc ctatcgtcag tacacccgac aacattgcgc cacccgcagt aatcattgat 14760

gctctaaacg cttcatcaat tgttttcatt tgcgtctgta aaacattgac ttgcagttcc 14820

caggccagcc gttgtttttc tacgttatag ctgcgcatga tatcgcgcag ctttttggca 14880

agctccatta gcttcatcca gatatcatca aataacagaa gcattgattc agtacccatt 14940

ccctccccgg agggagatgg agtggaagaa ggtgttaaca aggaaggcgc tggtaatacc 15000
agtgctacgt tactcgcttc catattttta tcctcagatt aagcgcgata gccagctatt 15060
ctcgcctgaa cgctactata gtgatcaatg gtatctaata catctctaag cgcggcaccg 15120
ctccccttat aaagcttctc taagcgtttt tgttctatct ttttctggtt ttctgtttgt 15180
tgcattatga aatccagaaa ccgttgctga gttattaatt gctctatttt cttttcgatc 15240
ttcgcttttt ctgtgttaac catgccagta ttcatctgac tggcgccctc ggttgcacat 15300
ctgatagcct gtaagccttt aaatgaacaa tccctcagta aggcatacag gatttttttg 15360
aacatattga agagaacttt attacggaat ttttttaaca ggaactttcc accttcttgc 15420
acacattttt ccagggcttc ttttgccgct tcttttgcca tggctttcac cccctctttc 15480
gtaaagcttt ttcccgcgct tcgcgtcata ttattttcta cgttacgaga aaactcggca 15540
gcctcctctg ccatctcatt cgccatagcc atttcacgtt caagcggctc aaattgtttg 15600
gaaaaacttt cgctcacttc ttcgccaaac ttttcagcca actcctctat ttctgcttcc 15660
cctgcaccta ccatacgctc aaccacttcc tcgccaaaac cggagtcaag cacttttgca 15720
gctgcgccag ataaacctct cgtcgccata aaagcacggc caatctggaa aacatccagt 15780
gccagcgcga cggcttcaca accaaattga atcttacttg tcacgtcaat aattgcctga 15840
caggtatcgt ggtcagcacc gcacatcatt gccgtttcgg ctccggcttt aaccattcct 15900
gcacaaccta cggctatata agctacgccg ctagccattt ctgcgggatt accggacaga 15960
aacccctcca caacttttaa ggagccaatc acagtttcaa atatgccggt aatccagtca 16020
aaaatagcgc caaaaatgcc cgctttacgc gctttatcct cctgctctat cgctttctgg 16080
atctgctcct gatactcctt tacctgctta tcacgtaatg cattttgcac ctcagttgcc 16140
cgctcaagct gttggcataa cgattgagcg ttattaccaa aaacgctgag tattaatgtc 16200
gtcatcaaca ttgataaaac cgcgggattg gtctgcaaaa agtcaggcaa tgatagacgc 16260
ttatgatttc cgggtacggc atcaagcagt tgcttcaacg cattgcttgc ctcctgaaga 16320
ctaattttcc ctgataacag gcacgcggcg ttgccatcgc caaaagtaga attcacacga 16380
tgccggcgct ttcccagcga acccgaggaa acgcaactga cattgcttaa gtgatgatgt 16440
gttaaggcgg ttactcctgc ggtgctgtcg ctattactgt gaattcgatt catttttagc 16500
tcctgtcaga aagttgctgt aacatctttt ctgcacgctg tcggagaatt tgatgttcac 16560
tgacctcgcc gcaaatacgc accacggcct ttaacgcttt gattgcataa cagacgttat 16620
cacacgcgag atagcattcc gctgcggccc atggcgcctg cggcgcatca atcttaattt 16680
gtgccgcgcg tccataagcg tatatcgctt ccccccaatg tttttgagcc tggcagcatt 16740
cccctaacct aaaccagtag tcaaatgacc aggcatcata tatcgtcaac aattgaaaaa 16800

gtcgcgctgc gccggcgaac tcttttacct ccataagctg catggcatag cgatacagag 16860
tattaagcgg ctgtgtaaca tcgtcatcca acaacatacg cagcgagccg ccacgccgga 16920
aaaaccgcat cgtgtcatgt gcctgttgta gggtcgggtc ttttttcatg agtacgtttt 16980
ctgcgctatc atactggaaa tttcccccca cttactgata agccctgtca gttgggtaag 17040
gacagcgtta agctcctgag acatttttg aattgttatc tgcccctgac tcataagatc 17100
ggtattccgg ttggcgtcat tatccaaagc cgctttgatc gcctgtaggc cacctttatc 17160
cagcttccca tgatcgccat atttagccat ataatcatca atggtcatac catcgatgag 17220
aataccatta tcacgcatgt atttaattac atcctcaggc acctcctctt tggttttagc 17280
atcccctttg gctgctttag caatcacctc atccatctca tttgactttt cctgggtatt 17340
tctggcacgt tcagcgttct tctggacttc aataaattta ttatttgcga tatcctgaat 17400
aaccataagg agaataagca aaacaccata cccttcggca aacggatttt gctgggataa 17460
gtcatcctgg ctcccggtat cagcgttgct gacgccgaag ctatttttaa acacaatagg 17520
gttttgactt ccccataaga tgtttcctga agacattatg ctttaccttt ttgtttttcc 17580
tgacggtatc tccaccgggg cttgagcatt aagttgtttc agtcgtactt caagttgttt 17640
aaacaaactt actattttct ttaaatcctt ctcggcctcc tggttaaccc cggcaacgcc 17700
ttgtggaaat aggttttgaa gaatactctc tgtctctctg ctctttttgg ggctctctgc 17760
cctttcagca agctgttgac tcaccttagc ccggatttgg tgaaattttt taagacagtg 17820
atttagctgc atgtaacttt gctctaaatc acgatattca ctaaacgcag ccttttcttt 17880
tatcattatt cccctccata tacacgatag ataattaacg tgctaactaa gagcctatcc 17940
cattagggct attttacttg ccattttgaa cctgggcagt gctcaaaatc ctcacgtact 18000
acgtgtacgc tccggttttt gcgcgctatc cgtgtccaaa ctggctgcgc caattaacgc 18060
ctggtgggat aggctctaag atatttttac tttactcttg ctcactcact acaagtgcgc 18120
tgttatggta acgataataa ataatgttga tgatattttc ggcctgctcg atcgcttcaa 18180
gcaataaggt gttttgctga tattgctgcg gatcctgtaa cttgccgttg ttctctccta 18240
actgtttccg ggcagccctt aattgtaaaa ttatgccttt ggcctcttca cgcgaatgaa 18300
gcagcaaatc ttctaaccgg gtcaaagttg tcattttcca ctcacttaaa atctaatgga 18360
tagttaatca aagtatcata atgtttaatc gttaccacat cggcactcag atggacaatt 18420
tctcccccat tgggtaacaa tgcccctaca cgtaaacgct ctttattcgt cagtaataag 18480
taattaccat ggcgactctg tacaaagcca gccactggcg caggcagata cttgctttca 18540
tcatgggaag gcgcaatatc ctgataaatt aaagaaagag cgggatcctt tttctttaat 18600
gctgctaacg tttcttgcaa aatgcgttga tgagattcat ccagtacacc actgataaca 18660
aaagagcgcc gcattggcgt aacattgaca agccccacta aaccgttctc tattatcgca 18720

gaaataatat catctccctg agactgatga gagtgactaa tctgccagtg caataacccg 18780
ggaatatctg caagtaatgg ttgaacctta cgccattgct gatccatttg tatatcatca 18840
tgaattaaca cgctccccgg cccttcgctg gatacttcag catgcgggta acccattttt 18900
atcaaaacat cctgcacttc tcgtaccaat aagtcatcac agattacacc atcccgatac 18960
atgacccccc atgattcgag agtcgctctc accttttgca tctgttcgct tgacgagcaa 19020
taaccggaca actgcaggct gccatcttct ttccattgcg cccgcacata atgaatattg 19080
cttttgtcta ataaaaactt aacccgcaaa ggtaagtcat ttaccgtttc aggctgacca 19140
ctaatactta acaggacacc cattccaccg atgaaaatca agaatacgcc agccaaccac 19200
cagtaccctg atctggaaac gggtatttga taatcagcaa gttcacaatc ctgtttacca 19260
aacgcgatag ccactcccgc aacctgcaaa accccactgg atggtagcgg cttatttgga 19320
ttaaatctgc ggccattaac tctaactctg gctttcccgg catcaacaaa taaattatct 19380
gcctgttctc tcagaataat tttttcattt atagtaagcg gaatacaaat atcgcatcct 19440
ttctcccccca gtgacaggtt accttcattc agccatactt cccggccttg taaaacgtga 19500
cctaaaaaac gtattttcca ggaactcttt ggattaacca tgagatatgc cattatttac 19560
tactgaggct ttaatcaaaa aaagcctgat tacactatgt acttgagtcg tatcattgcg 19620
aaacaaatgg cctacgacag gaatatcgcc caataaagga attttatttt gcgagtggat 19680
ttgtttacct tgtttaaatc ctcccagcaa tagactttgc ccggccaata atgtggcctg 19740
cgaagcaatt tcagaatttt gcacttcggg cagcgggtct gtttcgcttt gcgtatcact 19800
ttgttgtcca tcctgaatat taagatcaag cattattttt tgcgtgccat tatcatttaa 19860
caagcgaggt gtaacgcgca acaaagaacc cgtagtgatg gattcaagtt tagccacttt 19920
ttctccctgc agtttggtat agaaagtaat atttttatcc agcacagcct ggatattatt 19980
taaagtcacc acagatggct gggaaagtac ataagcctga gagctttttt ccagggcatt 20040
caaacgcacc ataaagtttg aggtatcgct gattaccgtt gaaaaaccgc tagcaccacc 20100
gtcattcaaa cctgtattga acgcaatttt cttgccaccc agcgacactg ccgttcccca 20160
gtcgatgcct aactggttaa tatctccagc attaacatcg ataattttca ccgaaatctc 20220
tatcatctgc tggcgttgat ctaattctgt gataagtttc cgatacccgg ccatattgga 20280
cgcataatca cgaacgatca ctgcattctg gcgtgggtcg gcagcaaaca tgggcaatgc 20340
ctgtgtagcg ggtgaaccat tgttcgtcga tgacgccggt acgctggttt tactcatctc 20400
acgcaataca ctcacgaccc ctggaaccac gacggactga tcgcgatatt ggtattgggt 20460
atccatcgca gtggcatact taagcgtgta tatacttaca ctcaccgcac tgtcttttcg 20520
tttgattaac gcattatcca gcactgaagc taattgacta atacgagtca ggcagctggg 20580

aacaccgctc acctccacag ctttggtacc ggtaatttct ttaacctcgc atcccggtga 20640
tgaaagaata ttctggctgc gtaagtaatg aatgaaccgt ccagtagata aaatattgaa 20700
agtgataacc tgatgtttta ataacgatgc aggatataca tataacatgc tgccatcaaa 20760
ccaggtaagc aaatcatatt gtgctgccag gttattcaaa atatcgaccg gtggtccagg 20820
cggaattttt ccactaaatg tagctgttat caatgggcta atagtaatag ccgtatcata 20880
gttctctgag agcagatgta aaacctctgc taatggcatt tgtctggcat aaagggtgaa 20940
gtcattacct ttccatgata actcatcact ctttgctgta ttgagtataa atagtaaaat 21000
taagattaaa cgtttattta ctaccatttt ataccccacc cgaataaagt ttatggtgat 21060
tgcgtattac attttttaaa atgcaagtta aagccaggtg tttttctatc tcaatagcaa 21120
taagctcaga gctactactt gtggtataat aaccgtttaa ccatccccca tccgctgtga 21180
gctgtatagc ataatcatgg acgtccgggt gtgctgcaag cagtagtgtc acataggcaa 21240
gacaaggctt aggtaagctt tccaggtcat ttaagaacaa agaaatagaa aatgcttctg 21300
agaaaatttc tcctctggca ggatgcccat caatagtcat tatccaggat cggctattac 21360
cttcggcctt gatatcctga attaatggaa tgccttttaa aactgccagc atgaatccct 21420
cctcagacat aaatgggagt ttctatcaaa ttcgctcaca accacatccg taaaaagcct 21480
gattcacatt tatttcgact atacttttct tgtacaatat caggatgctg tctacatata 21540
ccttgtcaca ggcgattcta tcattcggat tttccgataa attcacaatt acattttcag 21600
cactgacata aaaacttaca atttgaaaaa tcatttatta aatgaactgt tacgatgttt 21660
ttacatcgcc atcttattaa aaagtaattg tagtcatcga ctgggttata tatgaagaaa 21720
tttatcttcc taatgataac accatcgatt aatcttctga tgaaactata tgtactgcga 21780
tagtgatcaa gtgccaaaga ttttgcaaca ggcaactgga gggaagcatt atgaatttgc 21840
tcaatctcaa gaatacgctg caaacatctt tagtaatcag gctaactttt ttattttat 21900
taacaacaat aattatttgg ctgctatctg tgcttaccgc agcttatata tcaatggttc 21960
agaaacggca gcatataata gaggatttat ccgttctatc cgagatgaat attgtactaa 22020
gcaatcaacg gtttgaagaa gctgaacgtg acgctaaaaa tttaatgtat caatgctcat 22080
tagcgactga gattcatcat aacgatattt tccctgaggt gagccggcat ctatctgtcg 22140
gtccttcaaa ttgcacgccg acgctaaacg gagagaagca ccgtctcttt ctgcagtcct 22200
ctgatatcga tgaaaatagc tttcgtcgcg atagttttat tcttaatcat aaaaatgaga 22260
tttcgttatt atctactgat aacccttcag attattcaac tctacagcct ttaacgcgaa 22320
aaagctttcc tttatactca acccatgccg ggttttactg gagtgaacca gaatacataa 22380
acggcaaagg atggcacgct tccgttgcgg ttgccgatca gcaaggcgta ttttttgggg 22440
tgacggttaa acttcccgat ctcattacta agagccacct gccattagat gatagtattc 22500

gagtatggct ggatcaaaac aaccacttat tgccgttttc atacatcccg caaaaaatac 22560
gtacacagtt agaaaatgta acgctgcatg atggatggca gcaaattccc ggatttctga 22620
tattacgcac aaccttgcat ggccccggat ggagtctggt tacgctgtac ccatacggta 22680
atctacataa tcgcatctta aaaattatcc ttcaacaaat cccctttaca ttaacagcat 22740
tggtgttgat gacgtcggct ttttgctggt tactacatcg ctcactggcc aaaccgttat 22800
ggcattttgt cgatgtcatt aataaaaccg caactgcacc gctgagcaca cgtttaccag 22860
cacaacgact ggatgaatta gatagtattg ccggtgcttt taaccaactg cttgatactc 22920
tacaagtcca atacgacaat ctggaaaaca aagtcgcaga gcgcacccag gcgctaaatg 22980
aagcaaaaaa acgcgctgag cgagctaaca aacgtaaaag cattcatctt acggtaataa 23040
gtcatgagtt acgtactccg atgaatggcg tactcggtgc gattgaatta ttacaaacca 23100
cccctttaaa catagagcag caaggattag ctgataccgc cagaaattgt acactgtctt 23160
tgttagctat tattaataat ctgctggatt tttcatgcat cgagtctggt catttcacat 23220
tacatatgga agaaacagcg ttactgccgt tactggacca ggcaatgcaa accatccagg 23280
ggccagcgca aagcaaaaaa ctgtcattac gtacttttgt cggtcaacat gtccctctct 23340
attttcatac cgacagtatc cgtttacggc aaattttggt taatttactc gggaatgcgg 23400
taaaatttac cgaaaccgga gggatacgtc tgacggtcaa gcgtcatgag gaacaattaa 23460
tatttctggt tagcgatagc ggtaaaggga ttgaaataca gcagcagtct caaatcttta 23520
ctgcttttta tcaagcagac acaaattcgc aaggtacagg aattggactg actattgcgt 23580
caagcctggc taaaatgatg ggcggtaatc tgacactaaa aagtgtcccc ggggttggaa 23640
cctgtgtctc gctagtatta cccttacaag aataccagcc gcctcaacca attaaaggga 23700
cactatcagc gccgttctgc ctgcatcgcc aactggcttg ctggggaata cgcggcgaac 23760
caccccacca gcaaaatgcg cttctcaacg cagagctttt gtatttcccc ggaaaactct 23820
acgacctggc gcaacagtta atattgtgta caccaaatat accagtaata aataatttgt 23880
taccccccctg gcagttgcag attcttttgg ttgatgatgc cgatattaat cgggatatca 23940
tcggcaaaat gcttgtcagc ctgggacaac acgtcactgt tgccgccagt agtaacgagg 24000
ctctgacttt atcacaacag cagcgattcg atttagtact gattgacatt agaatgccag 24060
aaatagatgg tattgaatgt gtacaattat ggcacgatga gccgaataat ttagatcctg 24120
actgcatgtt tgtggcgcta tccgctagcg tagcgacaga agatattcat cgttgtaaaa 24180
aaaatgggat tcatcattac attaccaaac cagtgacatt ggctacctta gctcgctata 24240
tcagtattgc cgcagaatat caacttttgc gaaatataga gctacaggag caggatccga 24300
gtcgctgctc agcgttactg gcgacagatg atatggtcat taatagcaag attttccaat 24360

cactggacct cttgctggct gatattgaaa atgctgtatc ggctggacaa aaaatcgatc 24420
agttaattca cacattaaaa ggctgtttag gtcaaatagg gcagactgaa ttggtatgct 24480
atgtcataga cattgagaat cgcgtaaaaa tggggaaaat catcgcgctg gaggaactaa 24540
ccgacttacg ccagaaaata cgtatgatct tcaaaaacta caccattact taatattatc 24600
ttaattttcg cgagggcagc aaaatgaaag aatataagat cttattagta gacgatcatg 24660
aaatcatcat taacggcatt atgaatgcct tattaccctg gcctcatttt aaaattgtag 24720
agcatgttaa aaatggtctt gaggtttata atgcctgttg cgcatacgag cctgacatac 24780
ttatccttga tcttagctta cctggcatca atggcctgga tatcattcct caattacatc 24840
agcgttggcc agcaatgaat attctggttt acacagcata ccaacaagag tatatgacca 24900
ttaaaacttt agccgcaggt gctaatggct atgttttaaa aagcagtagt cagcaagttc 24960
tgttagcggc attgcaaaca gtagcagtaa acaagcgtta cattgaccca acgttgaatc 25020
gggaagctat cctggctgaa ttaaacgctg acacgaccaa tcatcaactg cttactttgc 25080
gcgagcgtca ggttcttaaa cttattgacg aggggtatac caatcatggg atcagcgaaa 25140
agctacatat cagtataaaa accgtcgaaa cacaccggat gaatatgatg agaaagctac 25200
aggttcataa agtgacagag ttacttaact gtgcccgaag aatgaggtta atagagtatt 25260
aa 25262

<210> 3
<211> 6053
<212> DNA
<213> Artificial Sequence

<220>
<223> spvRABCD operon

<400> 3

atggatttct tgattaataa aaaattaaaa attttcataa cactgatgga aacaggttcc 60
ttcagtatcg caacatcagt actgtatatc acccgaaccc cgctgagcag ggttatttca 120
gacctggaaa gagagctgaa acaaagactc tttatacgga agaatggcac tcttatccca 180
accgaatttg cacaaactat ttatcgaaaa gtaaaatccc attatatttt cttacatgca 240
ctggagcagg aaatcggacc tacgggtaaa acgaaacaac tagaaataat atttgacgaa 300
atttatccgg aaagtttaaa aaatctgatc atttcagcac tgaccatttc cggccaaaaa 360
acaaatataa tggggagagc cgttaacagc caaataatag aagaactgtg tcagacaaac 420
aactgcattg ttatttctgc cagaaattat tttcatcggg aatcgcttgt ctgccggaca 480
tcagtggagg gtggggtcat gttatttatt cctaaaaaat tctttctctg cggcaaacct 540
gatatcaaca ggctggccgg aacacctgta ctttttcatg aggggctaa aaattttaat 600

ctggacacca tataccattt ttttaaacag acactaggta ttaccaaccc tgcattcagt 660

tttgataacg tcgatttgtt cagttcactg taccggttac aacaagggct ggcgatgtta 720

ctcatccccg tcagagtctg tcgggctctg ggattatcaa cagatcacgc actgcacatc 780

aaaggcgtag cgctctgtac ctccttgtat tacccgacca agaaacggga gacaccagat 840

tatcgtaaag ctataaaact gatacagcag gaactgaaac agtccacctt ctgaccttat 900

gcagcgtaag ggccgcaaca cctgtattca cggcatttgc cagattcaga ttgtcagcaa 960

tccccatcct ccatagcggt agttcaccgc ggagcatgga gtaaaccggc tggtcgccgt 1020

caatctgaca cagaatcagt ttgatgctct ggtggattac ctaaaacatg ggcattaacg 1080

cgctggctca cgccacttta ctgaagaaac tgaataacgg tgactatgac ggcgcagcga 1140

atgaattcct gaaatgggac cacgccagcg gtcaggttgt tcccggcctg acccgacgcc 1200

ggagcgctga acgttgttta ttcctgagtt aatttgttgt gccatctttg cacaccggga 1260

accgcgattc cgcacagcag aaaaatagca cataaataaa ctcaatataa gccactcatt 1320

ttctggcaat acaaaataat tcccctgcag acattatcag tcttcaggat ttcattctgt 1380

ttattttcag gagtcatcat tatttatgaa tatgaatcag accaccagtc cggcactttc 1440

acaggtcgaa accgccatcc gggtcccggc agggaatttt gcaaaatata attattattc 1500

cgtgtttgat attgtccgtc agacccgtaa acagtttatt aacgccaata tgtcatggcc 1560

gggatcccgc ggaggtaaag cctgggacct ggcgatgggc caggcgcagt atatccgctg 1620

catgttccga gaaaatcaat tgacccgcag agttcggggg accttgcagc agacaccgga 1680

caatggcacg aacctgagca gttccgctgt cggcggtatt cagggacagg cagagcgtcg 1740

gccggacctg gccaccctga tggtggttaa tgatgccatt aaccagcaaa taccgaccct 1800

gctgccgtat cattttccac acgaccaggt ggagttatct ctgctgaata ccgatgtgtc 1860

gctggaagat attatcagcg agagcagcat tgactggccg tggttcctga gcaactcgct 1920

gaccggcgat aacagtaact atgccatgga gctcgccagc cggctgtcac cagagcagca 1980

gacactgccg accgagccgg acaacagtac cgccactgac ctgacctctt tttaccagac 2040

caatctgggg ctgaaaaccg ccgactatac gccatttgaa gcactgaata cctttgcccg 2100

acagttagcg attaccgttc ccccaggtgg aacagttgat tgcgggtact ctgcgtgcca 2160

gccggcagtt tagcttcccg cgctaccaga gtagtgagca gcagaccatt ctgcagaatc 2220

tgagcgacgt cattgttcag gtgcattcta ccgcgctgta cggcggcagc acttttgaac 2280

aggccgtaga gcagacgctg taagcagaaa atatacctgt ccatcgtcag acggccagtt 2340

tcaggagata gtgtatgttg atactaaatg gtttttcatc tgccacttta gcgctgatca 2400

ctcccccttt cctgccaaaa gggggcaagg cgctgagtca gtcaggccct gacggcctag 2460

ccagtataac gctgtctctg cccatcagcg ccgaacgcgg ctttgcgcct gcgctggcgc 2520

tgcactacag cagcggtggc ggcaatggcc ccttcggcgt gggctggtcc tgcgcgacaa 2580
tgagcattgc ccgccgcacc agccatggcg tgccgcagta taacgacagc gatgagtttc 2640
tggggccgga cggagaagtg ctggttcaaa cgctcagcac cggtgatgcc cccaatcccg 2700
tcacctgctt cgcgtacggt gacgtatcgt tcccgcaaag ctacacggtg acccgctatc 2760
agccccgcac ggagagcagt ttttatcgcc tggagtactg ggtgggcaac agcaacggcg 2820
atgatttctg gttactgcat gacagtaacg gcatcctgca cctgctgggg aaaaccgccg 2880
cagcacgcct cagcgatccg caggccgcct ctcatacggc gcaatggctg gttgaggagt 2940
cggtgacccc tgccggcgag catatctatt actcctactt ggcggagaac ggtgacaatg 3000
tggacctcaa tggggacgag gccggacgcg atcgcagcgc catgcgctat ctcagcaagg 3060
tacagtatgg caacgcgacc cccgccgccg atctgtacct ctggactagc gccacacccg 3120
cggtacagtg gctgttcacc ctagtgtttg actacggcga acgtggtgta gatccacagg 3180
taccgcctgc attcactgct cagaacagct ggctcgcccg ccaggatccc ttctccctgt 3240
ataactacgg ctttgagatc cgcctccatc gcctgtgccg ccaagtcctg atgttccacc 3300
actttcctga tgaactgggt gaagccgata cgctggtttc ccgtctgctg ctggagtatg 3360
acgaaaatcc gatactgaca cagctttgcg ctgctcggac gctggcctat gaaggcgacg 3420
gttatagaag agctcctgtc aacaatatga tgccaccgcc accgccaccg cctcctccga 3480
tgatgggagg taattcatct cgaccaaaat caaaatgggc gattgtagag gaatcaaagc 3540
agattcaagc tctgaggtac tattcagctc aagggtacag tgtgattaat aaatatttac 3600
gtggggatga ttatcctgaa acacaggcaa aagaaactct gctctccaga gactatcttt 3660
ccacaaatga acccagtgat gaggagttta aaaatgccat gtcagtttat ataaatgata 3720
ttgtggaggg attaagttca cttcccgaaa cagatcacag agtcgtatac cggggcctga 3780
agcttgataa gcccgcatta tcggatgtgc tgaaggaata cactactata ggtaatataa 3840
taatagataa agcttttatg agtacatcgc cagataaggc atggataaat gacactattc 3900
tcaacatata cctagaaaaa ggacataaag gtagaatact cggagatgtt gcacatttta 3960
agggagaggc agagatgctt ttccctccaa atactaaact caaaatcgaa agcattgtaa 4020
attgtggatc ccaagacttt gcaagccagc ttagtaagct gagattaagt gatgatgcaa 4080
ctgctgacac aaacaggata aaaagaataa taaacatgag ggtactcaac tcatagatac 4140
taagaatcta ttccagaagt ggtatgagcg gcctagctct ataaggggtt atactccgga 4200
accccagatt tttccgtcac cctaggcccg caaagtagtg catctaaact tttgccatta 4260
cccttcttta actttctgct cggaacggac cgaaatatca ttttttcgcc tgataaaaaa 4320
tgaggttttc tggataacta atcgttttat taaaaaaaac tgagaattta tatctaataa 4380

tatggcgata tatccatatc gcaaaggaga tttcccatgc ccataaatag gcctaatcta 4440
aatctaaaca tccctccttt gaatattgta gctgcttatg atggggcgga aataccatct 4500
acaagtaagc acctgaaaaa taatttcaac tccttgcaca accaaatgcg gaagatgccg 4560
gtatcccact ttaaagaggc gctggatgtg cctgactatt cagggatgcg ccagagtggt 4620
ttctttgcta tgagccaagg ttttcagctg aataaccatg gttacgatgt tttcatccat 4680
gctcgtcgag aatcacctca gtctcagggc aaatttgccg gtgacaagtt ccacatcagt 4740
gtgctcaggg atatggtgcc acaagcattt caagcgctgt ccggattgct gttttcagag 4800
gacagtccgg tagataagtg gaaagtgacc gatatggaga aggtcgctca acaagaccgt 4860
gttagcctgg gcgctcagtt cacgttgtat ataaaaccag accaggaaaa ttcgcagtac 4920
agtgcgtcgt ttctccacaa gacacggcaa tttatagagt gtctggaatc cagactatcc 4980
gaaaatgggg ttatttcagg acagtgtcct gagtcagacg ttcatcctga aaattggaaa 5040
tatctcagtt atcgtaatga actacgaagt gggcgtgatg gtggcgaaat gcagagacag 5100
gctttacgtg aggaaccgtt ttatcgtttg atgacagagt aagtatgggt ttggggagca 5160
acggaacagt aaacgccgtt aaacaactat tttaaatgct cattaattta ttaatcaata 5220
aattacaaat tttcattgaa ggctcccccc ttactgacga attccggcac cgtaaaggaa 5280
taacgctcat gcatattgat gtgtccgcac tgtaatggtg aaaattacat aagcaagagc 5340
gtttttttgaa aaatattata tttaatgttt tgtaatatgc attttattga ggtagtgtaa 5400
ctatgagagt ttctggtagt gcgtcatccc aagatataat atcacgtata aattcaaaaa 5460
atatcaataa taatgattca aatgaagtca agagaattaa agatgcgctt tgtattgaat 5520
caaaagagag aattttgtat ccaaaaaatt tgagtctaga taatttaaaa caaatggcta 5580
gatatgtaaa taatacatac atccattact ctgggaactg cgttttatta tcagcgtgtt 5640
tacattataa catacatcac cgacaggata tattaagttc gaagaacact gcctctccta 5700
cagtgggatt agacagcgcc attgttgata aaatcatttt tggtcatgag cttaaccaat 5760
catattgttt aaattccatc gatgaggtgg aaaaagaaat attaaaccgt tatgacatta 5820
agagggaaag ttcttttatc attagcgcag agaactacat agctccaata attggcgaat 5880
gtagacatga tttcaacgct gtggttatct gtgaatatga taaaaaacca tatgtacaat 5940
tcattgattc ttggaaaaca tccaacatac ttcctagctt acaagaaata aaaaaacact 6000
tctcatcatc aggggaattt tatgtcaggg cttatgatga aaaacacgat tga 6053

<210> 4
<211> 1590
<212> DNA
<213> Artificial Sequence

<220>
<223> faeHIoperon

<400> 4

```
atgaaaataa cgcatcatta taaatctatt atttccgccc tggccgcgct ggccctgttt      60
tattccgcag caccccgggg ccgaattctt gacggcgggg aaatacagtt tcaccgtctg     120
gtcactgacg aggctccgaa atggacctgg caggtgggct cccctgacca gacatgggcg     180
gtggataccg ctgatgcccg tacagcgaac ggacaactgg tttttgattt acgcggcaag     240
ggctccctgc cgtttctgga aggccatctg tatgaggtgg cagagcgcgg tggtcccggc     300
ttcacccctt ttatctcctc cagcagtaac gggcagccgt tttccgtgac ggatggcggc     360
acgacgacgg cgcaacactt ccgcgcctct gtcccggtac gtaacccgga gaacggtcac     420
gtggcgggac agctttcttt caccettgac cagggaatgg ccgtcagcgc cggacaccag     480
gaagacgggg cggttctacc ggcagcgatg tcgctcgtaa acgggcagag cgtgacgggt     540
gtgcaggccg gcaccctgcc gcagtggctc aaaaaccgtc tgccttccct gctgatgctg     600
aaccggggct tcggtaacgg aatgagcacg gcagataacg gtcaggttat cagtcagggc     660
gtgctggctg acggccgggt gacccggctg gcggcggcct atgcgtccgc cgtctcggat     720
tttgagctga cgctgccggc agaaaacacg ccggtgcagt ggcaggccgg gctgagtgtg     780
acggtgacgg tccagtaaag aacgggcagg gagaggaaga acacaatgaa acgaatgacg     840
attttactgc tggccgccag tctgctgccg tcctgtgtgc tggcgtggaa cacgccgggg     900
gaagacttca gcggagagct gaagctgggc gggccggtga ccagcacccg taatccctgg     960
gtctggaagg tcggggaagg gaacacacag ataaacacga aagctgtctc tgtcctgcgc    1020
agtggggagg aggtaatacc ggttcccctg ccggccatga cggtcctgct gggaaaaact    1080
atcctgacca cccgggccgg ccgggagggg cttgcgccgc aggtgacgta cggtaaggac    1140
acagagggtt ttgcactgac gtggacggca ccgggtatgg catcggtgac actgccggtg    1200
acgggggagg gaaatgtccg taccgggaca ttcaccttcc ggatgcaggc ggcgggtgtg    1260
ctgcgccatg tcctggggaa ccgggcggag tatgccgggc tgtatggcga cctgcagggc    1320
aacggcttgc cgccacagac gcaggtgatg ccggcagggc agacgccggg tgtgctgcag    1380
accctgtttg acagtgaagg cccggtctgg ctccgggaga tgacggtcag cagcgtgtcc    1440
ggactgagcc ggttcagtga cgccgccctg cgccaggttg acggggtata cggcgcacag    1500
acggtggcgg acagcggtga gctgcgtttt aagggggcgg taccgtcccg ctggcatacc    1560
tccctggcgg tgagcattga atatcggtaa                                      1590
```

<210> 5
<211> 70

<212> DNA
<213> Artificial Sequence

<220>
<223> SPI-1-P1 primer

<400> 5

**ttatggcgct ggaaggattt cctctggcag gcaaccttat aatttcatta gtgtaggctg 60**

**gagctgcttc 70**

<210> 6
<211> 70
<212> DNA
<213> Artificial Sequence

<220>
<223> SPI-1-P2 primer

<400> 6

**atgcaaaata tggtcttaat tatatcatga tgagttcagc caacggtgat catatgaata 60**

**tcctccttag 70**

<210> 7
<211> 21
<212> DNA
<213> Artificial Sequence

<220>
<223> SPI-1-P3 primer

<400> 7
atgttcttaa caacgttact g 21

<210> 8
<211> 21
<212> DNA
<213> Artificial Sequence

<220>
<223> SPI-1-P4 primer

<400> 8
aggtagtacg ttactgacca c 21

<210> 9
<211> 70
<212> DNA
<213> Artificial Sequence

<220>
<223> SPI-2-P1 primer

<400> 9

accctcttaa ccttcgcagt ggcctgaaga agcataccaa aagcatttat gtgtaggctg 60

gagctgcttc 70

<210> 10
<211> 70
<212> DNA
<213> Artificial Sequence

<220>
<223> SPI-2-P2 primer

<400> 10

actgcgtggc gtaaggctca tcaaaatatg accaatgctt aataccatcg catatgaata 60

tcctccttag 70

<210> 11
<211> 21
<212> DNA
<213> Artificial Sequence

<220>
<223> SPI-2-P3 primer

<400> 11
tgttcgtact gccgatgtcg c 21

<210> 12
<211> 21
<212> DNA
<213> Artificial Sequence

<220>
<223> SPI-2-P4 primer

<400> 12
agtacgacga ctgacgccaa t 21

<210> 13
<211> 70
<212> DNA
<213> Artificial Sequence

<220>
<223> spv-P1 primer

<400> 13

gtgcaaaaac aggtcaccgc catcctgttt ttgcacatca aaacattttt gtgtaggctg 60

gagctgcttc 70

<210> 14
<211> 70
<212> DNA
<213> Artificial Sequence

<220>
<223> spv-P2 primer

<400> 14

ttaccccaac agcttgccgt gtttgcgctt gaacataggg atgcgggctt catatgaata 60

tcctccttag 70

<210> 15
<211> 21
<212> DNA
<213> Artificial Sequence

<220>
<223> spv-P3 primer

<400> 15
gaccatatct gcctgcctca g 21

<210> 16
<211> 21
<212> DNA
<213> Artificial Sequence

<220>
<223> spv-P4 primer

<400> 16
cagagcccgt tctctaccga c 21

<210> 17
<211> 70
<212> DNA
<213> Artificial Sequence

<220>
<223> fae-P1 primer

<400> 17

ttaccgatat tcaatgctca ccgccaggga ggtatgccag cgggacggta gtgtaggctg 60

gagctgcttc 70

<210> 18

<211> 70
<212> DNA
<213> Artificial Sequence

<220>
<223> fae-P2 primer

<400> 18

atgaaaataa cgcatcatta taaatctatt atttccgccc tggccgcgct catatgaata 60

tcctccttag 70

<210> 19
<211> 21
<212> DNA
<213> Artificial Sequence

<220>
<223> fae-P3 primer

<400> 19
caggctcccc tgccaccggc t 21

<210> 20
<211> 21
<212> DNA
<213> Artificial Sequence

<220>
<223> fae-P4 primer

<400> 20
caggccaact atctttccct a 21

<210> 21
<211> 22
<212> DNA
<213> Artificial Sequence

<220>
<223> spv-S1 primer

<400> 21
ggtcaattaa atccactcag aa 22

<210> 22
<211> 20
<212> DNA
<213> Artificial Sequence

<220>
<223> spv-S2 primer

<400> 22
acgggagaca ccagattatc 20

<210> 23
<211> 20
<212> DNA
<213> Artificial Sequence

<220>
<223> spv-S3 primer

<400> 23
ttcagtaaag tggcgtgagc 20

<210> 24
<211> 20
<212> DNA
<213> Artificial Sequence

<220>
<223> spv-S4 primer

<400> 24
ccaggtggag ttatctctgc 20

<210> 25
<211> 20
<212> DNA
<213> Artificial Sequence

<220>
<223> spv-S5 primer

<400> 25
actgtcgggc aaaggtattc 20

<210> 26
<211> 21
<212> DNA
<213> Artificial Sequence

<220>
<223> spv-S6 primer

<400> 26
tttctggtta ctgcatgaca g 21

<210> 27
<211> 19
<212> DNA
<213> Artificial Sequence

<220>
<223> spv-S7 primer

<400> 27
tccagaggta cagatcggc 19

<210> 28
<211> 21
<212> DNA

<213> Artificial Sequence

<220>
<223> spv-S8 primer

<400> 28
gaaggaatac actactatag g 21

<210> 29
<211> 20
<212> DNA
<213> Artificial Sequence

<220>
<223> spv-S9 primer

<400> 29
gtgtcagcag ttgcatcatc 20

<210> 30
<211> 20
<212> DNA
<213> Artificial Sequence

<220>
<223> spv-S10 primer

<400> 30
agtgaccgat atggagaagg 20

<210> 31
<211> 20
<212> DNA
<213> Artificial Sequence

<220>
<223> spv-S11 primer

<400> 31
aagcctgtct ctgcatttcg 20

<210> 32
<211> 21
<212> DNA
<213> Artificial Sequence

<220>
<223> spv-S12 primer

<400> 32
aaccgttatg acattaagag g 21

<210> 33
<211> 21
<212> DNA
<213> Artificial Sequence

<220>

<223> spv-S13 primer

<400> 33
taaggctctc tattaactta c 21

<210> 34
<211> 19
<212> DNA
<213> Artificial Sequence

<220>
<223> spv-S14 primer

<400> 34
aaccgcttct ggctgtagc 19

<210> 35
<211> 22
<212> DNA
<213> Artificial Sequence

<220>
<223> spv-S15 primer

<400> 35
ccgtaacaat gacattatcc tc 22

## Claims

1. Modified *Salmonella Gallinarum* strains, which comprise the deletion of at least one entire gene cluster selected from the following (i) to (iv):

   (i) *Salmonella* Pathogenicity Island-2 (SPI-2),
   *(ii) Salmonella* Pathogenicity Island-1 (SPI-1) and SPI-2,
   (iii) *spvRABCD,* and
   (iv) SPI-1, SPI-2, spvRABCD, and *faeHI.*

2. The modified *Salmonella Gallinarum* strains of claim 1, wherein an entire gene cluster of *Salmonella* pathogenicity island-2 of SEQ ID NO: 2 is deleted (SG3-d2).

3. The modified *Salmonella Gallinarum* strains of claim 2, is deposited under accession No.KCCM1 1009P.

4. The modified *Salmonella Gallinarum* strains of claim 1, wherein both gene clusters of *Salmonella* Pathogenicity Island-1 of SEQ ID NO: 1 and *Salmonella* Pathogenicity Island-2 of SEQ ID NO: 2 are deleted (SG3-d1d2).

5. The modified *Salmonella Gallinarum* strains of claim 4, is deposited under accession No. KCCM11010P.

6. The modified *Salmonella Gallinarum* strains of claim 1, wherein all gene clusters of *Salmonella* Pathogenicity Island-1 of SEQ ID NO: 1, *Salmonella* Pathogenicity Island-2 of SEQ ID NO: 2, *spvRABCD* of SEQ ID NO: 3, and *faeHI* of SEQ ID NO: 4 have been deleted (SG3-d4).

7. The modified *Salmonella Gallinarum* strains of claim 6, is deposited under accession No. KCCM11011P.

8. Use of the modified *Salmonella Gallinarum* strains according to the preceding claims to produce *Salmonella-specific* lytic bacteriophage.

9. A pharmaceutical composition comprising the modified *Salmonella Gallinarum* strains of claim 1 as an effective ingredient.

10. A pharmaceutical composition for use in prevention or treatment of fowl typhoid, comprising the modified *Salmonella Gallinarum* strains of claim 1 as an effective ingredient.

11. A vaccine comprising the modified *Salmonella Gallinarum* strains of claim 1 as an effective ingredient.

12. A feed additive, comprising the modified *Salmonella Gallinarum* strains of claim 1 as an effective ingredient.

13. The modified Salmonella Gallinarum strains of claim 1 for use in a method for prevention or treatment of animals.

14. A method for producing bacteriophage, comprising:

culturing the modified *Salmonella Gallinarum* strains of claim 1 in a medium;
inoculating the bacteriophage into the medium; and
recovering bacteriophage progeny from the medium.

## Patentansprüche

1. Modifizierte *Salmonella-Gallinarum*-Stämme, die die Deletion von mindestens einem vollständigen Gencluster umfassen, ausgewählt aus den folgenden (i) bis (iv):

(i) *Salmonellla*-Pathogenitätsinsel 2 (SPI-2),
(ii) *Salmonellla*-Pathogenitätsinsel 1 (SPI-1) und SPI-2,
(iii) *spvRABCD,* und
(iv) SPI-1, SPI-2, *spvRABCD* und *faeHI.*

2. Modifizierte *Salmonella-Gallinarum*-Stämme gemäß Anspruch 1, wobei ein vollständiger Gencluster der *Salmonellla*-Pathogenitätsinsel 2 von SEQ ID NO: 2 deletiert ist (SG3-d2).

3. Modifizierte *Salmonella-Gallinarum*-Stämme gemäß Anspruch 2 ist unter der Zugangsnummer KCCM11009P hinterlegt.

4. Modifizierte *Salmonella-Gallinarum*-Stämme gemäß Anspruch 1, wobei sowohl der Gencluster der *Salmonellla*-Pathogenitätsinsel 1 von SEQ ID NO: 1 als auch der der *Salmonellla*-Pathogenitätsinsel 2 von SEQ ID NO: 2 deletiert sind (SG3-d1d2).

5. Modifizierte *Salmonella-Gallinarum*-Stämme gemäß Anspruch 4 ist unter der Zugangsnummer KCCM11010P hinterlegt.

6. Modifizierte *Salmonella-Gallinarum*-Stämme gemäß Anspruch 1, wobei alle Gencluster der *Salmonellla*-Pathogenitätsinsel 1 von SEQ ID NO: 1, der *Salmonellla*-Pathogenitätsinsel 2 von SEQ ID NO: 2, *spvRABCD* von SEQ ID NO: 3 und *faeHI* von SEQ ID NO: 4 deletiert sind (SG3-d4).

7. Modifizierte *Salmonella-Gallinarum*-Stämme gemäß Anspruch 6 ist unter der Zugangsnummer KCCM11011P hinterlegt.

8. Verwendung der modifizierten *Salmonella-Gallinarum*-Stämme gemäß den vorhergehenden Ansprüchen zur Herstellung eines *Salmonella*-spezifischen lytischen Bakteriophagen.

9. Pharmazeutische Zusammensetzung, umfassend die modifizierten *Salmonella-Gallinarum*-Stämme gemäß Anspruch 1 als wirksamen Bestandteil.

10. Pharmazeutische Zusammensetzung zur Verwendung bei der Vorbeugung oder Behandlung von Geflügeltyphus, umfassend die modifizierten *Salmonella-Gallinarum*-Stämme gemäß Anspruch 1 als wirksamen Bestandteil.

**11.** Impfstoff, umfassend die modifizierten *Salmonella-Gallinarum*-Stämme gemäß Anspruch 1 als wirksamen Bestandteil.

**12.** Futterzusatz, umfassend die modifizierten *Salmonella-Gallinarum*-Stämme gemäß Anspruch 1 als wirksamen Bestandteil.

**13.** Modifizierte *Salmonella-Gallinarum*-Stämme gemäß Anspruch 1 zur Verwendung bei einem Verfahren zur Prävention oder Behandlung von Tieren.

**14.** Verfahren zur Herstellung eines Bakteriophagen, umfassend:

Kultivieren der modifizierten *Salmonella-Gallinarum*-Stämme gemäß Anspruch 1 in einem Medium;
Inokulieren des Bakteriophagen in das Medium; und
Gewinnen der Nachkommen des Bakteriophagen aus dem Medium.

**Revendications**

**1.** Souches modifiées de *Salmonella Gallinarum,* qui comprend la suppression d'au moins un cluster de gènes entier choisi parmi les étapes suivantes de (i) à (iv) :

(i) *Salmonella* Pathogenicity Island-2 (SPI-2),
(ii) *Salmonella* Pathogenicity Island-1 (SPI-1) et SPI-2,
(iii) *spvRABCD,* et
(iv) SPI-1, SPI-2, spvRABCD, et *faeHI.*

**2.** Souches modifiées de *Salmonella Gallinarum* de la revendication 1, où un cluster de gènes entier de *Salmonella* Pathogenicity island-2 de SEQ ID N° : 2 est supprimé (SG3-d2).

**3.** Souches modifiées de *Salmonella Gallinarum* de la revendication 2, qui sont déposées selon l'accès N°KCCM11009P.

**4.** Souches modifiées de *Salmonella Gallinarum* de la revendication 1, où les deux clusters de gènes de *Salmonella* Pathogenicity Island-1 de SEQ ID N° : 1 et *Salmonella* Pathogenicity Island-2 de SEQ ID N° : 2 sont supprimés (SG3-d1d2).

**5.** Souches modifiées de *Salmonella Gallinarum* de la revendication 4, qui sont déposées selon l'accès N°KCCM11010P.

**6.** Souches modifiées de *Salmonella Gallinarum* de la revendication 1, où tous les clusters de gènes de *Salmonella* Pathogenicity Island-1 de SEQ ID N° : 1, *Salmonella* Pathogenicity Island-2 de SEQ ID N° : 2, *spvRABCD* de SEQ ID N° : 3, et *faeHI* de SEQ ID N° : 4 ont été supprimés (SG3-d4).

**7.** Souches modifiées de *Salmonella Gallinarum* de la revendication 6, qui sont déposées selon l'accès N°KCCM11011P.

**8.** Utilisation des souches modifiées de *Salmonella Gallinarum* selon les revendications précédentes pour produire des bactériophages lytiques spécifiques pour *Salmonella.*

**9.** Composition pharmaceutique comprenant les souches modifiées de *Salmonella Gallinarum* de la revendication 1 en tant qu'un ingrédient efficace.

**10.** Composition pharmaceutique pour une utilisation en prévention ou traitement de la typhose aviaire, comprenant les souches modifiées de *Salmonella Gallinarum* de la revendication 1 en tant qu'un ingrédient efficace.

**11.** Vaccin comprenant les souches modifiées de *Salmonella Gallinarum* de la revendication 1 en tant qu'un ingrédient efficace.

**12.** Additif alimentaire, comprenant les souches modifiées de *Salmonella Gallinarum* de la revendication 1 en tant qu'un ingrédient efficace.

**13.** Souches modifiées de *Salmonella Gallinarum* de la revendication 1 pour une utilisation dans une méthode pour la prévention ou le traitement d'animaux.

**14.** Méthode pour produire un bactériophage, comprenant :

la culture des souches modifiées de *Salmonella Gallinarum* de la revendication 1 dans un milieu ;
l'inoculation du bactériophage dans le milieu ; et
la récupération de la progéniture bactériophage du milieu.

[Fig. 1]

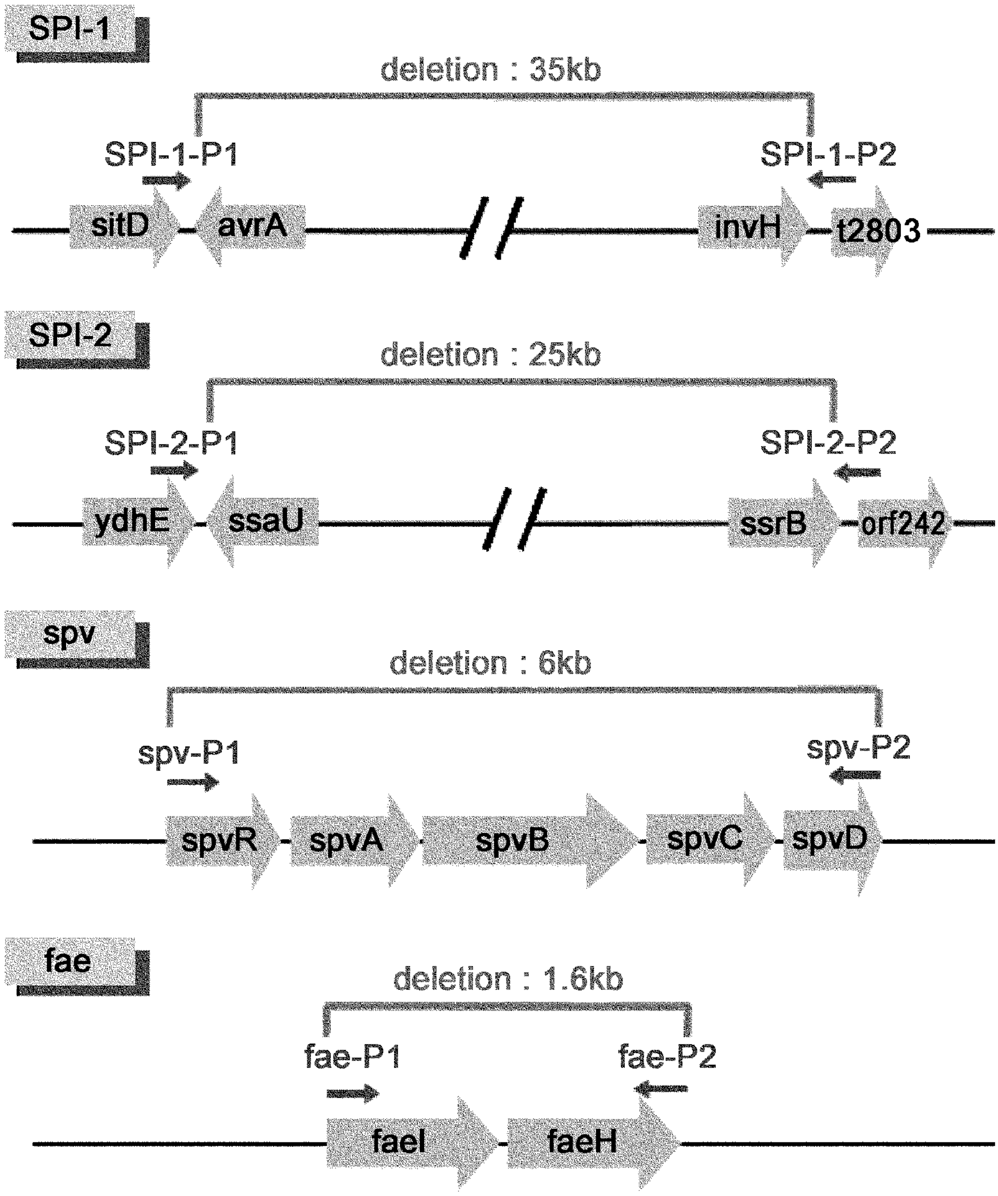
SPI-1
deletion : 35kb
SPI-1-P1
SPI-1-P2
sitD
avrA
invH
t2803
SPI-2
deletion : 25kb
SPI-2-P1
SPI-2-P2
ydhE
ssaU
ssrB
orf242
spv
deletion : 6kb
spv-P1
spv-P2
spvR
spvA
spvB
spvC
spvD
fae
deletion : 1.6kb
fae-P1
fae-P2
faeI
faeH

[Fig. 2]

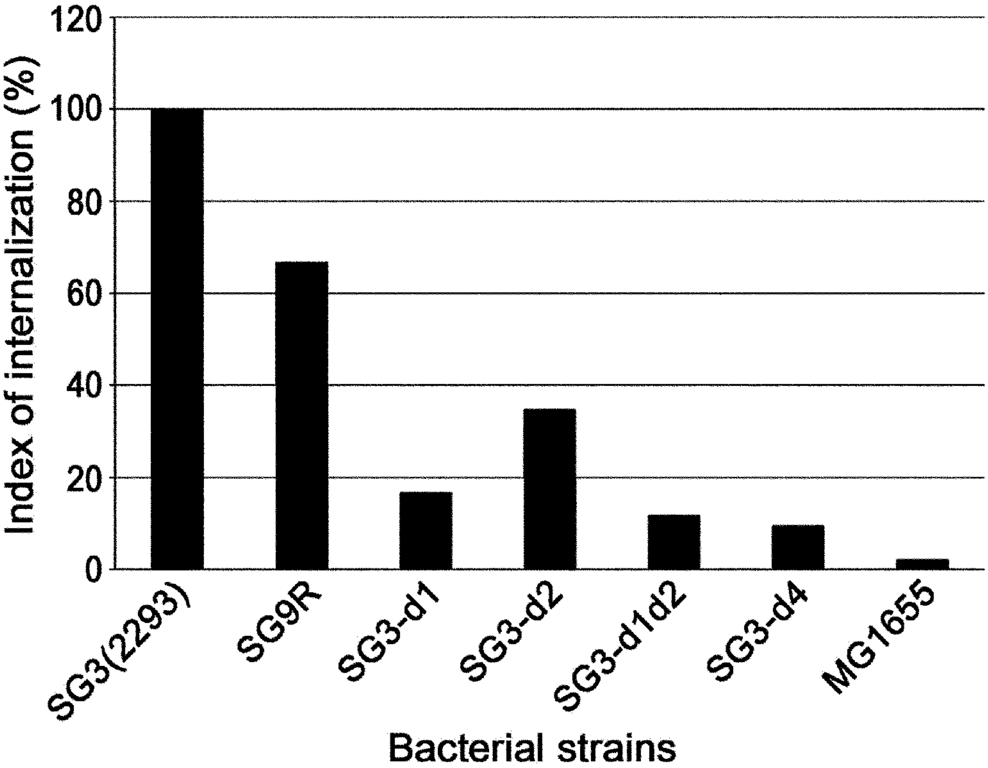
120
100
80
60
40
20
0
Index of internalization (%)
SG3(2293)
SG9R
SG3-d1
SG3-d2
SG3-d1d2
SG3-d4
MG1655
Bacterial strains

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199808944 A
- WO 199531562 A
- EP 1990202169 A
- WO 199003122 A
- US 6923957 B
- US 7211264 B
- US 7887816 B
- US 20100135962 A
- KR 102008121500
- US 20100158870 A
- US 20100166709 A
- US 61487137 B
- US 13274854 B


### Non-patent literature cited in the description


- **MOTA LJ et al.** *Ann Med.,* 2005, vol. 37 (4), 234-249
- **SCHLUMBERGER MC et al.** *Curr Opin Microbiol,* 2006, vol. 9 (1), 46-54
- **KIMBROUGH TG et al.** *Microbes Infect,* 2002, vol. 4 (1), 75-82
- **WATERMAN SR et al.** *Cell Microbiol,* 2003, vol. 5 (8), 501-511
- **ABRAHAMS GL.** *Cell Microbiol,* 2006, vol. 8 (5), 728-737
- **EDWARDS RA et al.** *PNAS,* 2000, vol. 97 (3), 1258-1262
- **GULIG PA et al.** *Mol Microbiol,* 1993, vol. 7 (6), 825-830
- **ATTERBURY RJ et al.** *Appl Environ Microbiol,* 2007, vol. 73 (14), 4543-4549
- **RIPP S et al.** *J Appl Microbiol,* 2006, vol. 100 (3), 488-499
- **D.H.SHAH.** *Microbiology,* 01 December 2005, vol. 151 (12
- **I. RYCHLIK et al.** *FEMS Microbiology letters,* 15 February 1998, vol. 159 (2
- **S. BARTH ; R. BAUERFEIND.** *Berl. Münch. Tierärztl. Wschr,* 01 January 2005, vol. 118
- **EDWARDS RA et al.** *Trends Microbiol,* 2002, vol. 10 (2), 94-99
- **JENSEN ; HAMMER.** *Biotechnology and Bioengineering,* 1998, vol. 58, 191195
- **CARRIER ; KEASLING.** *Biotechnology Progress,* 1999, vol. 15, 5864
- **FRANCH ; GERDES.** *Current Opinion in Microbiology,* 2000, vol. 3, 159164
- **KNIPPERS.** Molecular Genetics. 1995
- **WINNACKER.** *Genes and Clones,* 1990
- *Journal of Biological Chemistry,* 1997, vol. 272, 86118617
- **YANO et al.** *Proceedings of the National Academy of Sciences,* 1998, vol. 95, 5511-5515
- **WENTE ; SCHACHMANN.** *Journal of Biological Chemistry,* 1991, vol. 266
- **HAGEMANN.** *General Genetics,* 1986
- **SAMBROOK et al.** Molecular Cloning : A Laboratory Manual. Cold SpringHarbor Press, 2001
- **RUSSELL CB et al.** *J. Bacteriol.,* 1989, vol. 171, 2609-2613
- **HAMILTON CM et al.** *J. Bacteriol.,* 1989, vol. 171, 4617-4622
- **LINK AJ et al.** *J. Bacteriol.,* 1997, vol. 179, 6228-6237
- **DATSENKO KA et al.** *PNAS,* 2000, vol. 97 (12), 6640-6645
- **HAPFELMEIER S et al.** *J Immunol,* 2005, vol. 174 (3), 1675-1685
- **BRUMME S et al.** *Vet Microbiol,* 2007, vol. 124 (3-4), 274-285
- **DESIN TS et al.** *Infect Immun,* July 2009, 2866-2875
- **SAMBROOK et al.** Molecular Cloning, a Laboratory Manual. Cold Spring Harbor Laboratories, 1989
- **HENDERSON SC et al.** *Infect Immun,* 1999, vol. 67 (7), 3580-3586
- **LOSTROH CP et al.** *Microbes Infect,* 2001, vol. 3 (14-15), 1281-1291